(19) **Europäisches Patentamt / European Patent Office / Office européen des brevets**

(11) **EP 2 942 359 A1**

(12) **EUROPEAN PATENT APPLICATION**

(43) Date of publication:
**11.11.2015 Bulletin 2015/46**

(51) Int Cl.:
***C07K 16/24*** (2006.01)   ***A61K 39/395*** (2006.01)

(21) Application number: **15157149.4**

(22) Date of filing: **18.07.2003**

(84) Designated Contracting States:
**AT BE BG CH CY CZ DE DK EE ES FI FR GB GR HU IE IT LI LU MC NL PT RO SE SI SK TR**

(30) Priority: **19.07.2002 US 397275 P**
**16.09.2002 US 411081 P**
**10.10.2002 US 417490 P**
**18.03.2003 US 455777 P**

(62) Document number(s) of the earlier application(s) in accordance with Art. 76 EPC:
**07150442.7 / 1 944 322**
**03748949.9 / 1 542 720**

(71) Applicant: **AbbVie Biotechnology Ltd**
**HM 11 Hamilton (BM)**

(72) Inventors:
- **Banerjee, Subhashis**
  **Hamden, CT 06514 (US)**
- **Taylor, Lori K.**
  **Wadsworth, IL 60083 (US)**
- **Spiegler, Clive E.**
  **Emmer Green Reading, Berkshire RG4 8TL (GB)**
- **Tracey, Daniel, Edward**
  **Harvard, MS 01451 (US)**
- **Chartash, Eliot, Keith**
  **Randolph, NJ 07869 (US)**

- **Hoffman, Rebecca S.**
  **Wilmette, IL 60091 (US)**
- **Barchuk, William T.**
  **Madison, NJ 07940 (US)**
- **Yan, Philip**
  **Vernon Hills, IL 60061 (US)**
- **Murtaza, Anwar**
  **Westborough, MA 01581 (US)**
- **Salfeld, Jochen, G.**
  **North Grafton, MA 01536 (US)**
- **Fischkoff, Steven**
  **Short Hills, NJ 07078 (US)**

(74) Representative: **Adams, Harvey Vaughan John et al**
**Mathys & Squire LLP**
**The Shard**
**32 London Bridge Street**
**London SE1 9SG (GB)**

Remarks:
•Amended claims in accordance with Rule 137(2) EPC.
•This application was filed on 02-03-2015 as a divisional application to the application mentioned under INID code 62.
•Claims filed after the date of filing of the application (Rule 68(4) EPC).

(54) **Treatment of TNF alpha related disorders**

(57) Methods of treating TNFalpha-related disorders comprising administering TNFalpha inhibitors, including TNFalpha antibodies are described.

SEQUENCE LISTING

<110> Abbott Laboratories S.A., et al.

<120> Treatment of TNF alpha Related Disorders

<130> BPI-187PC

<140> EP07150442.7
<141> 2003-07-18

<150> 60/397,275
<151> 2002-07-19

<150> 60/411,081
<151> 2002-09-16

<150> 60/417,490
<151> 2002-10-10

<150> 60/455,777
<151> 2003-03-18

<160> 37

<170> FastSEQ for Windows Version 4.0

<210> 1
<211> 107
<212> PRT
<213> Artificial Sequence

<220>
<223> Mutated human antibody

<400> 1
Asp Ile Gln Met Thr Gln Ser Pro Ser Ser Leu Ser Ala Ser Val Gly
1               5                   10                  15
Asp Arg Val Thr Ile Thr Cys Arg Ala Ser Gln Gly Ile Arg Asn Tyr
                20                  25                  30
Leu Ala Trp Tyr Gln Gln Lys Pro Gly Lys Ala Pro Lys Leu Leu Ile
                35                  40                  45
Tyr Ala Ala Ser Thr Leu Gln Ser Gly Val Pro Ser Arg Phe Ser Gly
        50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Arg Tyr Asn Arg Ala Pro Tyr
                85                  90                  95
Thr Phe Gly Gln Gly Thr Lys Val Glu Ile Lys
                100                 105

<210> 2
<211> 121
<212> PRT
<213> Artificial Sequence

<220>
<223> Mutated human antibody

<400> 2
Glu Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
                20                  25                  30
Ala Met His Trp Val Arg Gln Ala Pro Gly Lys Gly Leu Glu Trp Val
                35                  40                  45

```
Ser Ala Ile Thr Trp Asn Ser Gly His Ile Asp Tyr Ala Asp Ser Val
    50                  55              60
Glu Gly Arg Phe Thr Ile Ser Arg Asp Asn Ala Lys Asn Ser Leu Tyr
65                  70                  75                      80
Leu Gln Met Asn Ser Leu Arg Ala Glu Asp Thr Ala Val Tyr Tyr Cys
                85                  90                  95
Ala Lys Val Ser Tyr Leu Ser Thr Ala Ser Ser Leu Asp Tyr Trp Gly
            100                 105                 110
Gln Gly Thr Leu Val Thr Val Ser Ser
        115                 120
```

```
<210> 3
<211> 9
<212> PRT
<213> Artificial Sequence

<220>
<223> Mutated human antibody

<220>
<221> VARIANT
<222> 9
<223> Ala

<400> 3
Gln Arg Tyr Asn Arg Ala Pro Tyr Thr
 1               5
```

```
<210> 4
<211> 12
<212> PRT
<213> Artificial Sequence

<220>
<223> Mutated human antibody

<220>
<221> VARIANT
<222> 12
<223> Asn

<400> 4
Val Ser Tyr Leu Ser Thr Ala Ser Ser Leu Asp Tyr
 1               5                   10
```

```
<210> 5
<211> 7
<212> PRT
<213> Artificial Sequence

<220>
<223> Mutated human antibody

<400> 5
Ala Ala Ser Thr Leu Gln Ser
 1               5
```

```
<210> 6
<211> 17
<212> PRT
<213> Artificial Sequence

<220>
<223> Mutated human antibody
```

<400> 6
Ala Ile Thr Trp Asn Ser Gly His Ile Asp Tyr Ala Asp Ser Val Glu
1               5                   10                  15
Gly

<210> 7
<211> 11
<212> PRT
<213> Artificial Sequence

<220>
<223> Mutated human antibody

<400> 7
Arg Ala Ser Gln Gly Ile Arg Asn Tyr Leu Ala
1               5                   10

<210> 8
<211> 5
<212> PRT
<213> Artificial Sequence

<220>
<223> Mutated human antibody

<400> 8
Asp Tyr Ala Met His
1               5

<210> 9
<211> 107
<212> PRT
<213> Artificial Sequence

<220>
<223> Mutated human antibody

<400> 9
Asp Ile Gln Met Thr Gln Ser Pro Ser Ser Leu Ser Ala Ser Ile Gly
1               5                   10                  15
Asp Arg Val Thr Ile Thr Cys Arg Ala Ser Gln Gly Ile Arg Asn Tyr
            20                  25                  30
Leu Ala Trp Tyr Gln Gln Lys Pro Gly Lys Ala Pro Lys Leu Leu Ile
        35                  40                  45
Tyr Ala Ala Ser Thr Leu Gln Ser Gly Val Pro Ser Arg Phe Ser Gly
    50                  55                  60
Ser Gly Ser Gly Thr Asp Phe Thr Leu Thr Ile Ser Ser Leu Gln Pro
65                  70                  75                  80
Glu Asp Val Ala Thr Tyr Tyr Cys Gln Lys Tyr Asn Ser Ala Pro Tyr
                85                  90                  95
Ala Phe Gly Gln Gly Thr Lys Val Glu Ile Lys
            100                 105

<210> 10
<211> 121
<212> PRT
<213> Artificial Sequence

<220>
<223> Mutated human antibody

<400> 10

```
Gln Val Gln Leu Val Glu Ser Gly Gly Gly Leu Val Gln Pro Gly Arg
1               5                   10                  15
Ser Leu Arg Leu Ser Cys Ala Ala Ser Gly Phe Thr Phe Asp Asp Tyr
            20                  25                  30
Ala Met His Trp Val Arg Gln Ala Pro Gly Lys Gly Leu Asp Trp Val
        35                  40                  45
Ser Ala Ile Thr Trp Asn Ser Gly His Ile Asp Tyr Ala Asp Ser Val
    50                  55                  60
Glu Gly Arg Phe Ala Val Ser Arg Asp Asn Ala Lys Asn Ala Leu Tyr
65                  70                  75                  80
Leu Gln Met Asn Ser Leu Arg Pro Glu Asp Thr Ala Val Tyr Tyr Cys
                85                  90                  95
Thr Lys Ala Ser Tyr Leu Ser Thr Ser Ser Ser Leu Asp Asn Trp Gly
            100                 105                 110
Gln Gly Thr Leu Val Thr Val Ser Ser
            115                 120
```

<210> 11
<211> 9
<212> PRT
<213> Artificial Sequence

<220>
<223> Mutated human antibody

<400> 11
```
Gln Lys Tyr Asn Ser Ala Pro Tyr Ala
1               5
```

<210> 12
<211> 9
<212> PRT
<213> Artificial Sequence

<220>
<223> Mutated human antibody

<400> 12
```
Gln Lys Tyr Asn Arg Ala Pro Tyr Ala
1               5
```

<210> 13
<211> 9
<212> PRT
<213> Artificial Sequence

<220>
<223> Mutated human antibody

<400> 13
```
Gln Lys Tyr Gln Arg Ala Pro Tyr Thr
1               5
```

<210> 14
<211> 9
<212> PRT
<213> Artificial Sequence

<220>
<223> Mutated human antibody

<400> 14
```
Gln Lys Tyr Ser Ser Ala Pro Tyr Thr
1               5
```

```
<210> 15
<211> 9
<212> PRT
<213> Artificial Sequence

<220>
<223> Mutated human antibody

<400> 15
Gln Lys Tyr Asn Ser Ala Pro Tyr Thr
1               5


<210> 16
<211> 9
<212> PRT
<213> Artificial Sequence

<220>
<223> Mutated human antibody

<400> 16
Gln Lys Tyr Asn Arg Ala Pro Tyr Thr
1               5


<210> 17
<211> 9
<212> PRT
<213> Artificial Sequence

<220>
<223> Mutated human antibody

<400> 17
Gln Lys Tyr Asn Ser Ala Pro Tyr Tyr
1               5


<210> 18
<211> 9
<212> PRT
<213> Artificial Sequence

<220>
<223> Mutated human antibody

<400> 18
Gln Lys Tyr Asn Ser Ala Pro Tyr Asn
1               5


<210> 19
<211> 9
<212> PRT
<213> Artificial Sequence

<220>
<223> Mutated human antibody

<400> 19
Gln Lys Tyr Thr Ser Ala Pro Tyr Thr
1               5


<210> 20
```

```
<211> 9
<212> PRT
<213> Artificial Sequence

<220>
<223> Mutated human antibody

<400> 20
Gln Lys Tyr Asn Arg Ala Pro Tyr Asn
 1               5


<210> 21
<211> 9
<212> PRT
<213> Artificial Sequence

<220>
<223> Mutated human antibody

<400> 21
Gln Lys Tyr Asn Ser Ala Ala Tyr Ser
 1               5


<210> 22
<211> 9
<212> PRT
<213> Artificial Sequence

<220>
<223> Mutated human antibody

<400> 22
Gln Gln Tyr Asn Ser Ala Pro Asp Thr
 1               5


<210> 23
<211> 9
<212> PRT
<213> Artificial Sequence

<220>
<223> Mutated human antibody

<400> 23
Gln Lys Tyr Asn Ser Asp Pro Tyr Thr
 1               5


<210> 24
<211> 9
<212> PRT
<213> Artificial Sequence

<220>
<223> Mutated human antibody

<400> 24
Gln Lys Tyr Ile Ser Ala Pro Tyr Thr
 1               5


<210> 25
<211> 9
<212> PRT
<213> Artificial Sequence
```

```
<220>
<223> Mutated human antibody

<400> 25
Gln Lys Tyr Asn Arg Pro Pro Tyr Thr
1               5


<210> 26
<211> 9
<212> PRT
<213> Artificial Sequence

<220>
<223> Mutated human antibody

<400> 26
Gln Arg Tyr Asn Arg Ala Pro Tyr Ala
1               5


<210> 27
<211> 12
<212> PRT
<213> Artificial Sequence

<220>
<223> Mutated human antibody

<400> 27
Ala Ser Tyr Leu Ser Thr Ser Ser Ser Leu Asp Asn
1               5                   10


<210> 28
<211> 12
<212> PRT
<213> Artificial Sequence

<220>
<223> Mutated human antibody

<400> 28
Ala Ser Tyr Leu Ser Thr Ser Ser Ser Leu Asp Lys
1               5                   10


<210> 29
<211> 12
<212> PRT
<213> Artificial Sequence

<220>
<223> Mutated human antibody

<400> 29
Ala Ser Tyr Leu Ser Thr Ser Ser Ser Leu Asp Tyr
1               5                   10


<210> 30
<211> 12
<212> PRT
<213> Artificial Sequence

<220>
<223> Mutated human antibody
```

76

<400> 30
Ala Ser Tyr Leu Ser Thr Ser Ser Ser Leu Asp Asp
1               5                   10

<210> 31
<211> 12
<212> PRT
<213> Artificial Sequence

<220>
<223> Mutated human antibody

<400> 31
Ala Ser Tyr Leu Ser Thr Ser Phe Ser Leu Asp Tyr
1               5                   10

<210> 32
<211> 12
<212> PRT
<213> Artificial Sequence

<220>
<223> Mutated human antibody

<400> 32
Ala Ser Tyr Leu Ser Thr Ser Ser Ser Leu His Tyr
1               5                   10

<210> 33
<211> 12
<212> PRT
<213> Artificial Sequence

<220>
<223> Mutated human antibody

<400> 33
Ala Ser Phe Leu Ser Thr Ser Ser Ser Leu Glu Tyr
1               5                   10

<210> 34
<211> 12
<212> PRT
<213> Artificial Sequence

<220>
<223> Mutated human antibody

<400> 34
Ala Ser Tyr Leu Ser Thr Ala Ser Ser Leu Glu Tyr
1               5                   10

<210> 35
<211> 12
<212> PRT
<213> Artificial Sequence

<220>
<223> Mutated human antibody

<400> 35
Val Ser Tyr Leu Ser Thr Ala Ser Ser Leu Asp Asn

1         5         10

```
<210> 36
<211> 321
<212> DNA
<213> Artificial Sequence

<220>
<223> Mutated human antibody

<400> 36
gacatccaga tgacccagtc tccatcctcc ctgtctgcat ctgtagggga cagagtcacc 60
atcacttgtc gggcaagtca gggcatcaga aattacttag cctggtatca gcaaaaacca 120
gggaaagccc ctaagctcct gatctatgct gcatccactt tgcaatcagg ggtcccatct 180
cggttcagtg gcagtggatc tgggacagat ttcactctca ccatcagcag cctacagcct 240
gaagatgttg caacttatta ctgtcaaagg tataaccgtg caccgtatac ttttggccag 300
gggaccaagg tggaaatcaa a 321


<210> 37
<211> 363
<212> DNA
<213> Artificial Sequence

<220>
<223> Mutated human antibody

<400> 37
gaggtgcagc tggtggagtc tgggggaggc ttggtacagc ccggcaggtc cctgagactc 60
tcctgtgcgg cctctggatt cacctttgat gattatgcca tgcactgggt ccggcaagct 120
ccagggaagg gcctggaatg ggtctcagct atcacttgga atagtggtca catagactat 180
gcggactctg tggagggccg attcaccatc tccagagaca acgccaagaa ctccctgtat 240
ctgcaaatga acagtctgag agctgaggat acggccgtat attactgtgc gaaagtctcg 300
taccttagca ccgcgtcctc ccttgactat tggggccaag gtaccctggt caccgtctcg 360
agt 363
```

## Description

### RELATED APPLICATIONS

This application claims priority to prior filed U.S. Provisional Application Serial No. 60/397,275, filed July 19, 2002. This application also claims priority to prior filed to U.S. Provisional Application Serial No. 60/411,081, filed September 16, 2002, and prior-filed U.S. Provisional Application Serial No. 60/417490, filed October 10, 2002. This application also claims priority to prior filed to U.S. Provisional Application Serial No. 60/455777, filed March 18, 2003. In addition, this application is related to U.S. Patent Nos. 6,090,382, 6,258,562, and 6,509,015. This application is also related to U.S. Patent Application Serial No. 09/801,185, filed March 7,2001; U.S. Patent Application Serial No. 10/302,356, filed November 22,2002; U.S. Patent Application Serial No. 10/163657, filed June 2,2002; and U.S. Patent Application Serial No. 10/133715, filed April 26, 2002.

This application is related to U.S. utility applications (Attorney Docket No. BPI-187) entitled "Treatment of TNFα-Related Disorders Using TNFα Inhibitors," (Attorney Docket No. BPI-188) entitled "Treatment of Spondyloarthropathies Using TNFα Inhibitors," (Attorney Docket No. BPI-189) entitled "Treatment of Pulmonary Disorders Using TNFα Inhibitors," (Attorney Docket No. BPI-190) entitled "Treatment of Coronary Disorders Using TNFα Inhibitors," (Attorney Docket No. BPI-191) entitled "Treatment of Metabolic Disorders Using TNFα Inhibitors," (Attorney Docket No. BPI-192) entitled "Treatment of Anemia Using TNFα Inhibitors," (Attorney Docket No. BPI-193) entitled "Treatment of Pain Using TNFα Inhibitors," (Attorney Docket No. BPI-194) entitled "Treatment of Hepatic Disorders Using TNFα Inhibitors," (Attorney Docket No. BPI-195) entitled "Treatment of Skin and Nail Disorders Using TNFα Inhibitors," (Attorney Docket No. BPI-196) entitled "Treatment of Vasculitides Using TNFα Inhibitors," and (Attorney Docket No. BPI-197) entitled "Treatment of TNFα-Related Disorders Using TNFα Inhibitors," all of which are filed on even date herewith. The entire contents of each of these patents and patent applications are hereby incorporated herein by reference.

### BACKGROUND OF THE INVENTION

Cytokines, such as interleukin-1 (IL-1)and tumor necrosis factor (TNF) are molecules produced by a variety of cells, such as monocytes and macrophages, which have been identified as mediators of inflammatory processes. Cytokines, including TNF, regulate the intensity and duration of the inflammatory response which occurs as the result of an injury or infection. TNFα (also referred to as TNF) has been implicated in the pathophysiology of a variety of human diseases and disorders, including sepsis, infections, autoimmune diseases, transplant rejection and graft-versus-host disease (see *e.g.,* Moeller et al. (1990) Cytokine 2:162; U.S. Patent No. 5,231,024 to Moeller et al.; European Patent Publication No. 260 610 B1 by Moeller, A. et al.; Vasilli (1992) Annu. Rev. Immunol. 10:411; Tracey and Cerami (1994) Annu. Rev. Med. 45:491).

### SUMMARY OF THE INVENTION

There is a need to treat TNFα-related disorders, where TNFα activity is detrimental, in a safe and effective manner. The present invention includes methods for safe and effective treatment of TNFα-related disorders where TNFα activity is detrimental.

One aspect of the invention describes a method of treating a TNFα-related disorder in a subject comprising administering to the subject a therapeutically effective amount of a neutralizing, high affinity TNFα antibody, such that said disorder is treated. In one embodiment the TNFα-related disorder is a spondyloarthropathy, a pulmonary disorder, a coronary disorder, a metabolic disorder, anemia, pain, a hepatic disorder, a skin disorder, a nail disorder, or vasculitis. In another embodiment, the TNFα-related disorder is age-related cachexia, Alzheimer's disease, brain edema, inflammatory brain injury, chronic fatigue syndrome, dermatomyositis, drug reactions, edema in and/or around the spinal cord, familial periodic fevers, Felty's syndrome, fibrosis, glomerulonephritides (e.g. post-streptococcal glomerulonephritis or IgA nephropathy), loosening of prostheses, microscopic polyangiitis, mixed connective tissue disorder, multiple myeloma, cancer and cachexia, multiple organ disorder, myelo dysplastic syndrome, orchitism ostefllysis, pancreatitis, including acute, chronic, and pancreatic abscess, periodontal disease polymyositis, progressive renal failure, pseudogout, pyoderma gangrenosum, relapsing polychondritis, rheumatic heart disease, sarcoidosis, sclerosing cholangitis, stroke, thoracoabdominal aortic aneurysm repair (TAAA), TNF receptor associated periodic syndrome (TRAPS), symptoms related to Yellow Fever vaccination, inflammatory diseases associated with the ear, chronic ear inflammation, or pediatric ear inflammation. In still another embodiment of the invention, the TNFα-related disorder is a Crohn's disease-related disorder, juvenile arthritis/Still's disease (JRA), uveitis, sciatica, prostatitis, endometriosis, choroidal neovascularization, lupus, Sjogren's syndrome, and wet macular degeneration.

In one embodiment, the antibody of the invention is an isolated human antibody, or an antigen-binding portion thereof, that dissociates from human TNFα with a $K_d$ of $1 \times 10^{-8}$ M or less and a $K_{off}$ rate constant of $1 \times 10^{-3}$ s$^{-1}$ or less,

both determined by surface plasmon resonance, and neutralizes human TNFα cytotoxicity in a standard in vitro L929 assay with an $IC_{50}$ of 1 x $10^{-7}$ M or less.

In another embodiment of the invention, the antibody is an isolated human antibody, or an antigen-binding portion thereof which dissociates from human TNFα with a $K_{off}$ rate constant of 1 x $10^{-3}$ $s^{-1}$ or less, as determined by surface plasmon resonance; has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9; and has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

In another embodiment of the invention, the antibody is an isolated human antibody, or an antigen-binding portion thereof, with a light chain variable region (LCVR) comprising the amino acid sequence of SEQ ID NO:1 and a heavy chain variable region (HCVR) comprising the amino acid sequence of SEQ ID NO: 2.

In a further embodiment of the invention, the antibody is D2E7, also referred to as HUMIRA® or adalimumab.

Another aspect of the invention includes a method of treating a subject suffering from a TNFα-related disorder comprising administering a therapeutically effective amount of a TNFα antibody, or an antigen-binding fragment thereof, to the subject, wherein the antibody dissociates from human TNFα with a $K_d$ of 1 x $10^{-8}$ M or less and a $K_{off}$ rate constant of 1 x $10^{-3}$ $s^{-1}$ or less, both determined by surface plasmon resonance, and neutralizes human TNFα cytotoxicity in a standard in vitro L929 assay with an $IC_{50}$ of 1 x $10^{-7}$ M or less, such that said TNFα-related disorder is treated.

Still another aspect of the invention includes a method of treating a subject suffering from a TNFα-related disorder, comprising administering a therapeutically effective amount a TNFα antibody, or an antigen-binding fragment thereof, wherein the antibody dissociates from human TNFα with a $K_{off}$ rate constant of 1 x $10^{-3}$ $s^{-1}$ or less, as determined by surface plasmon resonance; has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9; and has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12, such that said TNFα-related disorder is treated.

A further aspect of the invention features a method of treating a subject suffering from a TNFα-related disorder, comprising administering a therapeutically effective amount a TNFα antibody, or an antigen-binding fragment thereof, with a light chain variable region (LCVR) comprising the amino acid sequence of SEQ ID NO: 1 and a heavy chain variable region (HCVR) comprising the amino acid sequence of SEQ ID NO: 2, such that said TNFα-related disorder is treated. In one embodiment, the TNFα antibody, or antigen binding fragment thereof, is D2E7. In another embodiment, the TNFα antibody is administered with at least one additional therapeutic agent.

Yet another aspect of the invention features a method for inhibiting human TNFα activity in a human subject suffering from a TNFα-related disorder, comprising administering a therapeutically effective amount of a TNFα antibody, or an antigen-binding fragment thereof, to the subject, wherein the antibody dissociates from human TNFα with a $K_d$ of 1 x $10^{-8}$ M or less and a $K_{off}$ rate constant of 1 x $10^{-3}$ $s^{-1}$ or less, both determined by surface plasmon resonance, and neutralizes human TNFα cytotoxicity in a standard in vitro L929 assay with an $IC_{50}$ of 1 x $10^{-7}$ M or less. In one embodiment, the TNFα antibody, or antigen-binding fragment thereof, is D2E7.

Yet another aspect of the invention includes a method of treating a subject suffering from a TNFα-related disorder, comprising administering a therapeutically effective amount of D2E7, or an antigen-binding fragment thereof, to the subject, such that the disease is treated.

Still another aspect of the invention includes a method of treating a subject suffering from a TNFα-related disorder, comprising administering a therapeutically effective amount of D2E7, or an antigen-binding fragment thereof, to the subject, such that the disease is treated.

In one embodiment of the invention, D2E7 (also referred to as HUMIRA® or adalimumab) is administered with at least one additional therapeutic agent.

Another aspect of the invention is a kit comprising a pharmaceutical composition comprising a TNFα antibody, or an antigen binding portion thereof, and a pharmaceutically acceptable carrier; and instructions for administering to a subject the TNFα antibody pharmaceutical composition for treating a subject who is suffering from a TNFα-related disorder. In one embodiment, the TNFα antibody, or an antigen binding portion thereof, is D2E7 (HUMIRA®).

## DETAILED DESCRIPTION OF THE INVENTION

This invention pertains to methods of treating TNFα-related disorders in which TNFα activity, e.g., human TNFα activity, is detrimental. The methods include administering to the subject a therapeutically effective amount of a TNFα inhibitor, such that the TNFα-related disorder is treated. The invention also pertains to methods wherein the TNFα inhibitor is administered in combination with another therapeutic agent to treat a TNFα-related disorder. Various aspects of the invention relate to treatment with antibodies and antibody fragments, and pharmaceutical compositions comprising a TNFα inhibitor, and a pharmaceutically acceptable carrier for the treatment of TNFα-related disorders.

*Definitions*

In order that the present invention may be more readily understood, certain terms are first defined.

The term "human TNFα" (abbreviated herein as hTNFα, or simply hTNF), as used herein, is intended to refer to a human cytokine that exists as a 17 kD secreted form and a 26 kD membrane associated form, the biologically active form of which is composed of a trimer of noncovalently bound 17 kD molecules. The structure of hTNFα is described further in, for example, Pennica, D., et al. (1984) Nature 312:724-729; Davis, J.M., et al. (1987) Biochemistry 26:1322-1326; and Jones, E.Y., et al. (1989) Nature 338:225-228. The term human TNFα is intended to include recombinant human TNFα (rhTNFα), which can be prepared by standard recombinant expression methods or purchased commercially (R & D Systems, Catalog No. 210-TA, Minneapolis, MN). TNFα is also referred to as TNF.

The term "TNFα inhibitor" includes agents which inhibit TNFα. Examples of TNFα inhibitors include etanercept (Enbrel®, Amgen), infliximab (Remicade®, Johnson and Jobnson), human anti-TNF monoclonal antibody (D2E7/HUMIRA®, Abbott Laboratories), CDP 571 (Celltech), and CDP 870 (Celltech) and other compounds which inhibit TNFα activity, such that when administered to a subject suffering from or at risk of suffering from a disorder in which TNFα activity is detrimental, the disorder is treated. In one embodiment, a TNFα inhibitor is a compound, excluding etanercept and infliximab, which inhibits TNFα activity. In another embodiment, the TNFα inhibitors of the invention are used to treat a TNFα-related disorder, as described in more detail in section II. In one embodiment, the TNFα inhibitor, excluding etanercept and infliximab, is used to treat a TNFα-related disorder. In another embodiment, the TNFα inhibitor, excluding etanercept and infliximab, is used to treat ankylosing spondylitis. The term also includes each of the anti-TNFα human antibodies and antibody portions described herein as well as those described in U.S. Patent Nos. 6,090,382; 6,258,562; 6,509,015, and in U.S. Patent Application Serial Nos. 09/801185 and 10/302356.

The term "antibody", as used herein, is intended to refer to immunoglobulin molecules comprised of four polypeptide chains, two heavy (H) chains and two light (L) chains inter-connected by disulfide bonds. Each heavy chain is comprised of a heavy chain variable region (abbreviated herein as HCVR or VH) and a heavy chain constant region. The heavy chain constant region is comprised of three domains, CH1, CH2 and CH3. Each light chain is comprised of a light chain variable region (abbreviated herein as LCVR or VL) and a light chain constant region. The light chain constant region is comprised of one domain, CL. The VH and VL regions can be further subdivided into regions of hypervariability, termed complementarity determining regions (CDR), interspersed with regions that are more conserved, termed framework regions (FR). Each VH and VL is composed of three CDRs and four FRs, arranged from amino-terminus to carboxy-terminus in the following order: FR1, CDR1, FR2, CDR2, FR3, CDR3, FR4. The antibodies of the invention are described in further detail in U.S. Patent Nos. 6,090,382; 6,258,562; and 6,509,015, and in U.S. Patent Application Serial Nos. 09/801185 and 10/302356, each of which is incorporated herein by reference in its entirety.

The term "antigen-binding portion" of an antibody (or simply "antibody portion"), as used herein, refers to one or more fragments of an antibody that retain the ability to specifically bind to an antigen (e.g., hTNFα). It has been shown that the antigen-binding function of an antibody can be performed by fragments of a full-length antibody. Examples of binding fragments encompassed within the term "antigen-binding portion" of an antibody include (i) a Fab fragment, a monovalent fragment consisting of the VL, VH, CL and CH1 domains; (ii) a F(ab')$_2$ fragment, a bivalent fragment comprising two Fab fragments linked by a disulfide bridge at the hinge region; (iii) a Fd fragment consisting of the VH and CH1 domains; (iv) a Fv fragment consisting of the VL and VH domains of a single arm of an antibody, (v) a dAb fragment (Ward et al., (1989) Nature 341:544-546), which consists of a VH domain; and (vi) an isolated complementarity determining region (CDR). Furthermore, although the two domains of the Fv fragment, VL and VH, are coded for by separate genes, they can be joined, using recombinant methods, by a synthetic linker that enables them to be made as a single protein chain in which the VL and VH regions pair to form monovalent molecules (known as single chain Fv (scFv); see *e.g.,* Bird et al. (1988) Science 242:423-426; and Huston et al. (1988) Proc. Natl. Acad. Sci. USA 85:5879-5883). Such single chain antibodies are also intended to be encompassed within the term "antigen-binding portion" of an antibody. Other forms of single chain antibodies, such as diabodies are also encompassed. Diabodies are bivalent, bispecific antibodies in which VH and VL domains are expressed on a single polypeptide chain, but using a linker that is too short to allow for pairing between the two domains on the same chain, thereby forcing the domains to pair with complementary domains of another chain and creating two antigen binding sites (see *e.g.,* Holliger, P., et al. (1993) Proc. Natl. Acad. Sci. USA 90:6444-6448; Poljak, R.J., et al. (1994) Structure 2:1121-1123). The antibody portions of the invention are described in further detail in U.S. Patent Nos. 6,090,382, 6,258,562, 6,509,015, and in U.S. Patent Application Serial Nos. 09/801185 and 10/302356, each of which is incorporated herein by reference in its entirety.

Binding fragments are produced by recombinant DNA techniques, or by enzymatic or chemical cleavage of intact immunoglobulins. Binding fragments include Fab, Fab', F(ab')$_2$, Fabc, Fv, single chains, and single-chain antibodies. Other than "bispecific" or "bifunctional" immunoglobulins or antibodies, an immunoglobulin or antibody is understood to have each of its binding sites identical. A "bispecific" or "bifunctional antibody" is an artificial hybrid antibody having two different heavy/light chain pairs and two different binding sites. Bispecific antibodies can be produced by a variety of methods including fusion of hybridomas or linking of Fab' fragments. See, *e.g.,* Songsivilai & Lachmann, Clin. Exp. Immunol. 79:315-321 (1990); Kostelny et al., J. Immunol. 148,1547-1553 (1992).

A "conservative amino acid substitution", as used herein, is one in which one amino acid residue is replaced with another amino acid residue having a similar side chain. Families of amino acid residues having similar side chains have been defined in the art, including basic side chains (e.g., lysine, arginine, histidine), acidic side chains (*e.g.*, aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (*e.g.*, tyrosine, phenylalanine, tryptophan, histidine).

The term "human antibody", as used herein, is intended to include antibodies having variable and constant regions derived from human germline immunoglobulin sequences. The human antibodies of the invention may include amino acid residues not encoded by human germline immunoglobulin sequences (*e.g.*, mutations introduced by random or site-specific mutagenesis *in vitro* or by somatic mutation *in vivo*), for example in the CDRs and in particular CDR3. However, the term "human antibody", as used herein, is not intended to include antibodies in which CDR sequences derived from the germline of another mammalian species, such as a mouse, have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes (see *e.g.,* Taylor, L.D. et al. (1992) Nucl. Acids Res. 20:6287) or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to *in vitro* mutagenesis (or, when an animal transgenic for human Ig sequences is used, *in vivo* somatic mutagenesis) and thus the amino acid sequences of the VH and VL regions of the recombinant antibodies are sequences that, while derived from and related to human germline VH and VL sequences, may not naturally exist within the human antibody germline repertoire *in vivo.*

An "isolated antibody", as used herein, is intended to refer to an antibody that is substantially free of other antibodies having different antigenic specificities (e.g., an isolated antibody that specifically binds hTNFα is substantially free of antibodies that specifically bind antigens other than hTNFα). An isolated antibody that specifically binds hTNFα may, however, have cross-reactivity to other antigens, such as TNFα molecules from other species (discussed in further detail below). Moreover, an isolated antibody may be substantially free of other cellular material and/or chemicals.

A "neutralizing antibody", as used herein (or an "antibody that neutralized hTNFα activity"), is intended to refer to an antibody whose binding to hTNFα results in inhibition of the biological activity of hTNFα. This inhibition of the biological activity of hTNFα can be assessed by measuring one or more indicators of hTNFα biological activity, such as hTNFα-induced cytotoxicity (either *in vitro* or in *vivo*), hTNFα-induced cellular activation and hTNFα binding to hTNFα receptors. These indicators of hTNFα biological activity can be assessed by one or more of several standard *in vitro* or *in vivo* assays known in the art (see U.S. Patent No. 6,090,382). Preferably, the ability of an antibody to neutralize hTNFα activity is assessed by inhibition of hTNFα-induced cytotoxicity of L929 cells. As an additional or alternative parameter of hTNFα activity, the ability of an antibody to inhibit hTNFα-induced expression of ELAM-1 on HUVEC, as a measure of hTNFα-induced cellular activation, can be assessed.

The term "surface plasmon resonance", as used herein, refers to an optical phenomenon that allows for the analysis of real-time bispecific interactions by detection of alterations in protein concentrations within a biosensor matrix, for example using the BIAcore system (Pharmacia Biosensor AB, Uppsala, Sweden and Piscataway, NJ). For further descriptions, see Example 1 of U.S. Patent 6,258,562 and Jönsson et al. (1993) Ann. Biol Clin. 51:19; Jönsson et al. (1991) Biotechniques 11:620-627; Johnsson et al. (1995) J. Mol. Recognit. 8:125; and Johnnson et al. (1991) Anal. Biochem.198:268.

The tonn "$K_{off}$", as used herein, is intended to refer to the off rate constant for dissociation of an antibody from the antibody/antigen complex.

The term "$K_d$", as used herein, is intended to refer to the dissociation constant of a particular antibody-antigen interaction.

The term "$IC_{50}$" as used herein, is intended to refer to the concentration of the inhibitor required to inhibit the biological endpoint of interest, e.g., neutralize cytotoxicity activity.

The term "nucleic acid molecule", as used herein, is intended to include DNA molecules and RNA molecules. A nucleic acid molecule may be single-stranded or double-stranded, but preferably is double-stranded DNA.

The term "isolated nucleic acid molecule", as used herein in reference to nucleic acids encoding antibodies or antibody portions (e.g., VH, VL, CDR3) that bind hTNFα, is intended to refer to a nucleic acid molecule in which the nucleotide sequences encoding the antibody or antibody portion are free of other nucleotide sequences encoding antibodies or antibody portions that bind antigens other than hTNFα, which other sequences may naturally flank the nucleic acid in human genomic DNA. Thus, for example, an isolated nucleic acid of the invention encoding a VH region of an anti-hTNFα antibody contains no other sequences encoding other VH regions that bind antigens other than hTNFα.

The term "vector", as used herein, is intended to refer to a nucleic acid molecule capable of transporting another nucleic acid to which it has been linked. One type of vector is a "plasmid", which refers to a circular double stranded DNA loop into which additional DNA segments may be ligated. Another type of vector is a viral vector, wherein additional DNA segments may be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. Moreover, certain vectors are capable of directing the expression of genes to which they are operatively linked. Such vectors are referred to herein as "recombinant expression vectors" (or simply, "expression vectors"). In general, expression vectors of utility in recombinant DNA techniques are often in the form of plasmids. In the present specification, "plasmid" and "vector" may be used interchangeably as the plasmid is the most commonly used form of vector. However, the invention is intended to include such other forms of expression vectors, such as viral vectors (e.g., replication defective retroviruses, adenoviruses and adeno-associated viruses), which serve equivalent functions.

The term "recombinant host cell" (or simply "host cell"), as used herein, is intended to refer to a cell into which a recombinant expression vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein.

The term "dosing", as used herein, refers to the administration of a substance (e.g., an anti-TNFα antibody) to achieve a therapeutic objective (e.g., the treatment of a TNFα-associated disorder).

The terms "biweekly dosing regimen", "biweekly dosing", and "biweekly administration", as used herein, refer to the time course of administering a substance (e.g., an anti-TNFα antibody) to a subject to achieve a therapeutic objective (e.g., the treatment of a TNFα-associated disorder). The biweekly dosing regimen is not intended to include a weekly dosing regimen. Preferably, the substance is administered every 9-19 days, more preferably, every 11-17 days, even more preferably, every 13-15 days, and most preferably, every 14 days.

The term "combination" as in the phrase "a first agent in combination with a second agent" includes co-administration of a first agent and a second agent, which for example may be dissolved or intermixed in the same pharmaceutically acceptable carrier, or administration of a first agent, followed by the second agent, or administration of the second agent, followed by the first agent. The present invention, therefore, includes methods of combination therapeutic treatment and combination pharmaceutical compositions.

The term "concomitant" as in the phrase "concomitant therapeutic treatment" includes administering an agent in the presence of a second agent. A concomitant therapeutic treatment method includes methods in which the first, second, third, or additional agents are co-administered. A concomitant therapeutic treatment method also includes methods in which the first or additional agents are administered in the presence of a second or additional agents, wherein the second or additional agents, for example, may have been previously administered. A concomitant therapeutic treatment method may be executed step-wise by different actors. For example, one actor may administer to a subject a first agent and a second actor may to administer to the subject a second agent, and the administering steps may be executed at the same time, or nearly the same time, or at distant times, so long as the first agent (and additional agents) are after administration in the presence of the second agent (and additional agents). The actor and the subject may be the same entity (e.g., human).

The term "combination therapy", as used herein, refers to the administration of two or more therapeutic substances, e.g., an anti-TNFα antibody and another drug, such as a DMARD or NSAID. The other drug(s) may be administered concomitant with, prior to, or following the administration of an anti-TNFα antibody.

The term "TNFα-mediated condition" or "TNFα-related disorder" refers to a local and/or systemic physiological disorder where TNFα is a primary mediator leading to the manifestation of the disorder.

The term "inflammatory disorder" or "inflammatory disease," as used interchangeably herein, refers to an inflammation-mediated malady, whether or not also immune mediated. Inflammatory disorders are disorders in which an excessive or unregulated inflammatory response leads to excessive inflammatory symptoms, host tissue damage, or loss of tissue function. Examples include rheumatoid arthritis and spondyloarthropathies. In one embodiment, the inflammatory disorder of the invention refers to an inflammation-mediated malady excluding osteoarthritis and rheumatoid spondylitis.

The term "pulmonary disease" as used herein refers to any idiopathic interstitial lung disease and/or chronic obstructive airway disorder. In one embodiment of the invention, the term pulmonary disease includes any lung disease and/or chronic obstructive airway disorder excluding shock lung, chronic pulmonary inflammatory disease, pulmonary sacroidosis, pulmonary fibrosis, and silicosis.

The term "idiopathic interstitial lung disease" or "idiopathic interstitial lung disorder," as used interchangeably herein, refers to any one of several diseases of unknown etiology with similar clinical features, producing diffuse pathologic changes primarily in interalveolar interstitial tissue. Examples of idiopathic interstitial lung diseases include, but are not limited to, interstitial pulmonary fibrosis (IPF). In one embodiment, idiopathic interstitial lung diseases include any one of several diseases of unknown etiology with similar clinical features, producing diffuse pathologic changes primarily in interalveolar interstitial tissue but exclude shock lung, chronic pulmonary inflammatory disease, pulmonary sacroidosis, pulmonary fibrosis, and silicosis.

The term "chronic obstructive airway disorder" as used herein, refers to pulmonary diseases due to physiologically determined chronic airflow obstruction, regardless of etiology. Examples of chronic obstructive airway disorders include, but are not limited to, asthma and chronic obstructive pulmonary disease (COPD). In one embodiment, the term chronic obstructive airway disorder includes pulmonary diseases due to physiologically determined chronic airflow obstruction but excludes shock lung, chronic pulmonary inflammatory disease, pulmonary sacroidosis, pulmonary fibrosis, and silicosis

The term "airway obstruction" refers to an increased resistance to airflow exhibited by characteristic spirometric findings.

The term "cardiovascular disorder" or "coronary disorder" as used interchangeably herein, refers to any disease, disorder, or state involving the cardiovascular system, e.g., the heart, the blood vessels, and/or the blood. A coronary disorder is generally characterized by a narrowing of the blood vessels that supply blood and oxygen to the heart (coronary arteries). Coronary disease usually results from the build up of fatty material and plaque. As the coronary arteries narrow, the flow of blood to the heart can slow or stop. Coronary disorders of the invention can apply to any abnormality of an artery, whether structural, histological, biochemical or any other abnormality. An example of coronary heart disease is restenosis. In one embodiment, a coronary disorder refers to any disease, disorder, or state involving the cardiovascular system excluding ischemia of the heart and heart insufficiency.

The term "restenosis" as used herein refers to the recurrence of stenosis, which is the narrowing or constriction of an artery. Restenosis often occurs as a preocclusive lesion that develops following a reconstructive procedure in a diseased blood vessel. The term is not only applied to the recurrence of a pre-existing stenosis, but also to previously normal vessels that become partially occluded following vascular bypass. In another embodiment, the invention provides a method of treating restenosis comprising administering the antibody, or antigen binding portion thereof, of the invention to a subject who has or is at risk of developing restenosis.

The term "stent" as used herein refers to a structure that is inserted into the lumen of an anatomical vessel, e.g. an artery, especially to keep a formerly blocked passageway open. Stent is used to maintain the flow of fluids (e.g., blood) from one portion of a vessel to another, and an endovascular scaffolding or stent which holds open a body passageway and/or supports the graft or wrap. A stent is often used following balloon angioplasty, although they can also be used as direct therapy for treating stenosis.

In one embodiment of the invention, the stent is drug-eluting. The term "drug-eluting" refers to a stent which is coated with a slow-to-moderate release drug formulation. The terms "drug-eluting" or "drug-releasing" or "drug-coated" are used interchangeably herein. A stent can be coated with any drug which treats coronary heart disease, including, for example, the antibody, or antigen-binding fragment thereof, of the invention. In another embodiment, the stent delivers D2E7. In a further embodiment, the stent delivers D2E7 in combination with another drug used to treat coronary disorders, including dexamethasone, alkeran, cytoxan, leukeran, cis-platinum, BiCNU, adriamycin, doxorubicin, cerubidine, idamycin, mithracin, mutamycin, fluorouracil, methotrexate, thoguanine, toxotere, etoposide, vincristine, irinotecan, hycamptin, matulane, vumon, hexalin, hydroxyurea, gemzar, oncovin, etophophos, tacrolimus (FK506), and the following analogs of sirolimus: SDZ-RAD, CCI-779, 7-epi-rapamycin, 7-thiomethyl-rapamycin, 7-epi-trimethoxyphenyl- rapamycin, 7-epi-thiomethyl-rapamycin, 7-demethoxy-rapamycin, 32-demethoxy, 2-desmethyl and proline.

The term "metabolic disorder," as used herein, refers to diseases or disorders which affect how the body processes substances needed to carry out physiological functions. Examples of metabolic disorders include, but are not limited to, diabetes and obesity. In one embodiment of the invention, the term "metabolic disorder" is used to refer to disorders which affect how the body processes substances needed to carry out physiological functions, excluding autoimmune diabetes.

The term "diabetes" or "diabetic disorder" or "diabetes mellitus," as used interchangeably herein, refers to a disease which is marked by elevated levels of sugar (glucose) in the blood. Diabetes can be caused by too little insulin (a chemical produced by the pancreas to regulate blood sugar), resistance to insulin, or both.

The phrase "disorders associated with diabetes," as used herein, refers to conditions and other diseases which are commonly associated with or related to diabetes. Example of disorders associated with diabetes include, for example, hyperglycemia, hyperinsulinaemia, hyperlipidaemia, insulin resistance, impaired glucose metabolism, obesity, diabetic retinopathy, macular degeneration, cataracts, diabetic nephropathy, glomerulosclerosis, diabetic neuropathy, erectile dysfunction, premenstrual syndrome, vascular restenosis, ulcerative colitis, coronary heart disease, hypertension, angina pectoris, myocardial infarction, stroke, skin and connective tissue disorders, foot ulcerations, metabolic acidosis, arthritis, and osteoporosis.

The term "obesity" as used herein, refers to a condition in which the subject has an excess of body fat relative to lean body mass. In one embodiment, obesity refers to a condition in which an individual weighs at least about 20% or more over the maximum desirable for their height. When an adult is more than 100 pounds overweight, he or she is considered to be "morbidly obese." In another embodiment, obesity is defined as a BMI (body mass index) over 30 kg/m2.

The term "anemia" as used herein, refers to an abnormally low number of circulating red cells or a decreased concentration of hemoglobin in the blood.

The term "pain" as used herein, refers to all types of pain. The term shall refer to acute and chronic pains, such as neuropathic pain and post-operative pain, chronic lower back pain, cluster headaches, herpes neuralgia, phantom limb pain, central pain, dental pain, opioid-resistant pain, visceral pain, surgical pain, bone injury pain, pain during labor and delivery, pain resulting from bums, including sunburn, post partum pain, migraine, angina pain, and genitourinary tract-related pain including cystitis. The term also includes nociceptive pain or nociception.

As used herein, the term "hepatic disorder" refers to a mammalian and preferably a human liver disease or condition associated with hepatocellular injury or a biliary tract disorder. In one embodiment, hepatic disorders refers to a human liver disease or condition associated with hepatocellular injury or a biliary tract disorder excluding hepatitis, alcoholic hepatitis, and viral hepatitis.

The term "skin disorder" or "skin disease" as used interchangeably herein, refers to abnormalities, other than injury wounds, of the skin which have induced a state of inflammation. In one embodiment, the skin disorder of the invention is an inflammatory skin disorder, wherein the skin is characterized by capillary dilatation, leukocytic infiltration, redness, heat, and/or pain. Examples of skin disorders include, but are not limited to, psoriasis, pemphigus vulgaris, scleroderma, atopic dermatitis, sarcoidosis, erythema nodosum, hidradenitis suppurative, lichen planus, Sweet's syndrome, and vitiligo.

The term "psoriasis" as used herein, refers to skin disorders associated with epidermal hyperplasia. Example of psoriasis include, but are not limited to, chronic plaque psoriasis, guttate psoriasis, inverse psoriasis, pustular psoriasis, psoriasis vulgaris, and erythrodermic psoriasis. Psoriasis can also be associated with other inflammatory disorders, including inflammatory bowel disease (IBD) and rheumatoid arthritis (RA).

The term "healthy skin" or "normal skin" refers to non-lesional skin, *i.e.*, with no visually obvious erythema, edema, hyper-, hypo-, or uneven pigmentations, scale formation, xerosis, or blister formation. Histologically, healthy or normal skin refers to skin tissue with a morphological appearance comprising well-organized basal, spinous, and granular layers, and a coherent multi-layered stratum corneum.

The term "nail disorder" or "nail disease" as used herein, refers to conditions wherein the fingernails or toenails to abnormal color, shape, texture, or thickness.

The term "vasculitis" or "vasculitides" as used interchangeably herein, refers to a group of disorders which are characterized by the inflammation of blood vessels. Blood vessels of all sizes may be affected, from the largest vessel in the body (the aorta) to the smallest blood vessels in the skin (capillaries). The size of blood vessel affected varies according to the specific type of vasculitis.

The term "kit" as used herein refers to a packaged product comprising components with which to administer the TNFα antibody of the invention for treatment of a TNFα-related disorder. The kit preferably comprises a box or container that holds the components of the kit. The box or container is affixed with a label or a Food and Drug Administration approved protocol. The box or container holds components of the invention which are preferably contained within plastic, polyethylene, polypropylene, ethylene, or propylene vessels. The vessels can be capped-tubes or bottles. The kit can also include instructions for administering the TNFα antibody of the invention.

Various aspects of the invention are described in further detail herein.

I. TNFα Inhibitors of the Invention

This invention provides a method of treating a TNFα-related disorder in which the administration of a TNFα inhibitor is beneficial. In one embodiment, these methods include administration of isolated human antibodies, or antigen-binding portions thereof, that bind to human TNFα with high affinity and a low off rate, and have a high neutralizing capacity. Preferably, the human antibodies of the invention are recombinant, neutralizing human anti-hTNFα antibodies. The most preferred recombinant, neutralizing antibody of the invention is referred to herein as D2E7, also referred to as HUMIRA® and adalimumab (the amino acid sequence of the D2E7 VL region is shown in SEQ ID NO: 1; the amino acid sequence of the D2E7 VH region is shown in SEQ ID NO: 2). The properties of D2E7 (HUMIRA®) have been described in Salfeld et al., U.S. patent No. 6,090,382, which is incorporated by reference herein.

In one embodiment, the treatment of the invention includes the administration of D2E7 antibodies and antibody portions, D2E7-related antibodies and antibody portions, and other human antibodies and antibody portions with equivalent properties to D2E7, such as high affinity binding to hTNFα with low dissociation kinetics and high neutralizing capacity. In one embodiment, the invention provides treatment with an isolated human antibody, or an antigen-binding portion thereof, that dissociates from human TNFα with a $K_d$ of 1 x $10^{-8}$ M or less and a $K_{off}$ rate constant of 1 x $10^{-3}$ $s^{-1}$ or less, both determined by surface plasmon resonance, and neutralizes human TNFα cytotoxicity in a standard *in vitro* L929 assay with an $IC_{50}$ of 1 x $10^{-7}$ M or less. More preferably, the isolated human antibody, or antigen-binding portion thereof, dissociates from human TNFα with a $K_{off}$ of 5 x $10^{-4}$ $s^{-1}$ or less, or even more preferably, with a $K_{off}$ of 1 x $10^{-4}$ $s^{-1}$ or less. More preferably, the isolated human antibody, or antigen-binding portion thereof, neutralizes human TNFα cytotoxicity in a standard *in vitro* L929 assay with an $IC_{50}$ of 1 x $10^{-8}$ M or less, even more preferably with an $IC_{50}$ of 1 x $10^{-9}$ M or less and still more preferably with an $IC_{50}$ of 1 x $10^{-10}$ M or less. In a preferred embodiment, the antibody is an isolated human recombinant antibody, or an antigen-binding portion thereof.

It is well known in the art that antibody heavy and light chain CDR3 domains play an important role in the binding specificity/affinity of an antibody for an antigen. Accordingly, in another aspect, the invention pertains to methods of treating a TNFα-related disorder in which the TNFα activity is detrimental by administering human antibodies that have slow dissociation kinetics for association with hTNFα and that have light and heavy chain CDR3 domains that structurally are identical to or related to those of D2E7. Position 9 of the D2E7 VL CDR3 can be occupied by Ala or Thr without substantially affecting the $K_{off}$. Accordingly, a consensus motif for the D2E7 VL CDR3 comprises the amino acid sequence: Q-R-Y-N-R-A-P-Y-(T/A) (SEQ ID NO: 3). Additionally, position 12 of the D2E7 VH CDR3 can be occupied by Tyr or Asn, without substantially affecting the $K_{off}$. Accordingly, a consensus motif for the D2E7 VH CDR3 comprises the amino acid sequence: V-S-Y-L-S-T-A-S-S-L-D-(Y/N) (SEQ ID NO: 4). Moreover, as demonstrated in Example 2 of U.S. Patent No. 6,090,382, the CDR3 domain of the D2E7 heavy and light chains is amenable to substitution with a single alanine residue (at position 1, 4, 5, 7 or 8 within the VL CDR3 or at position 2, 3, 4, 5, 6, 8, 9, 10 or 11 within the VH CDR3) without substantially affecting the $K_{off}$. Still further, the skilled artisan will appreciate that, given the amenability of the D2E7 VL and VH CDR3 domains to substitutions by alanine, substitution of other amino acids within the CDR3 domains may be possible while still retaining the low off rate constant of the antibody, in particular substitutions with conservative amino acids. Preferably, no more than one to five conservative amino acid substitutions are made within the D2E7 VL and/or VH CDR3 domains. More preferably, no more than one to three conservative amino acid substitutions are made within the D2E7 VL and/or VH CDR3 domains. Additionally, conservative amino acid substitutions should not be made at amino acid positions critical for binding to hTNFα. Positions 2 and 5 of the D2E7 VL CDR3 and positions 1 and 7 of the D2E7 VH CDR3 appear to be critical for interaction with hTNFα and thus, conservative amino acid substitutions preferably are not made at these positions (although an alanine substitution at position 5 of the D2E7 VL CDR3 is acceptable, as described above) (*see* U.S. Patent No. 6,090,382).

Accordingly, in another embodiment, the invention provides methods of treating a TNFα-related disorder by the administration of an isolated human antibody, or antigen-binding portion thereof. The antibody or antigen-binding portion thereof preferably contains the following characteristics:

a) dissociates from human TNFα with a $K_{off}$ rate constant of 1 x $10^{-3}$ $s^{-1}$ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7,8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11 or by one to five conservative amino acid substitutions at positions 2, 3, 4, 5, 6, 8, 9, 10, 11 and/or 12.

More preferably, the antibody, or antigen-binding portion thereof, dissociates from human TNFα with a $K_{off}$ of 5 x $10^{-4}$ $s^{-1}$ or less. Even more preferably, the antibody, or antigen-binding portion thereof, dissociates from human TNFα with a $K_{off}$ of 1 x $10^{-4}$ $s^{-1}$ or less.

In yet another embodiment, the invention provides methods of treating a TNFα-related disorder by the administration of an isolated human antibody, or antigen-binding portion thereof. The antibody or antigen-binding portion thereof preferably contains a light chain variable region (LCVR) having a CDR3 domain comprising the amino acid sequence of SEQ ID NO: 3, or modified from SEQ ID NO: 3 by a single alanine substitution at position 1, 4, 5, 7 or 8, and with a heavy chain variable region (HCVR) having a CDR3 domain comprising the amino acid sequence of SEQ ID NO: 4, or modified from SEQ ID NO: 4 by a single alanine substitution at position 2, 3, 4, 5, 6, 8, 9, 10 or 11. Preferably, the LCVR further has a CDR2 domain comprising the amino acid sequence of SEQ ID NO: 5 (*i.e.*, the D2E7 VL CDR2) and the HCVR further has a CDR2 domain comprising the amino acid sequence of SEQ ID NO: 6 (*i.e.*, the D2E7 VH CDR2). Even more preferably, the LCVR further has CDR1 domain comprising the amino acid sequence of SEQ ID NO: 7 (*i.e.*, the D2B7 VL CDR1) and the HCVR has a CDR1 domain comprising the amino acid sequence of SEQ ID NO: 8 (*i.e.*, the D2E7 VH CDR1). The framework regions for VL preferably are from the V$_\kappa$I human germline family, more preferably from the A20 human germline Vk gene and most preferably from the D2E7 VL framework sequences shown in Figures 1A and 1B of U.S. Patent No. 6,090,382. The framework regions for VH preferably are from the V$_H$3 human germline family, more preferably from the DP-31 human germline VH gene and most preferably from the D2E7 VH framework sequences shown in Figures 2A and 2B of U.S. Patent No. 6,090,382.

Accordingly, in another embodiment, the invention provides methods of treating a TNFα-related disorder by the administration of an isolated human antibody, or antigen-binding portion thereof. The antibody or antigen-binding portion thereof preferably contains a light chain variable region (LCVR) comprising the amino acid sequence of SEQ ID NO: 1 (*i.e.*, the D2E7 VL) and a heavy chain variable region (HCVR) comprising the amino acid sequence of SEQ ID NO: 2 (*i.e.*, the D2E7 VH). In certain embodiments, the antibody comprises a heavy chain constant region, such as an IgG1, IgG2, IgG3, IgG4, IgA, IgE, IgM or IgD constant region. Preferably, the heavy chain constant region is an IgG1 heavy chain constant region or an IgG4 heavy chain constant region. Furthermore, the antibody can comprise a light chain constant region, either a kappa light chain constant region or a lambda light chain constant region. Preferably, the antibody comprises a kappa light chain constant region. Alternatively, the antibody portion can be, for example, a Fab fragment or a single chain Fv fragment.

In still other embodiments, the invention provides methods of treating a TNFα-related disorder in which the administration of an anti-TNFα antibody is beneficial administration of an isolated human antibody, or an antigen-binding portions thereof. The antibody or antigen-binding portion thereof preferably contains D2E7-related VL and VH CDR3 domains, for example, antibodies, or antigen-binding portions thereof, with a light chain variable region (LCVR) having a CDR3 domain comprising an amino acid sequence selected from the group consisting of SEQ ID NO: 3, SBQ ID NO: 11, SEQ ID NO: 12, SEQ ID NO: 13, SEQ ID NO: 14, SEQ ID NO: 15, SEQ ID NO: 16, SEQ ID NO: 17, SEQ ID NO:18, SEQ ID NO: 19, SEQ ID NO: 20, SEQ ID NO: 21, SEQ ID NO: 22, SEQ ID NO: 23, SEQ ID NO: 24, SEQ ID NO: 25 and SEQ ID NO: 26 or with a heavy chain variable region (HCVR) having a CDR3 domain comprising an amino acid sequence selected from the group consisting of SEQ ID NO: 4, SEQ ID NO: 27, SEQ ID NO: 28, SEQ ID NO: 29, SEQ ID NO: 30, SEQ ID NO: 31, SEQ ID NO: 32, SEQ ID NO: 33, SEQ ID NO: 34 and SEQ ID NO: 35.

In another embodiment, the TNFα inhibitor of the invention is etanercept (described in WO 91/03553 and WO 09/406476), infliximab (described in U.S. Patent No. 5,656,272), CDP571 (a humanized monoclonal anti-TNF-alpha IgG4 antibody), CDP 870 (a humanized monoclonal anti-TNF-alpha antibody fragment), D2E7 (a human anti-TNF mAb), soluble TNF receptor Type I, or a pegylated soluble TNF receptor Type I (PEGs TNF-R1).

The TNFα antibody of the invention can be modified. In some embodiments, the TNFα antibody or antigen binding fragments thereof, is chemically modified to provide a desired effect. For example, pegylation of antibodies and antibody fragments of the invention may be carried out by any of the pegylation reactions known in the art, as described, for example, in the following references: Focus on Growth Factors 3:4-10 (1992); EP 0 154 316; and EP 0 401 384 (each of which is incorporated by reference herein in its entirety). Preferably, the pegylation is carried out via an acylation reaction or an alkylation reaction with a reactive polyethylene glycol molecule (or an analogous reactive water-soluble polymer). A preferred water-soluble polymer for pegylation of the antibodies and antibody fragments of the invention is polyethylene glycol (PEG). As used herein, "polyethylene glycol" is meant to encompass any of the forms of PEG that have been used to derivatize other proteins, such as mono (C1-C10) alkoxy- or aryloxy-polyethylene glycol.

Methods for preparing pegylated antibodies and antibody fragments of the invention will generally comprise the steps of (a) reacting the antibody or antibody fragment with polyethylene glycol, such as a reactive ester or aldehyde derivative of PEG, under conditions whereby the antibody or antibody fragment becomes attached to one or more PEG groups, and (b) obtaining the reaction products. It will be apparent to one of ordinary skill in the art to select the optimal reaction conditions or the acylation reactions based on known parameters and the desired result.

Pegylated antibodies and antibody fragments may generally be used to treat TNFα-related disorders of the invention by administration of the TNFα antibodies and antibody fragments described herein. Generally the pegylated antibodies and antibody fragments have increased half-life, as compared to the nonpegylated antibodies and antibody fragments. The pegylated antibodies and antibody fragments may be employed alone, together, or in combination with other pharmaceutical compositions.

In yet another embodiment of the invention, TNFα antibodies or fragments thereof can be altered wherein the constant region of the antibody is modified to reduce at least one constant region-mediated biological effector function relative to an unmodified antibody. To modify an antibody of the invention such that it exhibits reduced binding to the Fc receptor, the immunoglobulin constant region segment of the antibody can be mutated at particular regions necessary for Fc receptor (FcR) interactions (see *e.g.*, Canfield, S.M. and S.L. Morrison (1991) J. Exp. Med. 173:1483-1491; and Lund, J. et al. (1991) J. of Immunol. 147:2657-2662). Reduction in FcR binding ability of the antibody may also reduce other effector functions which rely on FcR interactions, such as opsonization and phagocytosis and antigen-dependent cellular cytotoxicity.

An antibody or antibody portion of the invention can be derivatized or linked to another functional molecule (e.g., another peptide or protein). Accordingly, the antibodies and antibody portions of the invention are intended to include derivatized and otherwise modified forms of the human anti-hTNFα antibodies described herein, including immunoadhesion molecules. For example, an antibody or antibody portion of the invention can be functionally linked (by chemical coupling, genetic fusion, noncovalent association or otherwise) to one or more other molecular entities, such as another antibody (*e.g.,* a bispecific antibody or a diabody), a detectable agent, a cytotoxic agent, a pharmaceutical agent, and/or a protein or peptide that can mediate associate of the antibody or antibody portion with another molecule (such as a streptavidin core region or a polyhistidine tag).

One type of derivatized antibody is produced by crosslinking two or more antibodies (of the same type or of different types, e.g., to create bispecific antibodies). Suitable crosslinkers include those that are heterobifunctional, having two distinctly reactive groups separated by an appropriate spacer (*e.g.,* m-maleimidobenzoyl-N-hydroxysuccinimide ester) or homobifunctional (*e.g.,* disuccinimidyl suberate). Such linkers are available from Pierce Chemical Company, Rockford, IL.

Useful detectable agents with which an antibody or antibody portion of the invention may be derivatized include fluorescent compounds. Exemplary fluorescent detectable agents include fluorescein, fluorescein isothiocyanate, rhodamine, 5-dimethylamine-1-napthalenesulfonyl chloride, phycoerythrin and the like. An antibody may also be derivatized with detectable enzymes, such as alkaline phosphatase, horseradish peroxidase, glucose oxidase and the like. When an antibody is derivatized with a detectable enzyme, it is detected by adding additional reagents that the enzyme uses to produce a detectable reaction product. For example, when the detectable agent horseradish peroxidase is present, the addition of hydrogen peroxide and diaminobenzidine leads to a colored reaction product, which is detectable. An antibody may also be derivatized with biotin, and detected through indirect measurement of avidin or streptavidin binding.

An antibody, or antibody portion, of the invention can be prepared by recombinant expression of immunoglobulin light and heavy chain genes in a host cell. To express an antibody recombinantly, a host cell is transfected with one or more recombinant expression vectors carrying DNA fragments encoding the immunoglobulin light and heavy chains of the antibody such that the light and heavy chains are expressed in the host cell and, preferably, secreted into the medium in which the host cells are cultured, from which medium the antibodies can be recovered. Standard recombinant DNA methodologies are used to obtain antibody heavy and light chain genes, incorporate these genes into recombinant expression vectors and introduce the vectors into host cells, such as those described in Sambrook, Fritsch and Maniatis (eds), Molecular Cloning; A Laboratory Manual, Second Edition, Cold Spring Harbor, N.Y., (1989), Ausubel, F.M. et al. (eds.) Current Protocols in Molecular Biology, Greene Publishing Associates, (1989) and in U.S. Patent No. 4,816,397 by Boss et al.

To express D2E7 or a D2E7-related antibody, DNA fragments encoding the light and heavy chain variable regions are first obtained. These DNAs can be obtained by amplification and modification of germline light and heavy chain variable sequences using the polymerase chain reaction (PCR). Germline DNA sequences for human heavy and light chain variable region genes are known in the art (see *e.g.,* the "Vbase" human germline sequence database; see also Kabat, E.A., et al. (1991) Sequences of Proteins of Immunological Interest, Fifth Edition, U.S. Department of Health and Human Services, NIH Publication No. 91-3242; Tomlinson, I.M., et al. (1992) "The Repertoire of Human Germline VH Sequences Reveals about Fifty Groups of YH Segments with Different Hypervariable Loops" J. Mol. Biol. 227:776-798; and Cox, J.P.L. et al. (1994) "A Directory of Human Germ-line V78 Segments Reveals a Strong Bias in their Usage" Eur. J. Immunol. 24:827-836; the contents of each of which are expressly incorporated herein by reference). To obtain a DNA fragment encoding the heavy chain variable region of D2E7, or a D2E7-related antibody, a member of the $V_H3$ family of human germline $V_H$ genes is amplified by standard PCR. Most preferably, the DP-31 VH germline sequence is amplified. To obtain a DNA fragment encoding the light chain variable region of D2E7, or a D2E7-related antibody, a member of the $V_\kappa I$ family of human germline VL genes is amplified by standard PCR. Most preferably, the A20 VL germline sequence is amplified. PCR primers suitable for use in amplifying the DP-31 germline VH and A20 germline VL sequences can be designed based on the nucleotide sequences disclosed in the references cited *supra,* using standard methods.

Once the germline $V_H$ and VL fragments are obtained, these sequences can be mutated to encode the D2E7 or D2E7-related amino acid sequences disclosed herein. The amino acid sequences encoded by the germline VH and VL DNA sequences are first compared to the D2E7 or D2E7-related VH and VL amino acid sequences to identify amino acid residues in the D2E7 or D2E7-related sequence that differ from germline. Then, the appropriate nucleotides of the germline DNA sequences are mutated such that the mutated germline sequence encodes the D2E7 or D2E7-related amino acid sequence, using the genetic code to determine which nucleotide changes should be made. Mutagenesis of the germline sequences is carried out by standard methods, such as PCR-mediated mutagenesis (in which the mutated nucleotides are incorporated into the PCR primers such that the PCR product contains the mutations) or site-directed mutagenesis.

Once DNA fragments encoding D2E7 or D2E7-related VH and VL segments are obtained (by amplification and mutagenesis of germline VH and VL genes, as described above), these DNA fragments can be further manipulated by standard recombinant DNA techniques, for example to convert the variable region genes to full-length antibody chain genes, to Fab fragment genes or to a scFv gene. In these manipulations, a VL- or VH-encoding DNA fragment is operatively linked to another DNA fragment encoding another protein, such as an antibody constant region or a flexible linker. The term "operatively linked", as used in this context, is intended to mean that the two DNA fragments are joined such that the amino acid sequences encoded by the two DNA fragments remain in-frame.

The isolated DNA encoding the VH region can be converted to a full-length heavy chain gene by operatively linking the VH-encoding DNA to another DNA molecule encoding heavy chain constant regions (CH1, CH2 and CH3). The sequences of human heavy chain constant region genes are known in the art (see e.g., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The heavy chain constant region can be an IgG1, IgG2, IgG3, IgG4, IgA, IgE, IgM or IgD constant region, but most preferably is an IgG1 or IgG4 constant region. For a Fab fragment heavy chain gene, the VH-encoding DNA can be operatively linked to another DNA molecule encoding only the heavy chain CH1 constant region.

The isolated DNA encoding the VL region can be converted to a full-length light chain gene (as well as a Fab light chain gene) by operatively linking the VL-encoding DNA to another DNA molecule encoding the light chain constant region, CL. The sequences of human light chain constant region genes are known in the art (see e.g., Kabat, E.A., et al. (1991) Sequences of Proteins of Immunological Interest, Fifth Edition, U.S. Department of Health and Human Services, NIH Publication No. 91-3242) and DNA fragments encompassing these regions can be obtained by standard PCR amplification. The light chain constant region can be a kappa or lambda constant region, but most preferably is a kappa constant region.

To create a scFv gene, the VH- and VL-encoding DNA fragments are operatively linked to another fragment encoding a flexible linker, e.g., encoding the amino acid sequence $(Gly_4-Ser)_3$, such that the VH and VL sequences can be expressed as a contiguous single-chain protein, with the VL and VH regions joined by the flexible linker (see e.g., Bird et al. (1988) Science 242:423-426; Huston et al. (1988) Proc. Natl. Acad. Sci. USA 85:5879-5883; McCafferty et al., Nature (1990) 348:552-554).

To express the antibodies, or antibody portions of the invention, DNAs encoding partial or full-length light and heavy chains, obtained as described above, are inserted into expression vectors such that the genes are operatively linked to transcriptional and translational control sequences. In this context, the term "operatively linked" is intended to mean that an antibody gene is ligated into a vector such that transcriptional and translational control sequences within the vector serve their intended function of regulating the transcription and translation of the antibody gene. The expression vector and expression control sequences are chosen to be compatible with the expression host cell used. The antibody light chain gene and the antibody heavy chain gene can be inserted into separate vector or, more typically, both genes are inserted into the same expression vector. The antibody genes are inserted into the expression vector by standard methods (e.g., ligation of complementary restriction sites on the antibody gene fragment and vector, or blunt end ligation if no restriction sites are present). Prior to insertion of the D2E7 or D2E7-related light or heavy chain sequences, the expression vector may already carry antibody constant region sequences. For example, one approach to converting the D2E7 or D2E7-related VH and VL sequences to full-length antibody genes is to insert them into expression vectors already encoding heavy chain constant and light chain constant regions, respectively, such that the VH segment is operatively linked to the CH segment(s) within the vector and the VL segment is operatively linked to the CL segment within the vector. Additionally or alternatively, the recombinant expression vector can encode a signal peptide that facilitates secretion of the antibody chain from a host cell. The antibody chain gene can be cloned into the vector such that the signal peptide is linked in-frame to the amino terminus of the antibody chain gene. The signal peptide can be an immunoglobulin signal peptide or a heterologous signal peptide (i.e., a signal peptide from a non-immunoglobulin protein).

In addition to the antibody chain genes, the recombinant expression vectors of the invention carry regulatory sequences that control the expression of the antibody chain genes in a host cell. The term "regulatory sequence" is intended to includes promoters, enhancers and other expression control elements (e.g., polyadenylation signals) that control the transcription or translation of the antibody chain genes. Such regulatory sequences are described, for example, in Goeddel; Gene Expression Technology: Methods in Enzymology 185, Academic Press, San Diego, CA (1990). It will be appreciated by those skilled in the art that the design of the expression vector, including the selection of regulatory sequences may depend on such factors as the choice of the host cell to be transformed, the level of expression of protein desired, etc. Preferred regulatory sequences for mammalian host cell expression include viral elements that direct high levels of protein expression in mammalian cells, such as promoters and/or enhancers derived from cytomegalovirus (CMV) (such as the CMV promoter/enhancer), Simian Virus 40 (SV40) (such as the SV40 promoter/enhancer), adenovirus, (e.g., the adenovirus major late promoter (AdMLP)) and polyoma. For further description of viral regulatory elements, and sequences thereof, see e.g., U.S. Patent No. 5,168,062 by Stinski, U.S. Patent No. 4,510,245 by Bell et al. and U.S. Patent No. 4,968,615 by Schaffner et al.

In addition to the antibody chain genes and regulatory sequences, the recombinant expression vectors of the invention may carry additional sequences, such as sequences that regulate replication of the vector in host cells (e.g., origins of replication) and selectable marker genes. The selectable marker gene facilitates selection of host cells into which the vector has been introduced (see e.g., U.S. Patents Nos. 4,399,216, 4,634,665 and 5,179,017, all by Axel et al.). For example, typically the selectable marker gene confers resistance to drugs, such as G418, hygromycin or methotrexate, on a host cell into which the vector has been introduced. Preferred selectable marker genes include the dihydrofolate reductase (DHFR) gene (for use in dhfr⁻ host cells with methotrexate selection/amplification) and the *neo* gene (for G418 selection).

For expression of the light and heavy chains, the expression vector(s) encoding the heavy and light chains is transfected into a host cell by standard techniques. The various forms of the term "transfection" are intended to encompass a wide variety of techniques commonly used for the introduction of exogenous DNA into a prokaryotic or eukaryotic host cell, *e.g.,* electroporation, calcium-phosphate precipitation, DEAE-dextran transfection and the like. Although it is theoretically possible to express the antibodies of the invention in either prokaryotic or eukaryotic host cells, expression of antibodies in eukaryotic cells, and most preferably mammalian host cells, is the most preferred because such eukaryotic cells, and in particular mammalian cells, are more likely than prokaryotic cells to assemble and secrete a properly folded and immunologically active antibody. Prokaryotic expression of antibody genes has been reported to be ineffective for production of high yields of active antibody (Boss, M.A. and Wood, C. R. (1985) Immunology Today 6:12-13).

Preferred mammalian host cells for expressing the recombinant antibodies of the invention include Chinese Hamster Ovary (CHO cells) (including dhfr- CHO cells, described in Urlaub and Chasin, (1980) Proc. Natl. Acad. Sci. USA 77:4216-4220, used with a DHFR selectable marker, *e.g.,* as described in R.J. Kaufman and P.A. Sharp (1982) Mol. Biol 159:601-621), NS0 myeloma cells, COS cells and SP2 cells. When recombinant expression vectors encoding antibody genes are introduced into mammalian host cells, the antibodies are produced by culturing the host cells for a period of time sufficient to allow for expression of the antibody in the host cells or, more preferably, secretion of the antibody into the culture medium in which the host cells are grown. Antibodies can be recovered from the culture medium using standard protein purification methods.

Host cells can also be used to produce portions of intact antibodies, such as Fab fragments or scFv molecules. It is understood that variations on the above procedure are within the scope of the present invention. For example, it may be desirable to transfect a host cell with DNA encoding either the light chain or the heavy chain (but not both) of an antibody of this invention. Recombinant DNA technology may also be used to remove some or all of the DNA encoding either or both of the light and heavy chains that is not necessary for binding to hTNFα. The molecules expressed from such truncated DNA molecules are also encompassed by the antibodies of the invention. In addition, bifunctional antibodies may be produced in which one heavy and one light chain are an antibody of the invention and the other heavy and light chain are specific for an antigen other than hTNFα by crosslinking an antibody of the invention to a second antibody by standard chemical crosslinking methods.

In a preferred system for recombinant expression of an antibody, or antigen-binding portion thereof, of the invention, a recombinant expression vector encoding both the antibody heavy chain and the antibody light chain is introduced into dhfr-CHO cells by calcium phosphate-mediated transfection. Within the recombinant expression vector, the antibody heavy and light chain genes are each operatively linked to CMV enhancer/AdMLP promoter regulatory elements to drive high levels of transcription of the genes. The recombinant expression vector also carries a DHFR gene, which allows for selection of CHO cells that have been transfected with the vector using methotrexate selection/amplification. The selected transformant host cells are culture to allow for expression of the antibody heavy and light chains and intact antibody is recovered from the culture medium. Standard molecular biology techniques are used to prepare the recombinant expression vector, transfect the host cells, select for transformants, culture the host cells and recover the antibody from the culture medium.

Recombinant human antibodies of the invention in addition to D2E7 or an antigen binding portion thereof, or D2E7-related antibodies disclosed herein can be isolated by screening of a recombinant combinatorial antibody library, preferably a scFv phage display library, prepared using human VL and VH cDNAs prepared from mRNA derived from human lymphocytes. Methodologies for preparing and screening such libraries are known in the art. In addition to commercially available kits for generating phage display libraries (e.g., the Pharmacia *Recombinant Phage Antibody System,* catalog no. 27-9400-01; and the Stratagene *SurjZAP™* phage display kit, catalog no. 240612), examples of methods and reagents particularly amenable for use in generating and screening antibody display libraries can be found in, for example, Ladner et al. U.S. Patent No. 5,223,409; Kang et al. PCT Publication No. WO 92/18619; Dower et al. PCT Publication No. WO 91/17271; Winter et al. PCT Publication No. WO 92/20791; Markland et al PCT Publication No. WO 92/15679; Breitling et al. PCT Publication No. WO 93/01288; McCafferty et al. PCT Publication No. WO 92/01047; Garrard et al. PCT Publication No. WO 92/09690; Fuchs et al. (1991) Bio/Technology 9:1370-1.372; Hay et al. (1992) Hum Antibod Hybridomas 3:81-85; Huse et al. (1989) Science 246:1275-1281; McCafferty et al., Nature (1990) 348:552-554; Griffiths et al. (1993) EMBO J 12:725-734; Hawkins et al. (1992) J Mol Biol 226:889-896; Clackson et al. (1991) Nature 352:624-628; Gram et al. (1992) PNAS 89:3576-3580; Garrard et al. (1991) Bio/Technology 9:1373-1377; Hoogenboom et al. (1991) Nuc Acid Res 19:4133-4137; and Barbas et al. (1991) PNAS 88:7978-7982. Methods of isolating human antibodies with high affinity and a low off rate constant for hTNFα are described in U.S. Patent Nos. 6,090,382, 6,258,562, and 6,509,015, each of which is incorporated by reference herein.

II. Uses of TNFα Inhibitors of the Invention

In an embodiment, the invention provides a method for inhibiting TNFα activity in a subject suffering from a TNFα-related disorder in which TNFα activity is detrimental. In one embodiment, the TNFα inhibitor is D2B7, also referred to as HUMIRA® (adalimumab).

TNFα has been implicated in the pathophysiology of a wide variety of a TNFα-related disorders including sepsis, infections, autoimmune diseases, transplant rejection and graft-versus-host disease (see *e.g.,* Moeller, A., et al. (1990) Cytokine 2:162-169; U.S. Patent No. 5,231,024 to Moeller et al.; European Patent Publication No. 260 610 B1 by Moeller, A., et al. Vasilli, P. (1992) Annu. Rev Immunol. 10:411-452; Tracey, K.J. and Cerami, A. (1994) Annu. Rev. Med. 45:491-503). The invention provides methods for inhibiting TNFα activity in a subject suffering from a TNFα-related disorder, which method comprises administering to the subject an antibody, antibody portion, or other TNFα inhibitor such that TNFα activity in the subject suffering from the TNFα-related disorder is inhibited. The invention also provides methods for inhibiting or decreasing TNFα activity in a subject with vasculitis, comprising administering to the subject an antibody, or antibody portion, or other TNFα inhibitor of the invention such that TNFα activity in the subject is inhibited or decreased. Preferably, the TNFα is human TNFα and the subject is a human subject. Alternatively, the subject can be a mammal expressing a TNFα with which an antibody of the invention cross-reacts. Still further the subject can be a mammal into which has been introduced hTNFα (*e.g.,* by administration of hTNFα or by expression of an hTNFα transgene). An antibody of the invention can be administered to a human subject for therapeutic purposes (discussed further below).

Moreover, an antibody of the invention can be administered to a non-human mammal expressing a TNFα with which the antibody cross-reacts (*e.g.,* a primate, pig or mouse) for veterinary purposes or as an animal model of human disease. Regarding the latter, such animal models may be useful for evaluating the therapeutic efficacy of antibodies of the invention (*e.g.,* testing of dosages and time courses of administration). Examples of animal models used to study spondyloarthropathies include *ank/ank* transgenic mice, HLA-B27 transgenic rats (see Taurog et al. (1998) The Spondylarthritides. Oxford:Oxford University Press).

Examples of animal models used for evaluating the therapeutic efficacy of an agent for treating a hepatic disorder include the chimpanzee hepatitis C virus model (see Shimizu et al. (1990) Proc Natl Acad Sci. USA 87:6441). Examples of animal models used to study skin and nail disorder disorders include, for example, the severe combined immunodeficient (SCID) mouse model (psoriasis) and the Smith line (SL) chicken and depigmenting mouse (vitiligo) (see Nickoloff (2000) Investig Dermatol Symp Proc.5:67; Austin et al. (1995) Am J Pathol. 146:1529; Lerner et al. (1986) J Invest Dermatol. 87:299).

Examples of animal models for evaluating the efficacy of a TNFα antibody for the treatment of a metabolic disorder include NOD transgenic mice, Akita mice, NSY transgenic mice and *ob/ob* mice (see Baeder et al. (1992) Clin Exp Immunol. 89:174; Haseyama et al. (2002) Tohoku J Exp Med. 198:233; Makino et al. (1980): Exp.Anim. 29:1; Kolb (1987) Diabetes/Metabolism Reviews 3:751; Hamada et al.(2001) Metabolism. 50:1282; Coleman, (1978) Diabetologia, 14:141; Bailey et al. (1982) Int.J.Obesity 6:11). Examples of animal models used to study vasculitis includes the mouse HSV model (Behcet's disease), the mouse *L. casei* model (Kawasaki's disease), and the mouse ANCA model (Kawasaki's disease). Other models of vasculitis include the McR5-*lpr/lpr* strain (Nose, M., et al. (1996) Am. J. Path. 149:1763) and the SCG/Kj strain of mice (Kinjoh, et al. (1993) Proc. Natl. Acad. Sci., USA 90:3413). These mice strains spontaneously develop crescentic glomerulonephritis and necrotizing vasculitis of the small arteries and arterioles of the spleen, stomach, heart, uterus and ovaries. These animals develop hypergammaglobulinemia and ANCA autoantibodies that react with myeloperoxidase (MPO). Additionally, immunization of rats with human MPO results in ANCA-associated necrotizing crescentic glomerulonephritis (Brouwer, E., et al. (1993) J. Exp. Med. 177:905).

Examples of animal models used to study idiopathic interstitial lung disease and chronic obstructive airway disorders include ovalbumin (OVA) induced allergic asthma mice and cigarette smoke induced chronic obstructive pulmonary disease mice (see Hessel, EM., et al. (1995) Eur J Pharmacol. 293:401; Keast D, et al. (1981) J. Pathol. 135:249)

Commonly used animal models for studying coronary disorders, including restenosis, include the rat or mouse carotid artery ligation model and the carotid artery injury model (Ferns et al. (1991) Science 253:1129; Clowes et al. (1983) Lab. Invest. 49:208; Lindner et al. (1993) Circ Res. 73:792). In the carotid artery ligation model, arterial blood flow is disrupted by ligation of the vessel near the distal bifurnation. As described in Clowes *et al.,* the carotid artery injury model is performed such that the common carotid artery is denuded of endothelium by the intraluminal passage of a balloon catheter introduced through the external carotid artery. At 2 weeks, the carotid artery is markedly narrowed due to smooth muscle cell constriction, but between 2 and 12 weeks the intimal doubles in thickness leading to a decrease in luminal size. Any of these models can be used to determine the potential therapeutic action of the TNFα antibodies of the invention in the prevention and treatment of restenosis in humans.

Examples of animal models used to study anemia include rats inoculated with peptidolglycan-polysaccharide polymers (see Coccia et al., (2001) Exp Hematology. 29:1201-1209). Examples of animal models used to study pain are well known in the art, and include the rat sciatic nerve ligation model, and the rat segmental spinal nerve ligation model (see Bennett and Zie, (1988) Pain. 33:87-107; Kim and Chung, (1992) Pain 50:355-363).

As used herein, the term " TNFα-related disorder in which TNFα activity is detrimental" is intended to include TNFα-related diseases and other disorders in which the presence of TNFα in a subject suffering from the disorder has been shown to be or is suspected of being either responsible for the pathophysiology of the disorder or a factor that contributes to a worsening of the disorder, *e.g.,* juvenile rheumatoid arthritis. Accordingly, TNFα-related disorders in which TNFα activity is detrimental are disorders in which inhibition of TNFa activity is expected to alleviate the symptoms and/or progression of the disorder. Such disorders may be evidenced, for example, by an increase in the concentration of TNFα in a biological fluid of a subject suffering from the disorder (e.g., an increase in the concentration of TNFα in serum, plasma, synovial fluid, etc. of the subject), which can be detected, for example, using an anti-TNFα antibody as described above. The use of the antibodies, antibody portions, and other TNFα inhibitors of the invention in the treatment of specific TNFα-related disorder in which TNFα activity is detrimental, is discussed further below. In certain embodiments, the antibody, antibody portion, or other TNFα inhibitor of the invention is administered to the subject in combination with another therapeutic agent, as described below in Section III.

A. *Spondyloarthropathies*

TNFα has been implicated in the pathophysiology of a wide variety of disorders, including inflammatory diseases such as spondyloarthopathies (see *e.g.,* Moeller, A., et al. (1990) Cytokine 2:162-169; U.S. Patent No. 5,231,024 to Moeller et al.; European Patent Publication No. 260 610 B1 by Moeller, A). The invention provides methods for TNFα activity in a subject suffering from such a disorder, which method comprises administering to the subject an antibody, antibody portion, or other TNFα inhibitor such that TNFα activity in the subject suffering from a spondyloarthropathy is inhibited. In one embodiment, the invention provides a method of treating spondyloarthopathies.

As used herein, the term "spondyloarthropathy" or "spondyloarthropathies" is used to refer to any one of several diseases affecting the joints of the spine, wherein such diseases share common clinical, radiological, and histological features. A number of spondyloarthropathies share genetic characteristics, *i.e.* they are associated with the HLA-B27 allele. In one embodiment, the term spondyloarthropathy is used to refer to any one of several diseases affecting the joints of the spine, excluding ankylosing spondylitis, wherein such diseases share common clinical, radiological, and histological features. Examples of spondyloarthropathies include ankylosing spondylitis, psoriatic arthritis/spondylitis, enteropathic arthritis, reactive arthritis or Reiter's syndrome, and undifferentiated spondyloarthropathies.

The TNFα antibody of the invention can also be used to treat subjects who are at risk of developing a spondyloarthropathy. Examples of subjects who are at risk of having spondyloarthropathies include humans suffering from arthritis. Spondyloarthropathies can be associated with other forms of arthritis, including rheumatoid arthritis. In one embodiment, the antibody of the invention is used to treat a subject who suffers from a spondyloarthropathy associated with rheumatoid arthritis. Examples of spondyloarthropathies which can be treated with the TNFα antibody of the invention are described below:

1. *Ankylosing Spondylitis (AS)*

Tumor necrosis factor has been implicated in the pathophysiology of ankylosing spondylitis (see Verjans et al. (1991) Arthritis Rheum. 34(4):486; Verjans et al. (1994) Clin. Exp Immunol. 97(1):45; Kaijtzel et al. (1999) Hum Immunol 60(2): 140). Ankylosing spondylitis (AS) is an inflammatory disorder involving inflammation of one or more vertebrae. AS is a chronic inflammatory disease that affects the axial skeleton and/or peripheral joints, including joints between the vertebrae of the spine and sacroiliac joints and the joints between the spine and the pelvis. AS can eventually cause the affected vertebrae to fuse or grow together. Spondyarthropathies, including AS, can be associated with psoriatic arthritis (PsA) and/or inflammatory bowel disease (IBD), including ulcerative colitis and Crohn's disease.

Early manifestations of AS can be determined by radiographic tests, including CT scans and MRI scans. Early manifestations of AS often include scroiliitis and changes in the sacroliac joints as evidenced by the blurring of the cortical margins of the subchrondral bone, followed by erosions and sclerosis. Fatigue has also been noted as a common symptom of AS (Duffy et al. (2002) ACR 66th Annual Scientific Meeting Abstract). Accordingly, the antibody, or antigen-binding fragment thereof, of the invention can be used to treat AS. In one embodiment, the TNFα antibody, or antigen-binding fragment thereof, of the invention is used to treat spondyloarthropathy associated with IBD, including AS

AS is often treated with nonsteroidal anti-inflammatory medications (NSAIDs), such as aspirin or indomethacin. Accordingly, the TNFα antibody of the invention may also be administered in combination with agents commonly used to reduce inflammation and pain commonly associated with ankylosing spondylitis.

*2. Psoriatic arthritis*

Tumor necrosis factor has been implicated in the pathophysiology of psoriatic arthritis (Partsch et al. (1998) Ann Rheum Dis. 57:691; Ritchlin et al. (1998) J Rheumatol. 25:1544). As referred to herein, psoriatic arthritis (PsA) or psoriasis associated with the skin, refers to chronic inflammatory arthritis which is associated with psoriasis. Psoriasis is a common chronic skin condition that causes red patches on the body. About 1 in 20 individuals with psoriasis will develop arthritis along with the skin condition, and in about 75% of cases, psoriasis precedes the arthritis. PsA exhibits itself in a variety of ways, ranging from mild to severe arthritis, wherein the arthritis usually affects the fingers and the spine. When the spine is affected, the symptoms are similar to those of ankylosing spondylitis, as described above. The TNFα antibody, or antigen-binding fragment thereof, of the invention can be used to treat PsA.
PsA is sometimes associated with arthritis mutilans. Arthritis mutilans refers to a disorder which is characterized by excessive bone erosion resulting in a gross, erosive deformity which mutilates the joint In one embodiment, the TNFα antibody, or antigen-binding fragment thereof, of the invention can be used to treat arthritis mutilans.

*3. Reactive arthritis / Reiter's syndrome*

Tumor necrosis factor has been implicated in the pathophysiology of reactive arthritis, which is also referred to as Reiter's syndrome (Braun et al. (1999) Arthritics Rheum. 42(10):2039). Reactive arthritis (ReA) refers to arthritis which complicates an infection elsewhere in the body, often following enteric or urogenital infections. ReA is often characterized by certain clinical symptoms, including inflammation of the joints (arthritis), urethritis, conjunctivitis, and lesions of the skin and mucous membranes. In addition, ReA can occurs following infection with a sexually transmitted disease or dysenteric infection, including chlamydia, campylobacter, salmonella, or yersinia. Accordingly, the TNFα antibody, or antigen-binding fragment thereof, of the invention can be used to treat ReA.

*4. Undifferentiated spondyloarthropathies*

In one embodiment, the TNFα antibodies of the invention are used to treat subjects suffering from undifferentiated spondyloarthropathies (see Zeidler et al. (1992) Rheum Dis Clin North Am. 18:187). Other terms used to describe undifferentiated spondyloarthropathies include seronegative oligoarthritis and undifferentiated oligoarthritis. Undifferentiated spondyloarthropathies, as used herein, refers to a disorder wherein the subject demonstrates only some of the symptoms associated with a spondyloarthropathy. This condition is usually observed in young adults who do not have IBD, psoriasis, or the classic symptoms of AS or Reiter's syndrome. In some instances, undifferentiated spondyloarthropathies may be an early indication of AS. In one embodiment, the TNFα antibody, or antigen-binding fragment thereof, of the invention can be used to treat undifferentiated spondyloarthropathies.

*B. Pulmonary Disorders*

TNFα has been implicated in the pathophysiology of a wide variety of pulmonary disorders, including pulmonary disorders such as idiopathic interstitial lung disease and chronic obstructive airway disorders (see *e.g.,* Piquet PF et al. (1989) J Exp Med. 170:655-63; Whyte M, et al. (2000) Am J Respir Crit Care Med. 162:755-8; Anticevich SZ, et al. (1995) Eur J Pharmacol. 284:221-5). The invention provides methods for TNFα activity in a subject suffering from such a pulmonary disorder, which method comprises administering to the subject an antibody, antibody portion, or other TNFα inhibitor such that TNFα activity in the subject suffering from idiopathic interstitial lung disease or a chronic obstructive airway disorder is inhibited. Examples of idiopathic interstitial lung diseases and chronic obstructive airway disorders in which TNFα activity is detrimental are discussed further below.

*1. Idiopathic interstitial lung disease*

In one embodiment, the TNFα antibody of the invention is used to treat subjects who have an idiopathic interstitial lung disease. Idiopathic interstitial lung diseases affect the lungs in three ways: first, the lung tissue is damaged in some known or unknown way; second, the walls of the air sacs in the lung become inflamed; and finally, scarring (or fibrosis) begins in the interstitium (or tissue between the air sacs), and the lung becomes stiff. Examples of idiopathic interstitial lung diseases are described below.

*a. Idiopathic pulmonary fibrosis (IPF)*

Tumor necrosis factor has been implicated in the pathophysiology of idiopathic pulmonary fibrosis (IPF) (see Piquet PF, et al. (1989) J Exp Med. 170:655-63; Whyte M, et al. (2000) Am J Respir Crit Care Med 162:755-8; Corbett EL, et al. (2002) Am J Respir Crit Care Med. 165:690-3). For example, it has been found that IPF patients have increased levels of TNF expressiojn in macrophages and in type II epithelial cells (Piquet et al. (1993) Am J Pathol 143:651; Nash et al. (1993) Histopathology 22:343; Zhang et al. (1993) J Immunol 150:4188). Certain genetic polymorphisms are also associated with increased TNF expression, and are implicated in playing a role in IPF and silicosis (Whyte *et al., supra;* Corbett EL, *et al., supra*).

The term "idiopathic pulmonary fibrosis" or "IPF" refers to a group of disorders characterized by inflammation and eventually scarring of the deep lung tissues, leading to shortness of breath. The scarring of the alveoli (air sacs) and their supporting structures (the interstitium) in IPF eventually leads to a loss of the functional alveolar units and a reduction of the transfer of oxygen from air to blood. IPF is also referred to as diffuse parenchymal lung disease; alveolitis; cryptogenic fibrosing alveolitis (CFA); idiopathic pulmonary pneumonitis (IPP); and usual interstitial pneumonitis (UIP). IPF is often used synonymously with UIP ("IPF/UIP") because UIP is the most common cellular pattern seen in the pathologic diagnosis of IPF.

Patients with IPF often exhibit certain symptoms, including a dry cough, chest pain, and/or shortness of breath. Commonly used drugs for the treatment of IPF are prednisone and cytoxan, although only a fraction of patients improve with continued use of these drugs (American Thoracic Society (2000) Am. J. Respir. Crit. Care Med. 161:646). Oxygen administration and transplantation of the lung are other choices for treatment. In one embodiment, the TNF$\alpha$ antibody of the invention is administered to the subject in combination with another therapeutic agent, for example oxygen, for the treatment of idiopathic pulmonary fibrosis,.

## 2. *Chronic obstructive airway disorder*

In one embodiment, the TNF$\alpha$ antibody of the invention is used to treat a subject who has a chronic obstructive airflow disorder. In these diseases, airflow obstruction may be chronic and persistent or episodic and recurrent. Airflow obstruction is usually determined by forced expiratory spirometry, which is the recording of exhaled volume against time during a maximal expiration. In a subject who does not have an obstructed airflow, a full forced expiration usually takes between 3 and 4 seconds. In a patient with chronic obstructive airflow disorder, wherein airflow is obstructed, it usually takes up to 15 to 20 seconds and may be limited by breath-holding time. The normal forced expiratory volume in the first second of expiration ($FEV_1$) is easily measured and accurately predicted on the basis of age, sex, and height. The ratio of $FEV_1$ to forced vital capacity ($FEV_1/FVC$) normally exceeds 0.75. Recording airflow against volume during forced expiration and a subsequent forced inspiration-the flow-volume loop-is also useful, mainly for distinguishing upper from lower airway narrowing. Examples of chronic obstructive airway disorders are described below.

### a. *Asthma*

Tumor necrosis factor has been implicated in the pathophysiology of asthma, (Anticevich SZ, et al. (1995) Eur J Pharmacol. 284:221-5; Thomas PS, et al. 1995. Am J Respir Crit Care Med. 152:76-80; Thomas PS, Heywood G. (2002) Thorax. 57:774-8). For example, acute asthma attacks have been found to be associated with pulmonary neutrophilia and elevated BAL TNF levels (Ordonez CL. et al. (2000) Am J Respir Crit Care Med 161:1185). It has been found that the severity of asthma symptoms correlates with endotoxin levels in house dust. In rats, anti-TNF antibodies reduced endotoxin-induced airway changes (Kips et al. (1992) Am Rev Respir Dis 145:332).

The term "asthma" as used herein, refers to a disorder in which inflammation of the airways causes airflow into and out of the lungs to be restricted. Asthma is also referred to as bronchial asthma, exercise induced asthma - bronchial, and reactive airways disease (RAD). In some instances, asthma is associated with allergies and/or is familial. Asthma includes a condition which is characterized by widespread fluctuations in the diameter or caliber of bronchial airways over short periods of time, resulting in changes in lung function. The resulting increased resistance to air flow produces symptoms in the affected subject, including breathlessness (dyspnea), chest constriction or "tightness," and wheezing.

Patients with asthma are characterized according to NIH guidelines, are described as mild intermittent, mild persistent, moderate persistent, and severe persistent (see NAEPP Expert Panel Report Guidelines for the Diagnosis and Management of Asthma-Update on Selected Topics 2002. JACI 2002; 110: S141-S209; Guidelines for the Diagnosis and Management of Asthma. NIH Publication 97-4051, July 1997). Patients diagnosed with moderate persistent asthma are often treated with inhaled corticosteroids. Patients diagnosed with severe persistent asthma are often treated with high dose inhaled corticosteroids and p.o. corticosteroids.

### b. *Chronic obstructive pulmonary disease (COPD)*

Tumor necrosis factor has been implicated in the pathophysiology of chronic obstructive pulmonary disease, (Keatings VM. (2000) Chest. 118:971-5; Sakao S, et al.( 2001) Am J Respir Crit Car e Med. 163:420-22; Sakao S, et al. (2002) Chest. 122:416-20). The term " chronic obstructive pulmonary disease" or "COPD" as used interchangeably herein, refers to a group of lung diseases characterized by limited airflow with variable degrees of air sack enlargement and lung tissue destruction. The term COPD includes chronic bronchitis (mucous hypersecretion with goblet cell submucosal gland hyperplasia), chronic obstructive bronchitis, or emphysema (destruction of airway parenchyma), or combinations of these conditions. Emphysema and chronic bronchitis are the most common forms of chronic obstructive pulmonary disease. COPD is defined by irreversible airflow obstruction.

In COPD, chronic inflammation leads to fixed narrowing of small airways and lung parenchyma and alveolar wall destruction (emphysema). This is characterized by increased numbers of alveolar macrophages, neutrophils, and cytotoxic T lymphocytes, and the release of multiple inflammatory mediators (lipids, chemokines, cytokines, growth factors). This inflammation leads to fibrosis with a narrowing of the small airways and lung parenchymal destruction. There is also a high level of oxidative stress, which may amplify this inflammation.

C. *Coronary Disorders*

TNFα has been implicated in the pathophysiology of a wide variety of coronary disorders, including restenosis (see *e.g.,* Clausell *et al.* (1994), *supra;* Medall et al. (1997) Heart 78(3):273). As used herein, the term "a coronary disorder in which TNFα activity is detrimental" is intended to include coronary and cardiovascular diseases in which the presence of TNFα in a subject suffering from the disorder has been shown to be or is suspected of being either responsible for the pathophysiology of the disorder or a factor that contributes to a worsening of the disorder, including cardiovascular disorders, *e.g.*, restenosis. Coronary disorders in which TNFα activity is detrimental often result from a blockage in an artery. Such a blockage can be caused by a clot, which usually forms in a coronary artery that has been previously narrowed from changes usually related to atherosclerosis. For example, if the atherosclerotic plaque inside the arterial wall cracks, it can trigger the formation of a thrombus, or clot. Such disorders may be evidenced, for example, by an increase in the concentration of TNFα in a biological fluid of a subject suffering from the disorder (*e.g.*, an increase in the concentration of TNFα in serum, plasma, synovial fluid, *etc,* of the subject), which can be detected, for example, using an anti-TNFα antibody as described above. A coronary disorder can be also caused by an imbalance in arterial pressure, a malfunction of the heart, or an occlusion of a blood vessel, *e.g.,* by a thrombus. Coronary disorders includes both coronary artery disease and peripheral vascular disease.

There are numerous examples of coronary disorders in which TNFα activity is detrimental, including restenosis. The use of the antibodies, antibody portions, and other TNFα inhibitors of the invention in the treatment of specific coronary disorders are discussed further below. In certain embodiments, the antibody, antibody portion, or other TNFα inhibitor of the invention is administered to the subject in combination with another therapeutic agent, as described below

The invention provides a method for inhibiting TNFα activity in a subject with a coronary disorder. The invention provides methods for inhibiting or decreasing TNFα activity in a subject with a coronary disorder, comprising administering to the subject an antibody, or antibody portion, or other TNFα inhibitor of the invention such that TNFα activity in the subject is inhibited or decreased. Preferably, the TNFα is human TNFα and the subject is a human subject. Alternatively, the subject can be a mammal expressing a TNFα with which an antibody of the invention cross-reacts. Still further the subject can be a mammal into which has been introduced TNFα (*e.g.,* by administration of hTNFα or by expression of an hTNFα tranagene). An antibody of the invention can be administered to a human subject for therapeutic purposes (discussed further below). Moreover, an antibody of the invention can be administered to a non-human mammal expressing a TNFα with which the antibody cross-reacts (*e.g.,* a primate, pig or mouse) for veterinary purposes or as an animal model of human disease. Regarding the latter, such animal models may be useful for evaluating the therapeutic efficacy of antibodies of the invention (*e.g.,* testing of dosages and time courses of administration).

Commonly used animal models for studying coronary disorders, including restenosis, include the rat or mouse carotid artery ligation model and the carotid artery injury model (Ferns et al. (1991) Science 253:1129; Clowes et al. (1983) Lab. Invest. 49:208; Lindner et al. (1993) Circ Res. 73:792). In the carotid artery ligation model, arterial blood flow is disrupted by ligation of the vessel near the distal bifurnation. As described in Clowes *et al.,* the carotid artery injury model is performed such that the common carotid artery is denuded of endothelium by the intraluminal passage of a balloon catheter introduced through the external carotid artery. At 2 weeks, the carotid artery is markedly narrowed due to smooth muscle cell constriction, but between 2 and 12 weeks the intimal doubles in thickness leading to a decrease in luminal size. Any of these models can be used to determine the potential therapeutic action of the TNFα antibodies of the invention in the prevention and treatment of restenosis in humans.

The antibody of the invention can be used to treat cardiovascular disorders in which TNFα activity is detrimental, wherein inhibition of TNFα activity is expected to alleviate the symptoms and/or progression of the coronary disease or to prevent the coronary disease. Subjects suffering from or at risk of developing coronary disorders can be identified through clinical symptoms. Clinical symptoms in coronary disease often include chest pain, shortness of breath, weakness, fainting spells, alterations in consciousness, extremity pain, paroxysmal nocturnal dyspnea, transient ischemic attacks and other such phenomena experienced by the patient. Clinical signs of coronary disease can also include EKG abnormalities, altered peripheral pulses, arterial bruits, abnormal heart sounds, rates and wheezes, jugular venous distention, neurological alterations and other such findings discerned by the clinician. Coronary disorders may also be evidenced, for example, by an increase in the concentration of TNF$\alpha$ in a biological fluid of a subject suffering from the disorder (*e.g.*, an increase in the concentration of TNF$\alpha$ in serum, plasma, synovial fluid, etc. of the subject).

Examples of a cardiovascular disorder include, but are not limited to, coronary artery disease, angina pectoris, myocardial infarction, cardiovascular tissue damage caused by cardiac arrest, cardiovascular tissue damage caused by cardiac bypass, cardiogenic shock, and hypertension, atherosclerosis, coronary artery spasm, coronary artery disease, valvular disease, arrhythmias, and cardiomyopathies. The use of the antibodies, antibody portions, and other TNF$\alpha$ inhibitors of the invention in the treatment of specific cardiovascular diseases are discussed further below. In certain embodiments, the antibody, antibody portion, or other TNF$\alpha$ inhibitor of the invention is administered to the subject in combination with another therapeutic agent, as described below in section III.

*1. Restenosis*

TNF$\alpha$ has been implicated in the pathophysiology of restenosis (see Zhou et al. (2002) Atherosclerosis. 161:153; Javed et al. (2002) Exp and Mol Pathol 73:104). For example, in the murine wire carotid model, TNF -/- mice demonstrated a seven-fold reduction in intial hyperplasia compared to wild type mice (Zimmerman et al. (2002) Am J Phsiol Regul Integr Comp Physiol 283:R505). Restenosis can occur as the result of any type of vascular reconstruction, whether in the coronary vasculature or in the periphery (Colburn and Moore (1998) Myointimal Hyperplasia pp. 690-709 in Vascular Surgery: A Comprehensive Review Philadelphia: Saunders). For example, studies have reported symptomatic restenosis rates of 30-50% following coronary angioplasties (see Berk and Harris (1995) Adv. Intern. Med. 40:455-501). After carotid endarterectomies, as a further example, 20% of patients studied had a luminal narrowing greater than 50% (Clagett et al. (1986) J. Vase. Surg. 3:10-23). Restenosis is evidenced in different degrees of symptomatology which accompany preocclusive lesions in different anatomical locations, due to a combination of factors including the nature of the vessels involved, the extent of residual disease, and local hemodynamics.

"Stenosis," as used herein refers to a narrowing of an artery as seen in occlusive disorder or in restenosis. Stenosis can be accompanied by those symptoms reflecting a decrease in blood flow past the narrowed arterial segment, in which case the disorder giving rise to the stenosis is termed a disease (i.e., occlusive disease or restenosis disease). Stenosis can exist asymptomatically in a vessel, to be detected only by a diagnostic intervention such as an angiography or a vascular lab study.

The antibody of the invention can be used to treat a subject suffering from or at risk of developing restenosis. A subject at risk of developing restenosis includes a subject who has undergone PTCA. The subject may have also had a stent inserted to prevent restenosis. The TNF$\alpha$ antibody of the invention can be used alone or in combination with a stent to prevent the re-occurrence of stenosis in a subject suffering from cardiovascular disease.

2. *Congestive Heart Failure*

TNF$\alpha$ has been implicated in the pathophysiology of congestive heart failure (see Zhou et al. (2002) Atherosclerosis 161:153). Serum levels of TNF$\alpha$ are elevated in patients with congestive heart failure in a manner which is directly proportional to the severity of the disease (Levine et al. (1990) N Engl JMed 323:236; Torre-Amione et al. (1996) J Am Coll Cardiol 27:1201). In addition, inhibitors of TNF$\alpha$ have also been shown to improve congestive heart failure symptoms (Chung et al. (2003) Circulation 107:3133).

As used herein, the term "congestive heart failure" includes a condition characterized by a diminished capacity of the heart to supply the oxygen demands of the body. Symptoms and signs of congestive heart failure include diminished blood flow to the various tissues of the body, accumulation of excess blood in the various organs, *e.g*, when the heart is unable to pump out the blood returned to it by the great veins, exertional dyspnea, fatigue, and/or peripheral edema, *e.g.*, peripheral edema resulting from left ventricular dysfunction. Congestive heart failure may be acute or chronic. The manifestation of congestive heart failure usually occurs secondary to a variety of cardiac or systemic disorders that share a temporal or permanent loss of cardiac function. Examples of such disorders include hypertension, coronary artery disease, valvular disease, and cardiomyopathies, e.g., hypertrophic, dilative, or restrictive cardiomyopathies.

A "subject who has or is suffering from congestive heart failure" is a subject who has a disorder involving a clinical syndrome of diverse etiologies linked by the common denominator of impaired heart pumping in which the heart cannot pump blood commensurate with the requirements of the metabolizing tissues, or can do so only from an elevated filling pressure. A "subject at risk of developing congestive heart failure" is a subject who has a propensity of developing congestive heart failure because of certain factors affecting the cardiovascular system of the subject It is desirable to reduce the risk of or prevent the development of congestive heart failure in these subjects. The phrase "with congestive heart failure" includes patients who are at risk of suffering from this condition relative to the general population, even though they may not have suffered from it yet, by virtue of exhibiting risk factors. For example, a patient with untreated hypertension may not have suffered from congestive heart failure, but is at risk because of his or her hypertensive condition. In one embodiment of the invention, the antibody D2E7 is used to treat a subject at risk of developing congestive heart failure.

*3. Acute coronary syndromes*

TNFα has been implicated in the pathophysiology of acute coronary syndromes (see Libby (1995) Circulation 91:2844 ). Acute coronary syndromes include those disorders wherein the subject experiences pain due to a blood flow restriction resulting in not enough oxygen reaching the heart. Studies have found that TNFα plays a role in acute coronary syndromes. For example, in a novel rat heterotropic cardiac transplantation-coronary ligation model capable of inducing myocardial infarction in the absence of downstream hemodynamic effects, administration of chimeric soluble TNF receptor (sTNFR) abolished transient LV remodeling and dysfunction (Nakamura, et al. (2003) J. Cardiol. 41:41). It was also found that direct injection of an sTNFR expression plasmid to the myocardium, resulted in a reduction in the infarction size in acute myocardial infarction (AMI) experimental rats (Sugano et al. (2002) FASEB J 16:1421).

In one embodiment, TNFα antibody of the invention is used to treat or prevent an acute coronary syndrome in a subject, wherein the acute coronary syndrome is a myocardial infarction or angina.

As used herein, the term "myocardial infarction" or "MI" refers to a heart attack. A myocardial infarction involves the necorsis or permanent damage of a region of the heart due to an inadequate supply of oxygen to that area. This necrosis is typically caused by an obstruction in a coronary artery from either atherosclerosis or an embolis. MIs which are treated by the TNFα antibody of the invention include both Q-wave and non-Q-wave myocardial infarction. Most heart attacks are caused by a clot that blocks one of the coronary arteries (the blood vessels that bring blood and oxygen to the heart muscle). For example, a clot in the coronary artery interrupts the flow of blood and oxygen to the heart muscle, leading to the death of heart cells in that area. The damaged heart muscle permanently loses its ability to contract, and the remaining heart muscle needs to compensate for it. An MI can also be caused by overwhelming stress in the individual.

The term "angina" refers to spasmodic, choking, or suffocative pain, and especially as denoting angina pectoris which is a paroxysmal thoracic pain due, most often, to anoxia of the myocardium. Angina includes both variant angina and exertional angina. A subject having angina has ischemic heart disease which is manifested by sudden, severe, pressing substernal pain that often radiates to the left shoulder and along the left arm. TNFα has been implicated in angina, as TNFα levels are upregulated in patients with both MI and stable angina (Balbay et al. (2001) Angiology 52109).

*4. Artherosclerosis*

"Atherosclerosis" as used herein refers to a condition in which fatty material is deposited along the walls of arteries. This fatty material thickens, hardens, and may eventually block the arteries. Atherosclerosis is also referred to arteriosclerosis, hardening of the arteries, and arterial plaque buildup. Polyclonal antibodies directed against TNFα have been shown to be effective at neutralizing TNFα activity resulting in inflammation and restenosis in the rabbit atherosclerotic model (Zhou *et al., supra*). Accordingly, the TNFα antibody of the invention can be used to treat or prevent subjects afflicted with or at risk of having atherosclerosis.

*5. Cardiomyopathy*

The term "cardiomyopathy" as used herein is used to define diseases of the myocardium wherein the heart muscle or myocardium is weakened, usually resulting in inadequate heart pumping. Cardiomyopathy can be caused by viral infections, heart attacks, alcoholism, long-term, severe hypertension (high blood pressure), or by autoimmune causes..

In approximately 75-80% of heart failure patients coronary artery disease is the underlying cause of the cardiomyopathy and is designated "ischemic cardiomyopathy." Ischemic cardiomyopathy is caused by heart attacks, which leave scars in the heart muscle or myocardium. The affected myocardium is then unable to contribute to the heart pumping function. The larger the scars or the more numerous the heart attacks, the higher the chance there is of developing ischemic cardiomyopathy.

Cardiomyopathies that are not attributed to underlying coronary artery disease, and are designated "non-ischemic cardiomyopathies." Non-ischemic cardiomyopathies include, but are not limited to idiopathic cardiomyopathy, hypertrophic cardiomyopathy, alcoholic cardiomyopathy, dilated cardiomyopathy, peripartum cardiomyopathy, and restrictive cardiomyopathy.

*D. Metabolic Disorders*

TNFα has been implicated in the pathophysiology of a wide variety of disorders, including metabolic disorders, such as diabetes and obesity (Spiegelman and Hotamisligil (1993) Cell 73:625; Chu et al. (2000) Int J Obes Relat Metab Disord. 24:1085; Ishii et al. (2000) Metabolism. 49:1616). The invention provides methods for TNFα activity in a subject suffering from such a metabolic disorder, which method comprises administering to the subject an antibody, antibody portion, or other TNFα inhibitor such that TNFα activity in the subject suffering from a metabolic disorder is inhibited. The TNFα antibody of the invention can also be used to treat subjects who are at risk of developing a metabolic disorder. Metabolic disorders are often associated with arthritis, including rheumatoid arthritis. In one embodiment, the antibody of the invention is used to treat a subject who suffers from a metabolic disorder associated with rheumatoid arthritis. In another embodiment, the TNFα antibody of the invention is used to treat disorders associated with diabetes or obesity

Metabolic disorders affect how the body processes substances needed to carry out physiological functions. A number of metabolic disorders of the invention share certain characteristics, *i.e.* they are associated the insulin resistance, lack of ability to regulate blood sugar, weight gain, and increase in body mass index. Examples of metabolic disorders include diabetes and obesity. Examples of diabetes include type 1 diabetes mellitus, type 2 diabetes mellitus, diabetic neuropathy, peripheral neuropathy, diabetic retinopathy, diabetic ulcerations, retinopathy ulcerations, diabetic macrovasculopathy, and obesity. Examples of metabolic disorders which can be treated with the TNFσ antibody of the invention are described in more detail below:

*1. Diabetes*

Tumor necrosis factor has been implicated in the pathophysiology of diabetes. (see e.g., Navarro J.F., Mora C., Maca, Am J Kidney Dis. 2003 Jul;42(1):53-61; Daimon M et al., Diabetes Care. 2003 Jul;26(7):2015-20; Zhang M et al., J Tongji Med Univ. 1999;19(3):203-5, Barbieri M et al., Am J Hypertens. 2003 Jul;16(7):537-43.) For example, TNFα is implicated in the pathophysiology for insulin resistance. It has been found that serum TNF levels in patients with gastrointestinal cancer correlates with insulin resistance (see e.g., McCall, J. et al. Br. J. Surg. 1992; 79: 361-3).

Diabetes includes the two most common types of the disorder, namely type I diabetes and type II diabetes, which both result from the body's inability to regulate insulin. Insulin is a hormone released by the pancreas in response to increased levels of blood sugar (glucose) in the blood.

The term "type 1 diabetes," as used herein, refers to a chronic disease that occurs when the pancreas produces too little insulin to regulate blood sugar levels appropriately. Type 1 diabetes is also referred to as insulin-dependent diabetes mellitus, IDMM, juvenile onset diabetes, and diabetes - type I. Type 1 diabetes represents is the result of a progressive autoimmune destruction of the pancreatic β-cells with subsequent insulin deficiency.

The term "type 2 diabetes," refers to a chronic disease that occurs when the pancreas does not make enough insulin to keep blood glucose levels normal, often because the body does not respond well to the insulin. Type 2 diabetes is also referred to as noninsulin-dependent diabetes mellitus, NDDM, and diabetes - type II

Diabetes is can be diagnosed by the administration of a glucose tolerance test. Clinically, diabetes is often divided into several basic categories. Primary examples of these categories include, autoimmune diabetes mellitus, non-insulin-dependent diabetes mellitus (type 1 NDDM), insulin-dependant diabetes mellitus (type 2 IDDM), non-autoimmune diabetes mellitus, non-insulin-dependant diabetes mellitus (type 2 NIDDM), and maturity-onset diabetes of the young (MODY). A further category, often referred to as secondary, refers to diabetes brought about by some identifiable condition which causes or allows a diabetic syndrome to develop. Examples of secondary categories include, diabetes caused by pancreatic disease, hormonal abnormalities, drug-or chemical-induced diabetes, diabetes caused by insulin receptor abnormalities, diabetes associated with genetic syndromes, and diabetes of other causes. (see *e.g.*, Harrison's (1996) 14th ed., New York, McGraw-Hill).

Diabetes manifests itself in the foregoing categories and can cause several complications that are discussed in the following sections. Accordingly, the antibody, or antigen-binding fragment thereof, of the invention can be used to treat diabetes. In one embodiment, the TNFα antibody, or antigen-binding fragment thereof, of the invention is used to treat diabetes associated with the above identified catagores.

Diabetes is aften treated with diet, insulin dosages, and various medications described herein. Accordingly, the TNFα antibody of the invention may also be administered in combination with agents commonly used to treat metabolic disorders and pain commonly associated with diabetes.

In one embodiment, the TNFα antibody of the invention can also be used to treat disorders associated with diabetes. Diabetes manifests itself in many complications and conditions associated with diabetes, including the following catagories:

a. *Diabetic Neuropathy and Peripheral Neuropathy*

Tumor necrosis factor has been implicated in the pathophysiology of diabetic neuropathy and peripheral neuropathy. (See Benjafield et al. (2001) Diabetes Care. 24:753; Qiang, X. et al. (1998) Diabetologia.41:1321-6; Pfeiffer et al. (1997) Horm Metab Res. 29:111).

The term "neuropathy," also referred to as nerve damage-diabetic, as used herein, refers to a common complication of diabetes in which nerves are damaged as a result of hyperglycemia (high blood sugar levels). A variety of diabetic neuropathies are recognized, such as distal sensorimotror polyneuropathy, focal motor neuropathy, and autonomic neuropathy.

The term "peripheral neuropathy," also known as peripheral neuritis and diabetic neuropathy, as used herein, refers to the failure of the nerves to carry information to and from the brain and spinal cord. Peripheral neuropathy produces symptoms such as pain, loss of sensation, and the inability to control muscles. In some cases, the failure of nerves to control blood vessels, intestinal function, and other organs results in abnormal blood pressure, digestion, and loss of other basic involuntary processes. Peripheral neuropathy may involve damage to a single nerve or nerve group (mononeuropathy) or may affect multiple nerves (polyneuropathy).

Neuropathies that affect small myelinated and unmyelinated fibers of the sympathetic and parasympathetic nerves are known as "peripheral neuropathies." Furthermore, the related disorder of peripheral neuropathy, also known as peripheral neuritis and diabetic neuropathy, refers to the failure of the nerves to carry information to and from the brain and spinal cord. This produces symptoms such as pain, loss of sensation, and the inability to control muscles. In some cases, failure of nerves controlling blood vessels, intestinal function, and other organs results in abnormal blood pressure, digestion, and loss of other basic involuntary processes. Peripheral neuropathy may involve damage to a single nerve or nerve group (mononeuropathy) or may affect multiple nerves (polyneuropathy).

The term "diabetic neuropathy" refers to a common complication of diabetes in which nerves are damaged as a result of hyperglycemia (high blood sugar levels). Diabetic neuropathy is also referred to as neuropathy and nerve damage-diabetic. A variety of diabetic neuropathies are recognized, such as distal sensorimotror polyneuropathy, focal motor neuropathy, and autonomic neuropathy.

b. *Diabetic Retinopathy*

Tumor necrosis factor has been implicated in the pathophysiology of diabetic retinopathy (Scholz et al. (2003) Trends Microbiol. 11:171). The term "diabetic retinopathy" as used herein, refers to progressive damage to the eye's retina caused by long-term diabetes. Diabetic retinopathy, includes proliferative retinopathy. Proliferative neuropathy in turn includes includes neovascularization, pertinal hemmorrhave and retinal detachement.

In advanced retinopathy, small vessels proliferate on the surface of the retina. These blood vessels are fragile, tend to bleed and can cause peretinal hemorrhages. The hemorrhage can obscure vision, and as the hemorrhage is resorbed fibrous tissue forms predisposing to retinal detachments and loss of vision. In addition, diabetic retinopathy includes prolferative retinopathy which includes neovascularization, pertinal hemmorrhave and retinal detachement. Daibetic retinopathy also includes "background retinopathy" which involves changes occuring with the layers of the retina.

c. *Diabetic Ulcerations and Retinopathy Ulcerations*

Tumor necrosis factor has been implicated in the pathophysiology of diabetic ulcerations, (see Lee et al. (2003) Hum Immunol. 64:614; Navarro et al. (2003) Am J Kidney Dis. 42:53; Daimon et al. (2003) Diabetes Care. 26:2015; Zhang et al. (1999) J Tongji Med Univ. 19:203; Barbieri et al. (2003) Am J Hypertens. 16:537; Venn et al. (1993) Arthritis Rheum. 36:819; Westacott et al. (1994) J Rheumatol. 21:1710).

The term "diabetic ulcerations," as used herein, refers to an ulcer which results as a complication of diabetes. An ulcer is a crater-like lesion on the skin or mucous membrane caused by an inflammatory, infectious, malignant condition, or metabolic disorder. Typically diabetic ulcers can be found on limbs and extremeties, more typically the feet. These ulcers, caused by diabetic conditions, such as neurapthy and a vacualr insuffciency, can lead to ischemia and poor wound healing. More extensive ulcerations may progress to ostemyelitis. Once ostemyelitis develops, it may be dificulte to eradicate with antibotics alonda nd amputation may be necessary..

The term "retinopathy ulcerations," as used herein refers to an ulcer which causes or results in damages to the eye and the eye's retina. Retinopathy ulcerations may include conditions such has retinoathic hemmorages.

d. *Diabetic Macrovasculopathy*

Tumor necrosis factor has been implicated in the pathophysiology of diabetic macrovasculopathy (Devaraj et al. (2000) Circulation. 102:191; Hattori Y et al. (2000) Cardiovasc Res. 46:188; Clausell Net al. (1999) Cardiovasc Pathol.8:145). The term "diabetic macrovasculopathy," also referred to as "macrovascular disease," as used herein, refers to a disease of the blood vessels that results from diabetes. Diabetic macrovasculopathy complication occurs when, for example, fat and blood clots build up in the large blood vessels and stick to the vessel walls. Diabetic macrovasculopathies include diseases such as coronary disease, cerebrovascular disease, and peripheral vascular disease, hyperglycaemia and cardiovascular disease, and strokes.

2. *Obesity*

Tumor necrosis factor has been implicated in the pathophysiology of obesity (see *e.g.*, Pihlajamaki J et al. (2003) Obes Res.11:912; Barbieri et al. (2003) Am J Hypertens. 16:537; Tsuda et al. (2003) J Nutr. 133:2125). Obesity increases a person's risk of illness and death due to diabetes, stroke, coronary artery disease, hypertension, high cholesterol, and kidney and gallbladder disorders. Obesity may also increase the risk for some types of cancer, and may be a risk factor for the development of osteoarthritis and sleep apnea. Obesity can be treated with the antibody of the invention alone or in combination with other metabolic disorders, including diabetes.

E. *Anemia*

TNFα has been implicated in the pathophysiology of a wide variety of anemias (see *e.g.,* Jongen-Lavrencic M., et al. (1997) J. Rheumatol. 24(8):1504-9; Demeter J., et al. (2002) Ann Hematol. 81(10):566-9; DiCato M., (2003) The Oncologist 8 (suppl 1):19-21). The invention provides methods for inhibiting TNFα activity in a subject suffering from such a disorder, which method comprises administering to the subject an antibody, antibody portion, or other TNFα inhibitor of the invention such that TNFα activity in the subject suffering from anemia is inhibited. In one embodiment, the anemia is associated with rheumatoid arthritis.

The term "anemia" as used herein, refers to an abnormally low number of circulating red cells or a decreased concentration of hemoglobin in the blood. Examples of anemia related to rheumatoid arthritis include, for example, anemia of chronic disease, iron deficiency anemia, and autoimmune hemolytic anemia. In one embodiment, the invention provides a method of treating anemias related to, for example, anemias related to rheumatoid arthritis, anemias of infection and chronic inflammatory diseases, iron deficiency anemia, autoimmune hemolytic anemia, myelophthisic anemia, aplastic anemia, hypoplastic anemia, pure red cell aplasia and anemia associated with renal failure or endocrine disorders, megaloblastic anemias, defects in heme or globin synthesis, anemia caused by a structural defect in red blood cells, e.g., sickle-cell anemia, and anemias of unknown origins such as sideroblastic anemia, anemia associated with chronic infections such as malaria, trypanosomiasis, HIV, hepatitis virus or other viruses, and myelophthisic anemias caused by marrow deficiencies.

F. *Pain*

TNFα has been implicated in the pathophysiology of a wide variety of pain syndromes (see *e.g.*, Sorkin, LS. et al., (1997) Neuroscience. 81(1):255-62; Huygen FJ., et al. (2002) Mediators Inflamm. 11 (1):47-51; Parada CA., et al. (2003) Eur J Neurosci.17(9):1847-52). The invention provides methods for inhibiting TNFα activity in a subject suffering from such a pain disorder, which method comprises administering to the subject an antibody, antibody portion, or other TNFα inhibitor of the invention such that TNFα activity in the subject suffering from pain is inhibited. Pain has been defined in a variety of ways, including nociceptive pain and neuropathic pain. The most commonly experienced form of pain may be defined as the effect of a stimulus on nerve endings, which results in the transmission of impulses to the cerebrum. Pain is also commonly associated with inflammatory disorders, including, for example, rheumatoid arthritis. In one embodiment, the antibody of the invention is used to treat a subject who suffers from pain associated with rheumatoid arthritis. Examples of pain disorders in which TNFα activity is detrimental are discussed further below.

1. *Neuropathic Pain*

Tumor necrosis factor has been implicated in the pathophysiology of neuropathic pain (see Sommer C., (1999) Schmerz. 13(5):315-23; Empl M et al., (2001) Neurology. 56(10):1371-7; Schafers M et al., (2003) J Neurosci. 23(7):3028-38). As used herein the term "neuropathic pain" refers to pain that results from injury to a nerve, spinal cord, or brain, and often involves neural supersensitivity. Examples of neuropathic pain include chronic lower back pain, pain associated with arthritis, cancer-associated pain, herpes neuralgia, phantom limb pain, central pain, opioid resistant neuropathic pain, bone injury pain, and pain during labor and delivery. Other examples of neuropathic pain include post-operative pain, cluster headaches, dental pain, surgical pain, pain resulting from severe, for example third degree, burns, post partum pain, angina pain, genitourinary tract related pain, and including cystitis.

Neuropathic pain is distinguished from nociceptive pain. Pain involving a nociceptive mechanism usually is limited in duration to the period of tissue repair and generally is alleviated by available analgesic agents or opioids (Myers, Regional Anesthesia 20:173-184 (1995)). Neuropathic pain typically is long-lasting or chronic and often develops days or months following an initial acute tissue injury. Neuropathic pain can involve persistent, spontaneous pain as well as allodynia, which is a painful response to a stimulus that normally is not painful. Neuropathic pain also can be characterized by hyperalgesia, in which there is an accentuated response to a painful stimulus that usually is trivial, such as a pin prick. Unlike nociceptive pain, neuropathic pain generally is resistant to opioid therapy (Myers, supra, 1995).

Accordingly, the antibody, or antigen-binding fragment thereof, of the invention can be used to treat neuropathic pain.

2. *Nociceptive pain*

As used herein the term "nociceptive pain" refers to pain that is transmitted across intact neuronal pathways, *i.e.*, pain caused by injury to the body. Nociceptive pain includes somatic sensation and normal function of pain, and informs the subject of impending tissue damage. The nociceptive pathway exists for protection of the subject, e.g., the pain experienced in response to a burn). Nociceptive pain includes bone pain, visceral pain, and pain associated with soft tissue.

Tumor necrosis factor has been implicated in the pathophysiology of visceral pain (see Coelho A., et al. (2000) Am J Physiol Gastrointest Liver Physiol. 279:G781-G790; Coelho A, et al. (2000) Brain Res Bull. 52(3):223-8). Visceral pain is used to refer to nociceptive pain that is mediated by receptors on A-delta and C nerve fibers. A-delta and C-nerve fibers are which are located in skin, bone, connective tissue, muscle and viscera. Visceral pain can be vague in distribution, spasmodic in nature and is usually described as deep, aching, squeezing and colicky in nature. Examples of visceral pain include pain associated with a heart attack, wherein the visceral pain can be felt in the arm, neck and/or back, and liver capsule pain, wherein the visceral pain can be felt in the back and/or right shoulder. Accordingly, the TNFα antibody, or antigen-binding fragment thereof, of the invention can be used to treat visceral pain.

G. *Hepatic Disorders*

TNFα has been implicated in the pathophysiology of a wide variety of hepatic disorders (see *e.g.*, Colletti LM., et al. (1990) J Clin Invest. 85(6):1936-43; Tiegs G. (1997) Acta Gastroenterol Belg. 60(2):176-9; Fernandez ED., et al. (2000) J Endotoxin Res. 6(4):321-8). The invention provides methods for TNFα activity in a subject suffering from such a hepatic disorder, which method comprises administering to the subject an antibody, antibody portion, or other TNFα inhibitor of the invention such that TNFα activity in the subject suffering from a hepatic disorder is inhibited.

As used herein, the term "a hepatic disorder in which TNFα activity is detrimental" is intended to include diseases and other disorders of the liver in which the presence of TNFα in a subject suffering from the disorder has been shown to be or is suspected of being either responsible for the pathophysiology of the disorder or a factor that contributes to a worsening of the disorder. Accordingly, a hepatic disorder in which TNFα activity is detrimental is a disorder in which inhibition of TNFα activity is expected to alleviate the symptoms and/or progression of the hepatic disorder.

Hepatic disorders include many diseases and disorders wherein the liver functions improperly or ceases to function. Hepatocellular injuries can include alcoholic cirrhosis, α1 antitypsin deficiency, autoimmune cirrhosis, cryptogenic cirrhosis, fulminant hepatitis, hepatitis B and C, and steatohepatitis. Examples of biliary tract disorders include cystic fibrosis, primary biliary cirrhosis, sclerosing cholangitis and biliary obstruction (Wiesner, R. H, Current Indications, Contra Indications and Timing for Liver Transplantation (1996), in Transplantation of the Liver, Saunders (publ.); Busuttil, R. W. and Klintmalm, G. B. (eds.) Chapter 6, e.g., Tables 6-3 and 6-5 as well as FIGS. 6-11; Klein, A. W., (1998) Partial Hypertension: The Role of Liver Transplantation, Musby (publ.) in Current Surgical Therapy 6.sup.th Ed. Cameron, J. (ed).

The term "hepatitis" refers to inflammation of the liver. Hepatitis can be caused by infections with various organisms, including bacteria, viruses (Hepatitis A, B, C, etc.), or parasites. Chemical toxins such as alcohol, drugs, or poisonous mushrooms can also damage the liver and cause it to become inflamed. A rare but extremely dangerous cause of hepatitis results from overdose of acetaminophen (Tylenol), which can be deadly. In addition, immune cells in the body may attack the liver and cause autoimmune hepatitis. Hepatitis may resolve quickly (acute hepatitis), or cause long-term disease (chronic hepatitis). In some instances, progressive liver damage or liver failure may result. The incidence and severity of hepatitis vary depending on many factors, including the cause of the liver damage and any underlying illnesses in a patient.

In one embodiment, the invention features a method for treating a hepatic disorder in which TNFα activity is detrimental, comprising administering to a subject an effective amount of a TNFα inhibitor, such that said disorder is treated. In one embodiment, the hepatic disorder is selected from the group consisting of hepatitis C virus, autoimmune hepatitis, fatty-liver disease, hepatitis B virus, hepatotoxicity, and non-alcoholic hepatitis, including non-alcoholic steatohepatitis (NASH). Examples of hepatic disorders are further described below.

1. *Hepatitis C Virus (HCV)*

Tumor necrosis factor has been implicated in the pathophysiology of the hepatitis C virus (see Gonzalez-Amaro. (1994) J Exp Med. 179:841-8; Nelson DR, et al. (1997) Dig Dis Sci 42:2487-94; Kallinowski B, et al. (1998) Clin Exp Imnunol. 111:269-77). The term "hepatitis C virus" or "HCV" is used to describe the hepatitis virus which is the causative agent of non-A, non-B hepatitis. Hepatitis C virus causes an inflammation of the liver. HCV infection causes hepatitis C. Hepatitis C in the acute stage is, in general, milder than hepatitis B, but a greater proportion of such infections become chronic. HCV is a major cause of acute hepatitis and chronic liver disease, including cirrhosis and liver cancer. HCV is one of the viruses (A, B, C, D, and B), which together account for the vast majority of cases of viral hepatitis. It is an enveloped RNA virus in the flaviviridae family which appears to have a narrow host range. An important feature of the virus is the relative mutability of its genome, which in turn is probably related to the high propensity (80%) of inducing chronic infection. HCV is clustered into several distinct genotypes which may be important in determining the severity of the disease and the response to treatment. In one embodiment, the TNFα antibody, or antigen-binding fragment thereof, of the invention can be used to treat HCV.

In one embodiment, subjects who are infected with HCV are treated with the TNFα antibody of the invention. Symptoms of HCV infection (hepatitis C) include at least one of the following: jaundice, abdominal pain (especially in the right upper abdomen), fatigue, loss of appetite, nausea and vomiting, low-grade fever, pale or clay-colored stools, dark urine, and generalized itching. However, it should be noted that many people who are infected with the hepatitis C do not have symptoms, as hepatitis C is often detected during blood tests for a routine physical or other medical procedure.

*2. Autoimmune Hepatitis (AIH)*

Tumor necrosis factor has been implicated in the pathophysiology of autoimmune hepatitis (see Cookson S. et al., (1999) Hepatology 30(4):851-6; Jazrawi S. et al., (2003) Liver Transpl. 9(4):377-82). As used herein, "autoimmune hepatitis" refers to a hepatic disorder characterized by inflammation of the liver caused by rogue immune cells that mistake the liver's normal cells for a foreign tissue or pathogen (disease-causing agent). Autoimmune hepatitis is often responsible for a progressive destruction of the hepatic parenchyma with a high mortality if left untreated (Johnson P. J. et al., (1993) Hepatology, 18:998-1005). One of the characteristics of autoimmune hepatitis is the presence of circulating autoantibodies in almost 90% of patients' sera. Such antibodies can be used to identify subjects who have autoimmune hepatitis.

Clinical and serological differences between patients have lead to the classification of AIH into two types. Type 1 is characterized by the presence of anti-smooth muscle (SMA) and/or anti-nuclear antibodies (ANA) in patients' sera, while sera from Type II patients show anti-liver kidney microsomal antibodies type 1 (LKM1) (Homberg J. C. et al., (1987) Hepatology, 7:1333-1339; Maggiore G. et al., (1993) J. Pediatr. Gastroenterol Nutr., 17:376-381). A serological marker, anti-liver cytosol type I antibodies (LC1), has been identified in 30% of patients with an AIH type II. In addition, LC1 proved to be the only serological marker in 10% of patients tested (Martini E. et al., (1988) Hepatology, 8:1662-1666). In one embodiment, the TNFα antibody, or antigen-binding fragment thereof, of the invention is used to treat AIH.

*3. Fatty-liver disease*

Tumor necrosis factor has been implicated in the pathophysiology of fatty-liver disease (see Valenti L. et al., (2002) Gastrocnerology 122(2):274-80; Li Z. et al., (2003) Hepatology 37(2):343-50). Fatty-liver disease refers to a disease wherein fat (hepatocytes) is excessively accumulated in the liver. Fatty liver disease is believed to be caused by supernutrition, hyperingestion of alcohol, diabetes and side effects due to administration of pharmaceuticals. Fatty liver disease can cause severe diseases such as chronic hepatitis and hepatic cirrhosis. In patients with fatty liver disease, lipids, particularly neutral fat, accumulate in hepatocytes to the extent that the amount exceeds the physiologically permissible range. From a biochemical point of view, a standard for judgment of fatty liver is that the weight of neutral fat is about 10% (100 mg/g wet weight) or more of the wet weight of hepatic tissue. In one embodiment, the TNFα antibody, or antigen-binding fragment thereof, of the invention can be used to treat fatty liver disease.

*4. Hepatitis B Virus (HBV)*

Tumor necrosis factor has been implicated in the pathophysiology of hepatitis B virus (see Kasahara S. et al., (2003) J Virol 77(4):2469-76; Wang F.S., (2003) World J Gastroenterol. 9(4):641-4;Biermer M. et al., (2003) J Virol. 77(7):4033-42). The term "hepatitis B virus" (HBV) is used to describe the virus (serum hepatitis virus) which produces viral hepatitis type B in humans. This is a viral disease with a long incubation period (about 50 to 160 days) in contrast to hepatitis A virus (infectious hepatitis virus) which has a short incubation period. The hepatitis B virus is usually transmitted by injection of infected blood or blood derivatives or merely by use of contaminated needles, lancets or other instruments. Clinically and pathologically, the disease is similar to viral hepatitis type A; however, there is no cross-protective immunity. Viral antigen (HBAg) is found in the serum after infection.

Hepatitis B virus infects humans at a very high rate. Most people who become infected with Hepatitis B get rid of the virus within 6 months, wherein a short infection is known as an "acute" case of Hepatitis B. It is estimated that at least about 300 million people are chronic carriers of HBV. Infection with the virus results in a range of clinical symptoms including minor flu-like symptoms to death. In one embodiment, the TNFα antibody, or antigen-binding fragment thereof, of the invention can be used to treat HBV infection.

*5. Hepatotoxicity*

Tumor necrosis factor has been implicated in the pathophysiology of hepatotoxicity (see Bruccoleri A. et al., (1997) Hepatology 25(1):133-41; Luster M.I. et al., (2000) Ann NY Acad Sci. 919:214-20; Simeonova P. et al., (2001) Toxicol Appl Pharmacol. 177(2):112-20). The term hepatotoxicity refers to liver damage caused by medications and other chemicals or drugs. The best indicator for identifying liver toxicity in a subject is the elevation of certain enzyme measurements in the blood, such as AST (aspartate aminotransferase), ALT (alanine aminotransferase), and GOT (glutamate oxalacetate transaminase).

Hepatotoxicity can cause permanent injury and death. Initial symptoms of hepatotoxicity can include acute gastrointestinal symptoms, e.g., severe diarrhea. The second phase of hepatotoxicity is characterized by abatement of symptoms. During this apparent subsidence, biochemical evidence of hepatic injury appears. Oliguria (decreased urine output) is usual during the second phase. The third phase, that of overt hepatic damage, becomes clinically apparent 3 to 5 days after ingestion of the chemical, with the appearance of jaundice. Renal failure may also occur. The symptoms of chemically-induced (drug-induced) hepatitis are similar to that of infectious hepatitis. In one embodiment, the TNFα antibody, or antigen-binding fragment thereof, of the invention can be used to treat hepatotoxicity.

6. *Liver failure (e.g. chronic liver failure)*

Tumor necrosis factor has been implicated in the pathophysiology of liver failure (e.g. chronic liver failure) (see Takenaka K. et al., (1998) Dig Dis Sci. 43(4):887-92; Nagaki M. et al., (1999) J Hepatol. 31(6):997-1005; Streetz K. et al., (2000) Gastroenterology. 119(2):446-60. Liver failure, including chronic liver failure, usually develops over a period of years and is caused by a repeated insult to the liver (such as alcohol abuse or infection with hepatitis virus) which slowly damages the organ. Less commonly, liver failure is acute, and occurs over a period of days or weeks. Causes of acute liver failure include hepatitis virus infections, drugs, pregnancy, autoimmune disease, and sudden low blood flow to the liver. In one embodiment, the TNFα antibody, or antigen-binding fragment thereof, of the invention can be used to liver failure.

7. *Non-alcoholic hepatitis, including NASH*

Tumor necrosis factor has been implicated in the pathophysiology of non-alcoholic hepatitis, including nonalcoholic steatohepatitis (see Crespo J. et al., (2001) Hepatology. 34(6):1158-63;PessayreD. *et al.,* (2002) 282(2):G193-9). The term "nonalcoholic steatohepatitis" or "NASH" refers to the development of histologic changes in the liver that are comparable to those induced by excessive alcohol intake, but in the absence of alcohol abuse. NASH is characterized by macrovesicular and/or microvesicular steatosis, lobular and portal inflammation, and occasionally Mallory bodies with fibrosis and cirrhosis. NASH is also commonly associated with hyperlipidemia, obesity, and type II diabetes mellitus.

Additional clinical conditions which characterize hepatic steatosis and inflammation include excessive fasting, jejunoileal bypass, total parental nutrition, chronic hepatitis C, Wilson's disease, and adverse drug effects such as those from corticosteroids, calcium channel blockers, high dose synthetic estrogens, methotrexate and amiodarone. Thus, the term "nonalcoholic steatohepatitis" can be used to describe those patients who exhibit these biopsy findings, coupled with the absence of (a) significant alcohol consumption, (b) previous surgery for weight loss, (c) history of drug use associated with steatohepatitis, (d) evidence of genetic liver disease or (e) chronic hepatitis C infection (see, Ludwig, J. R. et al., (1980) Mayo Clin. Proc., 55:434; Powell E. et al., (1990) Hepatol., 11:74). In one embodiment, the TNFα antibody, or antigen-binding fragment thereof, of the invention can be used to treat NASH.

H. *Skin and Nail Disorders*

In one ambodiment, the TNFα antibody of the invention is used to treat skin and nail disorders. As used herein, the term "skin and nail disorder in which TNFα activity is detrimental" is intended to include skin and/or nail disorders and other disorders in which the presence of TNFα in a subject suffering from the disorder has been shown to be or is suspected of being either responsible for the pathophysiology of the disorder or a factor that contributes to a worsening of the disorder, *e.g.*, psoriasis. Accordingly, skin and nail disorders in which TNFα activity is detrimental are disorders in which inhibition of TNFα activity is expected to alleviate the symptoms and/or progression of the disorder. The use of the antibodies, antibody portions, and other TNFα inhibitors of the invention in the treatment of specific skin and nail disorders is discussed further below. In certain embodiments, the antibody, antibody portion, or other TNFα inhibitor of the invention is administered to the subject in combination with another therapeutic agent, as described below in Section III. In one embodiment, the TNFα antibody of the invention is administered to the subject in combination with another therapeutic agent for the treatment of psoriasis and the treatment of psoriasis associated with arthritis.

*1. Psoriasis*

Tumor necrosis factor has been implicated in the pathophysiology of psoriasis (Takematsu et al. (1989) Arch Dermatol Res. 281:398; Victor and Gottlieb (2002) J Drugs Dermatol 1(3):264). Psoriasis is described as a skin inflammation (irritation and redness) characterized by frequent episodes of redness, itching, and thick, dry, silvery scales on the skin. In particular, lesions are formed which involve primary and secondary alterations in epidermal proliferation, inflammatory responses of the skin, and an expression of regulatory molecules such as lymphokines and inflammatory factors. Psoriatic skin is morphologically characterized by an increased turnover of epidermal cells, thickened epidermis, abnormal keratinization, inflammatory cell infiltrates into the epidermis and polymorphonuclear leukocyte and lymphocyte infiltration into the epidermis layer resulting in an increase in the basal cell cycle. Psoriasis often involves the nails, which frequently exhibit pitting, separation of the nail, thickening, and discoloration. Psoriasis is often associated with other inflammatory disorders, for example arthritis, including rheumatoid arthritis, inflammatory bowel disease (IBD), and Crohn's disease.

Evidence of psoriasis is most commonly seen on the trunk, elbows, knees, scalp, skin folds, or fingernails, but it may affect any or all parts of the skin. Normally, it takes about a month for new skin cells to move up from the lower layers to the surface. In psoriasis, this process takes only a few days, resulting in a build-up of dead skin cells and formation of thick scales. Symptoms of psoriasis include: skin patches, that are dry or red, covered with silvery scales, raised patches of skin, accompanied by red borders, that may crack and become painful, and that are usually lovated on the elbows, knees, trunk, scalp, and hands; skin lesions, including pustules, cracking of the skin, and skin redness; joint pain or aching which maybe associated with of arthritis, e.g., psoriatic arthritis.

Treatment for psoriasis often includes a topical corticosteroids, vitamin D analogs, and topical or oral retinoids, or combinations thereof In one embodiment, the TNFα inhibitor of the invention is administered in combination with or the presence of one of these common treatments. Additional therapeutic agents which can also be combined with the TNFα inhibitor of the invention for treatment of psoriasis are described in more detail in Section III.B.

The diagnosis of psoriasis is usually based on the appearance of the skin. Additionally a skin biopsy, or scraping and culture of skin patches may be needed to rule out other skin disorders. An x-ray may be used to check for psoriatic arthritis if joint pain is present and persistent.

In one embodiment of the invention, a TNFα inhibitor is used to treat psoriasis, including chronic plaque psoriasis, guttate psoriasis, inverse psoriasis, pustular psoriasis, pemphigus vulgaris, erythrodermic psoriasis, psoriasis associated with inflammatory bowel disease (IBD), and psoriasis associated with rheumatoid arthritis (RA). Specific types of psoriasis included in the treatment methods of the invention are described in detail below:

a. *Chronic plaque psoriasis*

Tumor necrosis factor has been implicated in the pathophysiology of chronic plaque psoriasis (Asadullah et al. (1999) Br J Dermatol. 141:94). Chronic plaque psoriasis (also referred to as psoriasis vulgaris) is the most common form of psoriasis. Chronic plaque psoriasis is characterized by raised reddened patches of skin, ranging from coin-sized to much larger. In chronic plaque psoriasis, the plaques may be single or multiple, they may vary in size from a few millimeters to several centimeters. The plaques are usually red with a scaly surface, and reflect light when gently scratched, creating a "silvery" effect Lesions (which are often symmetrical) from chronic plaque psoriasis occur all over body, but with predilection for extensor surfaces, including the knees, elbows, lumbosacral regions, scalp, and nails. Occasionally chronic plaque psoriasis can occur on the penis, vulva and flexures, but scaling is usually absent. Diagnosis of patients with chronic plaque psoriasis is usually based on the clinical features described above. In particular, the distribution, color and typical silvery scaling of the lesion in chronic plaque psoriasis are characteristic of chronic plaque psoriasis.

b. *Guttate psoriasis*

Guttate psoriasis refers to a form of psoriasis with characteristic water drop shaped scaly plaques. Flares of guttate psoriasis generally follow an infection, most notably a streptococcal throat infection. Diagnosis of guttate psoriasis is usually based on the appearance of the skin, and the fact that there is often a history of recent sore throat.

c. *Inverse psoriasis*

Inverse psoriasis is a form of psoriasis in which the patient has smooth, usually moist areas of skin that are red and inflamed, which is unlike the scaling associated with plaque psoriasis. Inverse psoriasis is also referred to as intertiginous psoriasis or flexural psoriasis. Inverse psoriasis occurs mostly in the armpits, groin, under the breasts and in other skin folds around the genitals and buttocks, and, as a result of the locations of presentation, rubbing and sweating can irriate the affected areas.

d. *Pustular psoriasis*

Pustular psoriasis is a form of psoriasis that causes pus-filled blisters that vary in size and location, but often occur on the hands and feet. The blisters may be localized, or spread over large areas of the body. Pustular psoriasis can be both tender and painful, can cause fevers.

e. *Other psoriasis disorders*

Other examples of psoriatic disorders which can be treated with the TNF$\alpha$ antibody of the invention include erythrodermic psoriasis, vulgaris, psoriasis associated with IBD, and psoriasis associated with arthritis, including rheumatoid arthritis.

*2. Pemphigus vulgaris*

Pemphigus vulgaris is a serious autoimmune systemic dermatologic disease that often affects the oral mucous membrane and skin. The pathogenesis of pemphigus vulgaris is thought to be an autoimmune process that is directed at skin and oral mucous membrane desmosomes. Consequentially, cells do not adhere to each other. The disorder manifests as large fluid-filled, rupture-prone bullae, and has a distinctive histologic appearance. Anti-inflammatory agents are the only effective therapy for this disease which has a high mortality rate. Complications that arise in patients suffering from pemphigus vulgaris are intractable pain, interference with nutrition and fluid loss, and infections.

*3. Atopic dermatitis / eczema*

Atopic dermatitis (also referred to as eczema) is a chronic skin disorder categorized by scaly and itching plaques. People with eczema often have a family history of allergic conditions like asthma, hay fever, or eczema. Atopic dermatitis is a hypersensitivity reaction (similar to an allergy) which occurs in the skin, causing chronic inflammation. The inflammation causes the skin to become itchy and scaly. Chronic irritation and scratching can cause the skin to thicken and become leathery-textured. Exposure to environmental irritants can worsen symptoms, as can dryness of the skin, exposure to water, temperature changes, and stress.
Subjects with atopic dermatitis can be identified by certain symptoms, which often include intense itching, blisters with oozing and crusting, skin redness or inflammation around the blisters, rash, dry, leathery skin areas, raw areas of the skin from scratching, and ear discharges/bleeding.

*4. Sarcoidosis*

Sarcoidosis is a disease in which granulomatous inflammation occurs in the lymph nodes, lungs, liver, eyes, skin, and/or other tissues. Sarcoidosis includes cutaneous sarcoidosis (sarcoidosis of the skin) and nodular sarcoidosis (sarcoidosis of the lymph nodes). Patients with sarcoidosis can be identified by the symptoms, which often include general discomfort, uneasiness, or an ill feeling; fever; skin lesions.

*5. Erythema nodosum*

Erythema nodosum refers to an inflammatory disorder that is characterized by tender, red nodules under the skin, typically on the anterior lower legs. Lesions associated with erythema nodosum often begin as flat, but firm, hot red painful lumps (approximately an inch across). Within a few days the lesions may become purplish, and then over several weeks fade to a brownish flat patch.
In some instances, erythema nodosum may be associated with infections including, streptococcus, coccidioidomycosis, tuberculosis, hepatitis B, syphilis, cat scratch disease, tularemia, yersinia, leptospirosis psittacosis, histoplasmosis, mononucleosis (BBV). In other instances, erythema nodosum may be associated with sensitivity to certain medications including, oralcontraceptives, penicillin, sulfonamides, sulfones, barbiturates, hydantoin, phenacetin, salicylates, iodides, and progestin. Erythema nodosum is often associated with other disorders including, leukemia, sarcoidosis, rheumatic fever, and ulcerative colitis.
Symptoms of erythema nodosum usually present themselves on the shins, but lesions may also occur on other areas of the body, including the buttocks, calves, ankles, thighs and upper extremities. Other symptoms in subjects with erythema nodosum can include fever and malaise.

### 6. *Hidradenitis suppurative*

Hidradenitis suppurativa refers to a skin disorder in which swollen, painful, inflamed lesions or lumps develop in the groin and sometimes under the arms and under the breasts. Hidradenitis suppurativa occurs when apocrine gland outlets become blocked by perspiration or are unable to drain normally because of incomplete gland development. Secretions trapped in the glands force perspiration and bacteria into surrounding tissue, causing subcutaneous induration, inflammation, and infection. Hidradenitis suppurativa is confined to areas of the body that contain apocrine glands. These areas are the axillae, areola of the nipple, groin, perineum, circumanal, and periumbilical regions.

### 7. *Lichen planus*

Tumor necrosis factor has been implicated in the pathophysiology of lichen planus (Sklavounou et al. (2000) J Oral Pathol Med. 29:370). Lichen planus refers to a disorder of the skin and the mucous membranes resulting in inflammation, itching, and distinctive skin lesions. Lichen planus may be associated with hepatitis C or certain medications.

### 8. *Sweet's syndrome*

Inflammatory cytokines, including tumor necrosis factor, have been implicated in the pathophysiology of Sweet's syndrome (Reuss-Borst et al. (1993) Br J Haematol. 84:356). Sweet's syndrome, which was described by R.D. Sweet in 1964, is characterized by the sudden onset of fever, leukocytosis, and cutaneous eruption. The eruption consists of tender, erythematous, well-demarcated papules and plaques which show dense neutrophilic infiltrates microscopically. The lesions may appear anywhere, but favor the upper body including the face. The individual lesions are often described as pseudovesicular or pseudopustular, but may be frankly pustular, bullous, or ulcerative. Oral and eye involvement (conjunctivitis or episcleritis) have also been frequently reported in patients with Sweet's syndrome. Leukemia has also been associated with Sweet's syndrome.

### 9. *Vitiligo*

Vitiligo refers to a skin condition in which there is loss of pigment from areas of skin resulting in irregular white patches with normal skin texture. Lesions characteristic of vitiligo appear as flat depigmented areas. The edges of the lesions are sharply defined but irregular. Frequently affected areas in subjects with vitiligo include the face, elbows and knees, hands and feet, and genitalia.

### 10. *Scleroderma*

Tumor necrosis factor has been implicated in the pathophysiology of scleroderma (Tutuncu Z et al. (2002) Clin Exp Rheumatol. 20(6 Suppl 28):S146-51; Mackiewicz Z et al. (2003) Clin Exp Rheumatol. 21(1):41-8; Murota H et al. (2003) Arthritis Rheum. 48(4):1117-25). Scleroderma refers to a a diffuse connective tissue disease characterized by changes in the skin, blood vessels, skeletal muscles, and internal organs. Scleroderma is also referred to as CREST syndrome or Progressive systemic sclerosis, and usually affects people between the ages 30-50. Women are affected more often than men.

The cause of scleroderma is unknown. The disease may produce local or systemic symptoms. The course and severity of the disease varies widely in those affected.Excess collagen deposits in the skin and other organs produce the symptoms. Damage to small blood vessels within the skin and affected organs also occurs. In the skin, ulceration, calcification, and changes in pigmentation may occur. Systemic features may include fibrosis and degeneration of the heart, lungs, kidneys and gastrointestinal tract.

Patients suffering from scleroderma exhibit certain clinical features, including, blanching, blueness, or redness of fingers and toes in response to heat and cold (Raynaud's phenomenon), pain, stiffness, and swelling of fingers and joints, skin thickening and shiny hands and forearm, esophageal reflux or heartburn, difficulty swallowing, and shortness of breath. Other clinical sypmtoms used to diagnose scleroderma include, an elevated erythrocyte sedimentaion rate (ESR), an elevated rheumatoid factor (RF), a positive antinuclear antibody test, urinalysis that shows protein and microscopic blood, a chest X-ray that may show fibrosis, and pulmonary funtion studies that show restricitive lung disease.

### 11. *Nail disorders*

Nail disorders include any abnormality of the nail. Specific nail disorders include, but are not limited to, pitting, koilonychia, Beau's lines, spoon nails, onycholysis, yellow nails, pterygium (seen in lichen planus), and leukonychia. Pitting is characterised by the presence of small depressions on the nail surface. Ridges or linear elevations can develop along the nail occurring in a "lengthwise" or "crosswise" direction. Beau's lines are linear depressions that occur "crosswise" (transverse) in the fingernail. Leukonychia describes white streaks or spots on the nails. Koilonychia is an abnormal shape of the fingernail where the nail has raised ridges and is thin and concave Koilonychia is often associated with iron deficiency.

Nail disorders which can be treated with the TNFα antibody of the invention also include psoriatic nails. Psoriatic nails include changes in nails which are attributable to psoriasis. In some instances psoriasis may occur only in the nails and nowhere else on the body. Psoriatic changes in nails range from mild to severe, generally reflecting the extent of psoriatic involvement of the nail plate, nail matrix, *i.e.*, tissue from which the nail grows, nail bed, *i.e.*, tissue under the nail, and skin at the base of the nail. Damage to the nail bed by the pustular type of psoriasis can result in loss of the nail. Nail changes in psoriasis fall into general categories that may occur singly or all together. In one category of psoriatic nails, the nail plate is deeply pitted, probably due to defects in nail growth caused by psoriasis. IN another category, the nail has a yellow to yellow-pink discoloration, probably due to psoriatic involvement of the nail bed. A third subtype of psoriatic nails are characterized by white areas which appear under the nail plate. The white areas are actually air bubbles marking spots where the nail plate is becoming detached from the nail bed. There may also be reddened skin around the nail. A fourth category is evidenced by the nail plate crumbling in yellowish patches, *i.e.*, onychodystrophy, probably due to psoriatic involvement in the nail matrix. A fifth category is characterized by the loss of the nail in its entirety due to psoriatic involvement of the nail matrix and nail bed.

The TNFα antibody of the invention can also be used to treat nail disorders often associated with lichen planus. Nails in subjects with lichen planus often show thinning and surface roughness of the nail plate with longitudinal ridges or pterygium.

The TNFα antibody of the invention can be used to treat nail disorders, such as those described herein. Often nail disorders are associated with skin disorders. In one embodiment, the invention includes a method of treatment for nail disorders with a TNFα antibody. In another embodiment, the nail disorder is associated with another disorder, including a skin disorder such as psoriasis. In another embodiment, the disorder associated with a nail disorder is arthritis, including psoriatic arthritis.

12. *Other Skin and Nail Disorders*

The TNFα antibody of the invention can be used to treat other skin and nail disorders, such as chronic actinic dermatitis, bullous pemphigoid, and alopecia areata. Chronic actinic dermatitis (CAD) is also referred to as photosensitivity dermatitis/actinic reticuloid syndrome (PD/AR). CAD is a condition in which the skin becomes inflamed, particularly in areas that have been exposed to sunlight or artificial light. Commonly, CAD patients have allergies to certain substances that come into contact with their skin, particularly various flowers, woods, perfumes, sunscreens and rubber compounds. Bullous pemphigoid refers to A skin disorder characterized by the formation of large blisters on the trunk and extremities. Alopecia areata refers to hair loss characterized by round patches of complete baldness in the scalp or beard.

I. *Vasculitides*

TNFα has been implicated in the pathophysiology of a variety of vasculitides, (see e.g., Deguchi et al. (1989) Lancet. 2:745). In one embodiment, the invention provides a method for inhibiting TNFα activity in a subject suffering from a vasculitis in which TNFα activity is detrimental.

As used herein, the term "a vasculitis in which TNFα activity is detrimental" is intended to include vasculitis in which the presence of TNFα in a subject suffering from the disorder has been shown to be or is suspected of being either responsible for the pathophysiology of the disorder or a factor that contributes to a worsening of the disorder. Such disorders may be evidenced, for example, by an increase in the concentration of TNFα in a biological fluid of a subject suffering from the disorder (e.g., an increase in the concentration of TNFα in serum, plasma, synovial fluid, *etc.* of the subject), which can be detected, for example, using an anti-TNFα antibody as described above.

There are numerous examples of vasculitides in which TNFα activity is detrimental, including Behcet's disease. The use of the antibodies, antibody portions, and other TNFα inhibitors of the invention in the treatment of specific vasculitides are discussed further below. In certain embodiments, the antibody, antibody portion, or other TNFα inhibitor of the invention is administered to the subject in combination with another therapeutic agent, as described below

The antibody of the invention can be used to treat vasculitis in which TNFα activity is detrimental, wherein inhibition of TNFα activity is expected to alleviate the symptoms and/or progression of the vasculitis or to prevent the vasculitis. Subjects suffering from or at risk of developing vasculitis can be identified through clinical symptoms and tests. For example, subjects with vasculitides often develop antibodies to certain proteins in the cytoplasm of neutrophils, antineutrophil cytoplasmic antibodies (ANCA). Thus, in some instances, vasculitides may be evidenced by tests (e.g., ELISA), which measure ANCA presence.

Vasculitis and its consequences may be the sole manifestation of disease or it maybe a secondary component of another primary disease. Vasculitis may be confined to a single organ or it may simultaneously affect several organs. and depending on the syndrome, arteries and veins of all sizes can be affected. Vasculitis can affect any organ in the body.

In vasculitis, the vessel lumen is usually compromised, which is associated with ischemia of the tissues supplied by the involved vessel. The broad range of disorders that may result from this process is due to the fact that any type, size and location of vessel (*e.g.,* artery, vein, arteriole, venule, capillary) can be involved. Vasoulitides are generally classified according to the size of the affected vessels, as described below. It should be noted that some small and large vessel vasculitides may involve medium-sized arteries; but large and medium-sized vessel vasculitides do not involve vessels smaller than arteries. Large vessel disease includes, but is not limited to, giant cell arteritis, also known as temporal arteritis or cranial arteritis, polymyalgia rheumatica, and Takayasu's disease or arteritis, which is also known as aortic arch syndrome, young female arteritis and Pulseless disease. Medium vessel disease includes, but is not limited to, classic polyarthritis nodosa and Kawasaki's disease, also known as mucocutaneous lymph node syndrome. Non-limiting examples of small vessel disease are Behcet's Syndrome, Wegner's granulomatosis, microscopic polyangitis, hypersensitivity vasculitis, also known as cutaneous vasculitis, small vessel vasculitis, Henoch-Schonlein purpura, allergic granulamotosis and vasculitis, also known as Churg Strauss syndrome. Other vasculitides include, but are not limited to, isolated central nervous system vasculitis, and thromboangitis obliterans, also known as Buerger's disease. Classic Polyarteritis nodosa (PAN), microscopic PAN, and allergic granulomatosis are also often grouped together and are called the systemic necrotizing vasculitides. A further description of vasculitis is described below:

*1. Large vessel vasculitis*

In one embodiment, the TNFα antibody of the invention is used to treat subjects who have large vessel vasculitis. The term "large vessel(s)" as used herein, refers to the aorta and the largest branches directed toward major body regions. Large vessels include, for example, the aorta, and its branches and corresponding veins, *e.g.,* the subclavian artery; the brachiocephalic artery; the common carotid artery; the innonimate vein; internal and external jugular veins; the pulmonary arteries and veins; the venae cavae; the renal arteries and veins; the femoral arteries and veins; and the carotid arteries. Examples of large vessel vasculitides are described below.

a. *Giant cell arteritis (GCA)*

Tumor necrosis factor has been implicated in the pathophysiology of giant cell arteritis (Sneller, M.C. (2002) Cleve. Clin. J. Med. 69:SII40-3; Schett, G., et al. (2002) Ann. Rheum. Dis. 61:463). Giant cell arteritis (GCA), refers to a vasculitis involving inflammation and damage to blood vessels, particularly the large or medium arteries that branch from the external carotid artery of the neck. GCA is also referred to as temporal arteritis or cranial arteritis, and is the most common primary vasculitis in the elderly. It almost exclusively affects individuals over 50 years of age, however, there are well-documented cases of patients 40 years and younger. GCA usually affects extracranial arteries. GCA can affect the branches of the carotid arteries, including the temporal artery. GCA is also a systemic disease which can involve arteries in multiple locations.

Histopathologically, GCA is a panarteritis with inflammatory mononuclear cell infiltrates within the vessel wall with frequent Langhans type giant cell formation. There is proliferation of the intima, granulomatous inflammation and fragmentation of the internal elastic lamina. The pathological findings in organs is the result of ischemia related to the involved vessels.

Patients suffering from GCA exhibit certain clinical symptoms, including fever, headache, anemia and high erythrocyte sedimentation rate (ESR). Other typical indications of GCA include jaw or tongue claudication, scalp tenderness, constitutional symptoms, pale optic disc edema (particularly 'chalky white' disc edema), and vision disturbances. The diagnosis is confirmed by temporal artery biopsy.

b. *Polymyalgia rheumatica*

Tumor necrosis factor has been implicated in the pathophysiology of polymyalgia rheumatica (Straub, R.H., et al. (2002) Rheumatology (Oxford) 41:423; Uddhammar, A., et al. (1998) Br. J. Rheumatol.37:766). Polymyalgia rheumatica refers to a rheumatic disorder that is associated with moderate to severe muscle pain and stiffness in the neck, shoulder, and hip, most noticeable in the morning. IL-6 and IL-1β expression has also been detected in a majority of the circulating monocytes in patients with the polymyalgia rheumatica. Polymyalgia rheumatica may occur independently, or it may coexist with or precede GCA, which is an inflammation of blood vessels.

c. *Takayasu's Arteritis*

Tumor necrosis factor has been implicated in the pathophysiology of Takayasu's arteritis (Kobayashi, Y. and Numano, F. (2002) Intern. Med 41:44; Fraga, A. and Medina F. (2002) Curr. Rheumatol. Rep.4:30). Takayasu's arteritis refers to a vasculitis characterized by an inflammation of the aorta and its major branches. Takayasu's arteritis (also known as Aortic arch syndrome, young female arteritis and Pulseless disease) affects the thoracic and abdominal aorta and its main branches or the pulmonary arteries. Fibrotic thickening of the aortic wall and its branches (*e.g.,* carotid, inominate, and subclavian arteries) can lead to reduction of lumen size of vessels that arise from the aortic arch. This condition also typically affects the renal arteries.

Takayasu's arteritis primarily affects young women, usually aged 20-40 years old, particularly of Asian descent, and may be manifested by malaise, arthralgias and the gradual onset of extremity claudication. Most patients have asymmetrically reduced pulses, usually along with a blood pressure differential in the arms. Coronary and/or renal artery stenosis may occur.

The clinical features of Takayasu's arteritis maybe divided into the features of the early inflammatory disease and the features of the later disease. The clinical features of the early inflammatory stage of Takayasu's disease are: malaise, low grade fever, weight loss, myalgia, arthralgia, and erythema multiforme. Later stages of Takayasu's disease are characterised by fibrotic stenosis of arteries and thrombosis. The main resulting clinical features are ischaemic phenomena, e.g. weak and asymmetrical arterial pulses, blood pressure discrepancy between the arms, visual disturbance, *e.g.* scotomata and hemianopia, other neurological features including vertigo and syncope, hemiparesis or stroke. The clinical features result from ischaemia due to arterial stenosis and thrombosis.

2. *Medium Vessel Disease*

In one embodiment, the TNFα antibody of the invention is used to treat subjects who have medium vessel vasculitis. The term "medium vessel(s)" is used to refer to those blood vessels which are the main visceral arteries. Examples of medium vessels include the mesenteric arteries and veins, the iliac arteries and veins, and the maxillary arteries and veins. Examples of medium vessel vasculitides are described below.

a. *Polyarteritis Nodosa*

Tumor necrosis factor has been implicated in the pathophysiology of polyarteritis nodosa (DiGirolamo, N., et al. (1997) J. Leukoc. Biol. 61:667). Polyarteritis nodosa, or periarteritis nodosa refers to vasculitis which is a serious blood vessel disease in which small and medium-sized arteries become swollen and damaged because they are attacked by rogue immune cells. Polyarteritis nodosa usually affects adults more frequently than children. It damages the tissues supplied by the affected arteries because they don't receive enough oxygen and nourishment without a proper blood supply.

Symptoms which are exhibited in patients with polyarteritis nodosa generally result from damage to affected organs, often the skin, heart, kidneys, and nervous system. Generalized symptoms of polyarteritis nodosa include fever, fatigue, weakness, loss of appetite, and weight loss. Muscle aches (myalgia) and joint aches(arthralgia) are common. The skin of subjects with Polyarteritis nodosa may also show rashes, swelling, ulcers, and lumps (nodular lesions).

Classic PAN (polyarteritis nodosa) is a systemic arteritis of small to medium muscular arteritis in which involvement of renal and visceral arteries is common. Abdominal vessels have aneurysms or occlusions in 50% of PAN patients. Classic PAN does not involve the pulmonary arteries although the bronchial vessels may be involved. Granulomas, significant eosinophilia and an allergic diathesis are not part of the syndrome. Although any organ system may be involved, the most common manifestations include peripheral neuropathy, mononeuritis multiplex, intestinal ischemia, renal ischemia, testicular pain and livedo reticularis.

b. *Kawasaki's Disease*

Tumor necrosis factor has been implicated in the pathophysiology of Kawasaki's disease (Sundel, R.P. (2002) Curr. Rheumatol. Rep. 4:474; Gedalia, A.(2002) Curr. Rheumatol. Rep. 4:25). Although the cause of Kawasaki's disease is unknown, it is associated with acute inflammation of the coronary arteries, suggesting that the tissue damage associated with this disease maybe mediated by proinflammatory agents such as TNFα. Kawasaki's disease refers to a vasculitis that affects the mucus membranes, lymph nodes, lining of the blood vessels, and the heart. Kawasaki's disease is also often referred to as mucocutaneous lymph node syndrome, mucocutaneous lymph node disease, and infantile polyarteatis. Subjects afflicted with Kawasaki's disease develop vasculitis often involving the coronary arteries which can lead to myocarditis and pericarditis. Often as the acute inflammation diminishes, the coronary arteries may develop aneurysm, thrombosis, and lead to myocardial infarction.

Kawasaki's disease is a febrile systemic vasculitis associated with edema in the palms and the soles of the feet, with enlargement of cervical lymph nodes, cracked lips and "strawberry tongue". Although the inflammatory response is found in vessels throughout the body, the most common site of end-organ damage is the coronary arteries. Kawasaki's Disease predominantly affects children under the age of 5. The highest incidence is in Japan but is becoming increasingly recognized in the West and is now the leading cause of acquired heart disease in US children. The most serious complication of Kawasaki disease is coronary arteritis and aneurysm formation that occurs in a third of untreated patients.

3. *Small vessel disease*

In one embodiment, the TNFα antibody of the invention is used to treat subjects who have small vessel vasculitis. The term "small vessel(s)" is used to refer to arterioles, venules and capillaries. Arterioles are arteries that contain only 1 or 2 layers of sooth muscle cells and are terminal to and continuous with the capillary network. Venules carry blood from the capillary network to veins and capillaries connect arterioles and venules. Examples of small vessel vasculitides are described below.

a. *Behcet's Disease*

Tumor necrosis factor has been implicated in the pathophysiology of Behcet's disease (Sfikakis, P.P. (2002) Ann. Rheum. Dis. 61:ii51-3; Dogan, D. and Farah, C. (2002) Oftalmologia. 52:23). Behcet's disease is a chronic disorder that involves inflammation of blood vessels throughout the body. Behcet's disease may also cause various types of skin lesions, arthritis, bowel inflammation, and meningitis (inflammation of the membranes of the brain and spinal cord). As a result of Behcet's disease, the subject with the disorder may have inflammation in tissues and organs throughout the body, including the gastrointestinal tract, central nervous system, vascular system, lungs, and kidneys. Behcet's disease is three times more common in males than females and is more common in the east Mediterranean and Japan.
Subjects who have Behcet's disease may show clinical symptoms including recurrent oral ulcers (resembling canker sores), recurrent genital ulcers, and eye inflammation. Serum levels of TNFα, IL-8, IL-1, IL-6 INF-γ and IL-12 are elevated in Behcet's patients, and the production of these factors has been shown to be elevated in the monocytes of Behcet's patients (see, *e.g.,* Inflammatory Disease of Blood Vessels (2001) Marcel Dekker, Inc., eds. G.S. Hoffman and C.M. Weyand, p. 473).

b. *Wegener's granulomatosis*

Tumor necrosis factor has been implicated in the pathophysiology of Wegener's granulomatosis (Marquez, J., et al. (2003) Curr. Rheumatol. Rep. 5:128; Harman, L.E. and Margo, C.E. (1998) Surv. Ophthalmol. 42:458). Wegener's granulomatosis refers to a vasculitis that causes inflammation of blood vessels in the upper respiratory tract (nose, sinuses, ears), lungs, and kidneys. Wegener's granulomatosis is also referred to as midline granulomatosis. Wegener's granulomatosis includes a granulomatous inflammation involving the respiratory tract, and necrotizing vasculitis affecting small to medium-sized vessels. Subjects who have Wegener's *granulomatosis* often also have arthritis (joint inflammation). Glomerulonephritis may also be present in affected subjects, but virtually any organ may be involved.
Patients affected with Wegener's *granulomatosis* typically show clinical symptoms comprising recurrent sinusitis or epistaxis, mucosal ulcerations, otitis media, cough, hemoptysis and dyspnea. The first symptoms of Wegener's *granulomatosis* frequently include upper respiratory tract symptoms, joint pains, weakness, and tiredness.

c. *Churg-Strauss syndrome*

Tumor necrosis factor has been implicated in the pathophysiology of Churg-Strauss syndrome (Gross, W.L (2002) Curr. Opin. Rheumatol. 14:11; Churg, W.A.(2001) Mod. Pathol. 14:1284). *Churg-Strauss* syndrome refers to a vasculitis that is systemic and shows early manifestation signs of asthma and eosinophilia. Churg-Strauss syndrome is also referred to as allergic granulomatosis and angiitis, and occurs in the setting of allergic rhinitis, asthma and eosinophilia. Sinusitis and pulmonary infiltrates also occur in *Churg-Strauss* syndrome, primarily affecting the lung and heart Peripheral neuropathy, coronary arteritis and gastrointestinal involvement are common.
Patients afflicted with *Churg-Strauss* syndrome can be diagnosed according to criteria established by the American College of Rheumatology (ACR). These criteria were intended to distinguish CSS from other forms of vasculitis. Not all patients meet every criterion. Some, in fact, may have only 2 or 3 criteria, yet they are still classified as *Churg-Strauss* syndrome. The ACR selected 6 disease features (criteria) as being those that best distinguished *Churg-Strauss* syndrome from other vasculitides. These criteria include: 1) asthma; 2) eosinophilia [>10% on differential WBC count]; 3) mononeuropathy; 4) transient pulmonary infiltrates on chest X-rays; 5) paranasal sinus abnormalities; and 6) biopsy containing a blood vessel with extravascular eosinophils.

J. *Other TNFα-Related Disorders*

In one embodiment, the invention features a method for treating a TNFα-related disorder in which TNFα activity is detrimental, comprising administering to a subject an effective amount of a TNFα inhibitor, such that said TNFα-related disorder is treated. Examples of TNFα-related disorders in which TNFα activity is detrimental, are discussed further below.

1. *Crohn's Disease-Related Disorders*

Tumor necrosis factor has been implicated in the pathophysiology of inflammatory bowel disorders (IBD), including Crohn's disease (see *e.g.,* Tracy, K.J., et al. (1986) Science 234:470-474; Sun, X-M., et al. (1988) J. Clin. Invest. 81:1328-1331; MacDonald, T.T., et al. (1990) Clin. Exp. Immunol 81:301-305).

In one embodiment, the TNFα inhibitor of the invention is used to treat disorders often associated with IBD and Crohn's disease. The term "inflammatory bowel disorder (IBD)-related disorder" or "Crohn's disease-related disorder," as used interchangeably herein, is used to describe conditions and complications commonly associated with IBD and Crohn's disease. Examples of Crohn's disease-related disorders include fistulas in the bladder, vagina, and skin; bowel obstructions; abscesses; nutritional deficiencies; complications from corticosteroid use; inflammation of the joints; erythem nodosum; pyoderma gangrenosum; and lesions of the eye. Other disorders commonly associated with Crohn's disease include Crohn's-related arthralgias, fistulizing Crohn's, indeterminant colitis, and pouchitis.

2. *Juvenile Arthritis*

Tumor necrosis factor has been implicated in the pathophysiology of juvenile arthritis, including juvenile rheumatoid arthritis (Grom et al. (1996) Arthritis Rheum. 39:1703; Mangge et al. (1995) Arthritis Rheum. 8:211). In one embodiment, the TNFα antibody of the invention is used to treat juvenile rheumatoid arthritis.

The term "juvenile rheumatoid arthritis" or "JRA" as used herein refers to a chronic, inflammatory disease which occurs before age 16 that may cause joint or connective tissue damage. JRA is also referred to as juvenile chronic polyarthritis and Still's disease.

JRA causes joint inflammation and stiffness for more than 6 weeks in a child of 16 years of age or less. Inflammation causes redness, swelling, warmth, and soreness in the joints. Any joint can be affected and inflammation may limit the mobility of affected joints. One type of JRA can also affect the internal organs.

JRA is often classified into three types by the number of joints involved, the symptoms, and the presence or absence of certain antibodies found by a blood test. These classifications help the physician determine how the disease will progress and whether the internal organs or skin is affected. The classifications of JRA include the following

a. Pauciarticular JRA, wherein the patient has four or fewer joints are affected. Pauciarticular is the most common form of JRA, and typically affects large joints, such as the knees.
b. Polyarticular HRA, wherein five or more joints are affected. The small joints, such as those in the hands and feet, are most commonly involved, but the disease may also affect large joints.
c. Systemic JRA is characterized by joint swelling, fever, a light skin rash, and may also affect internal organs such as the heart, liver, spleen, and lymph nodes. Systemic JRA is also referred to as it Still's disease. A small percentage of these children develop arthritis in many joints and can have severe arthritis that continues into adulthood.

3. *Endometriosis*

Tumor necrosis factor has been implicated in the pathophysiology of endometriosis, as women with endometriosis have elevated peritoneal levels of TNF (Eisermann J, et al. (1988) Fertil Steril 50:573; Halme J. (1989) Am J Obstet Gynecol 161:1718; Mori H, et al. (1991) Am J Reprod Immunol 26:62; .Taketani Y, et al. (1992) Am J Obstet Gynecol 167:265; Overton C, at al. (1996) Hum Reprod 1996; 11:380). In one embodiment, the TNFα antibody of the invention is used to treat endometriosis. The term "endometriosis" as used herein refers to a condition in which the tissue that normally lines the uterus (endometrium) grows in other areas of the body, causing pain, irregular bleeding, and frequently infertility.

4. *Prostatitis*

Tumor necrosis factor has been implicated in the pathophysiology of prostatitis, as men with chronic prostatitis and chronic pelvic pain have significantly higher levels of TNF and IL-1 in semen compared to controls (Alexander RB, et al. (1998) Urology 52:744; Nadler RB, et al. (2000) J Urol 164:214; Orhan et al. (2001) Int J Urol 8:495) Furthermore, in a rat model of prostatitis TNF levels were also increased in comparison to controls (Asakawa K, et al. (2001) Hinyokika Kiyo 47:459; Harris et al. (2000) Prostate 44:25). In one embodiment, the TNFα antibody of the invention is used to treat prostatitis.

The term "prostatitis" as used herein refers to an inflammation of the prostate. Prostatitis is also referred to as pelvic pain syndrome. Prostatitis manifests itself in a variety of forms, including nonbacterial prostatitis, acute prostatitis, bacterial prostatitis, and acute prostatitis. Acute prostatitis refers to an inflammation of the prostate gland that develops suddenly. Acute prostatitis is usually caused by a bacterial infection of the prostate gland. Chronic prostatitis is an inflammation of the prostate gland that develops gradually, continues for a prolonged period, and typically has subtle symptoms. Chronic prostatitis is also usually caused by a bacterial infection.

*5. Autoimmune disorders*

Tumor necrosis factor has been implicated in the pathophysiology of many autoimmune disorders, including lupus (Shvidel et al. (2002) Hematol J. 3:32; Studnicka-Benke et al. (1996) Br J Rheumatol. 35:1067). In one embodiment, the TNFα antibody of the invention is used to treat autoimmune disorders such as lupus, multisystem autoimmune diseases, and autoimmune hearing loss.

The term "lupus" as used herein refers to a chronic, inflammatory autoimmune disorder called lupus erythematosus that may affect many organ systems including the skin, joints and internal organs. Lupus is a general term which includes a number of specific types of lupus, including systemic lupus, lupus nephritis, and lupus cerebritis. In systemic lupus (SLE), the body's natural defenses are turned against the body and rogue immune cells attack the body's tissues. Antibodies may be produced that can react against the body's blood cells, organs, and tissues. This reaction leads to immune cells attacking the affected systems, producing a chronic disease. Lupus nephritis, also referred to as lupus glomerular disease, is kidney disorder that is usually a complication of SLE, and is characterized by damage to the glomerulus and progressive loss of kidney function. Lupus cerebritis refers to another complication of SLE, which is inflammation of the brain and/or central nervous system.

*6. Choroidal neovascularization*

Tumor necrosis factor has been implicated in the pathophysiology of choroidal neovascularization. For example, in surgically excised choroidal neovascular membranes, neovascular vessels stained positive for both TNF and IL-1 (Oh H et al. (1999) Invest Ophthalmol Vis Sci 40:1891). In one embodiment, the TNFα antibody of the invention is used to treat choroidal neovascularization. The term "choroidal neovascularization" as used herein refers to the growth of new blood vessels that originate from the choroid through a break in the Bruch membrane into the sub-retinal pigment epithelium (sub-RPE) or subretinal space. Choroidal neovascularization (CNV) is a major cause of visual loss in patients with the condition.

*7. Sciatica*

Tumor necrosis factor has been implicated in the pathophysiology of sciatica (Ozaktay et al. (2002) Eur Spine J. 11:467; Brisby et al. (2002) Lur Spine J. 11:62). In one embodiment, the TNFα antibody of the invention is used to treat sciatica. The term "sciatica" as used herein refers to a condition involving impaired movement and/or sensation in the leg, caused by damage to the sciatic nerve. Sciatica is also commonly referred to as neuropathy of the sciatic nerve and sciatic nerve dysfunction. Sciatica is a form of peripheral neuropathy. It occurs when there is damage to the sciatic nerve, located in the back of the leg. The sciatic nerve controls the muscles of the back of the knee and lower leg and provides sensation to the back of the thigh, part of the lower leg and the sole of the foot. Sciatica can be indicative of another disorder, including a lumbar herniated disc, spinal stenosis, degenerative disc disease, isthmic spondyloisthesis and piniformis syndrome.

*8. Sjogren's syndrome*

Tumor necrosis factor has been implicated in the pathophysiology of Sjogren's syndrome (Koski et al. (2001) Clin Exp Rheumatol. 19:131). In one embodiment, the TNFα antibody of the invention is used to treat Sjogren's syndrome. The term "Sjogren's syndrome" as used herein refers to a systemic inflammatory disorder characterized by dry mouth, decreased tearing, and other dry mucous membranes, and is often associated with autoimmune rheumatic disorders, such as rheumatoid arthritis. Dryness of the eyes and mouth are the most common symptoms of this syndrome. The symptoms may occur alone, or with symptoms associated with rheumatoid arthritis or other connective tissue diseases. There may be an associated enlargement of the salivary glands. Other organs may become affected. The syndrome may be associated with rheumatoid arthritis, systemic lupus erythematosus, scleroderma, polymyositis, and other diseases.

9. *Uveitis*

Tumor necrosis factor has been implicated in the pathophysiology of uveitis (Wakefield and Lloyd (1992) Cytokine 4:1; Woon et al. (1998) Curr Eye Res. 17:955). In one embodiment, the TNFα antibody of the invention is used to treat uveitis. The term "uveitis" as used herein refers to an inflammation of the the uvea, which is the layer between the sclera and the retina, which includes the iris, ciliary body, and the choroid. Uveitis is also commonly referred to as iritis, pars planitis, chroiditis, chorioretinitis. anterior uveitis, and posterior uveitis. The most common form of uveitis is anterior uveitis, which involves inflammation in the front part of the eye, which is usually isolated to the iris. This condition is often called iritis. In one embodiment, the term uveitis refers to an inflammation of the the uvea which excludes inflammation associated with an autoimmune disease, *i.e.,* excludes autoimmune uveitis.

10. *Wet macular degeneration*

Tumor necrosis factor has been implicated in the pathophysiology of wet macular degeneration. In one embodiment, the TNFα antibody of the invention is used to treat wet macular degeneration. The term "wet macular degeneration" as used herein refers to a disorder that affects the macula (the central part of the retina of the eye) and causes decreased visual acuity and possible loss of central vision. Patients with wet macular degeneration develop new blood vessels under the retina, which causes hemorrhage, swelling, and scar tissue.

11. *Osteoporosis*

Tumor necrosis factor has been implicated in the pathophysiology of osteoporosis, (Tsutsumimato et al. (1999) JBone Miner Res. 14:1751). Osteoporosis is used to refer to a disorder characterized by the progressive loss of bone density and thinning of bone tissue. Osteoporosis occurs when the body fails to form enough new bone, or when too much old bone is reabsorbed by the body, or both. The TNFα antibody, or antigen-binding fragment thereof, of the invention can be used to treat osteoporosis.

12. *Osteoarthritis*

Tumor necrosis factor has been implicated in the pathophysiology of osteoarthritis, (Venn et al. (1993) Arthritis Rheum. 36:819; Westacott et al. (1994) J Rheumatol. 21:1710). Osteoarthritis (OA) is also referred to as hypertrophic osteoarthritis, osteoarthrosis, and degenerative joint disease. OA is a chronic degenerative disease of skeletal joints, which affects specific joints, commonly knees, hips, hand joints and spine, in adults of all ages. OA is characterized by a number of the following manifestations including degeneration and thinning of the articular cartilage with associated development of "ulcers" or craters, osteophyte formation, hypertrophy of bone at the margins, and changes in the snyovial membrane and enlargement of affected joints. Furthermore, osteoarthritis is accompanied by pain and stiffness, particularly after prolonged activity. The antibody, or antigen-binding fragment thereof, of the invention can be used to treat osteoarthritis. Characteristic radiographic features of osteoarthritis include joint space narrowing, subchondral sclerosis, osteophytosis, subchondral cyst formation, loose osseous body (or "joint mouse").

Medications used to treat osteoarthritis include a variety of nonsteroidal, anti-inflammatory drugs (NSAIDs). In addition, COX 2 inhibitors, including Celebrex, Vioxx, and Bextra, aand Etoricoxib, are also used to treat OA. Steroids, which are injected directly into the joint, may also be used to reduce inflammation and pain. In one embodiment of the invention, TNFα antibodies of the invention are administered in combination with a NSAIDs, a COX2 inhibitor, and/or steroids.

13. *Other*

The antibodies, and antibody portions, of the invention, also can be used to treat various other disorders in which TNFα activity is detrimental. Examples of other diseases and disorders in which TNFα activity has been implicated in the pathophysiology, and thus which can be treated using an antibody, or antibody portion, of the invention, include age-related cacliexia, Alzheimer's disease, brain edema, inflammatory brain injury, cancer, cancer and cachexia, chronic fatigue syndrome, dermatomyositis, drug reactions, such as Stevens-Johnson syndrome and Jarisch-Herxheimer reaction, edema in and/or around the spinal cord, familial periodic fevers, Felty's syndrome, fibrosis, glomerulonephritides (e.g. post-streptococcal glomerulonephritis or IgA nephropathy), loosening of prostheses, microscopic polyangiitis, mixed connective tissue disorder, multiple myeloma, cancer and cachexia, multiple organ disorder, myelo dysplastic syndrome, orchitism osteolysis, pancreatitis, including acute, chronic, and pancreatic abscess, periodontal disease polynyositis, progressive renal failure, pseudogout, pyoderma gangrenosum, relapsing polychondritis, rheumatic heart disease, sarcoidosis, sclerosing cholangitis, stroke, thoracoabdominal aortic aneurysm repair (TAAA), TNF receptor associated periodic syndrome (TRAPS), symptoms related to Yellow Fever vaccination, inflammatory diseases associated with the ear, chronic ear inflammation, chronic otitis media with or without cholesteatoma, pediatric ear inflammation, myotosis, ovarian cancer, colorectal cancer, disorders associated with transplantation, therapy associated with induced inflammatory syndrome (e.g., syndromes following IL-2 administration), and a disorder associated with a reperfussion injury.

It is understood that all of the above-mentioned TNF$\alpha$-related disorders include both the adult and juvenile forms of the disease where appropriate. It is also understood that all of the above-mentioned disorders include both chronic and acute forms of the disease. In addition, the TNF$\alpha$ antibody of the invention can be used to treat each of the above-mentioned TNF$\alpha$-related disorders alone or in combination with one another, *e.g.,* a subject who is suffering from uveitis and lupus.

III. Pharmaceutical Compositions and Pharmaceutical Administration

A. *Compositions and Administration*

The antibodies, antibody-portions, and other TNF$\alpha$ inhibitors of the invention can be incorporated into pharmaceutical compositions suitable for administration to a subject. Typically, the pharmaceutical composition comprises an antibody, antibody portion, or other TNF$\alpha$ inhibitor of the invention and a pharmaceutically acceptable carrier. As used herein, "pharmaceutically acceptable carrier" includes any and all solvents, dispersion media, coatings, antibacterial and antifungal agents, isotonic and absorption delaying agents, and the like that are physiologically compatible. Examples of pharmaceutically acceptable carriers include one or more of water, saline, phosphate buffered saline, dextrose, glycerol, ethanol and the like, as well as combinations thereof. In many cases, it is preferable to include isotonic agents, for example, sugars, polyalcohols such as mannitol, sorbitol, or sodium chloride in the composition. Pharmaceutically acceptable carriers may further comprise minor amounts of auxiliary substances such as wetting or emulsifying agents, preservatives or buffers, which enhance the shelf life or effectiveness of the antibody, antibody portion, or other TNF$\alpha$ inhibitor.

The compositions of this invention may be in a variety of forms. These include, for example, liquid, semi-solid and solid dosage forms, such as liquid solutions (e.g., injectable and infusible solutions), dispersions or suspensions, tablets, pills, powders, liposomes and suppositories. The preferred form depends on the intended mode of administration and therapeutic application. Typical preferred compositions are in the form of injectable or infusible solutions, such as compositions similar to those used for passive immunization of humans with other antibodies or other TNF$\alpha$ inhibitors. The preferred mode of administration is parenteral (*e.g.,* intravenous, subcutaneous, intraperitoneal, intramuscular). In a preferred embodiment, the antibody or other TNF$\alpha$ inhibitor is administered by intravenous infusion or injection. In another preferred embodiment, the antibody or other TNF$\alpha$ inhibitor is administered by intramuscular or subcutaneous injection.

Therapeutic compositions typically must be sterile and stable under the conditions of manufacture and storage. The composition can be formulated as a solution, microemulsion, dispersion, liposome, or other ordered structure suitable to high drug concentration. Sterile injectable solutions can be prepared by incorporating the active compound (*i.e.,* antibody, antibody portion, or other TNF$\alpha$ inhibitor) in the required amount in an appropriate solvent with one or a combination of ingredients enumerated above, as required, followed by filtered sterilization. Generally, dispersions are prepared by incorporating the active compound into a sterile vehicle that contains a basic dispersion medium and the required other ingredients from those enumerated above. In the case of sterile powders for the preparation of sterile injectable solutions, the preferred methods of preparation are vacuum drying and freeze-drying that yields a powder of the active ingredient plus any additional desired ingredient from a previously sterile-filtered solution thereof. The proper fluidity of a solution can be maintained, for example, by the use of a coating such as lecithin, by the maintenance of the required particle size in the case of dispersion and by the use of surfactants. Prolonged absorption of injectable compositions can be brought about by including in the composition an agent that delays absorption, for example, monostearate salts and gelatin.

Supplementary active compounds can also be incorporated into the compositions. In certain embodiments, an antibody or antibody portion of the invention is coformulated with and/or coadministered with one or more additional therapeutic agents. For example, an anti-hTNF$\alpha$ antibody or antibody portion of the invention maybe coformulated and/or coadministered with one or more DMARD or one or more NSAID or one or more additional antibodies that bind other targets (*e.g.,* antibodies that bind other cytokines or that bind cell surface molecules), one or more cytokines, soluble TNF$\alpha$ receptor (see e.g., PCT Publication No. WO 94/06476) and/or one or more chemical agents that inhibit hTNF$\alpha$ production or activity (such as cyclobexane-ylidene derivatives as described in PCT Publication No. WO 93/19751) or any combination thereof. Furthermore, one or more antibodies of the invention may be used in combination with two or more of the foregoing therapeutic agents. Such combination therapies may advantageously utilize lower dosages of the administered therapeutic agents, thus avoiding possible side effects, complications or low level of response by the patient associated with the various monotherapies.

In one embodiment, the invention includes pharmaceutical compositions comprising an effective amount of a TNFα inhibitor and a pharmaceutically acceptable carrier, wherein the effective amount of the TNFα inhibitor may be effective to treat a TNFα-related disorder, including, for example, sciatica, endometriosis, and prostatitis.

The antibodies, antibody-portions, and other TNFα inhibitors of the present invention can be administered by a variety of methods known in the art, although for many therapeutic applications, the preferred route/mode of administration is intravenous injection or infusion. As will be appreciated by the skilled artisan, the route and/or mode of administration will vary depending upon the desired results. In certain embodiments, the active compound may be prepared with a carrier that will protect the compound against rapid release, such as a controlled release formulation, including implants, transdermal patches, and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art. See, *e.g.,* Sustained and Controlled Release Drug Delivery Systems, J.R. Robinson, ed., Marcel Dekker, Inc., New York, 1978.

The TNFα antibodies of the invention can also be administered in the form of protein crystal formulations which include a combination of protein crystals encapsulated within a polymeric carrier to form coated particles. The coated particles of the protein crystal formulation may have a spherical morphology and be microspheres of up to 500 micro meters in diameter or they may have some other morphology and be microparticulates. The enhanced concentration of protein crystals allows the antibody of the invention to be delivered subcutaneously. In one embodiment, the TNFa antibodies of the invention are delivered via a protein delivery system, wherein one or more of a protein crystal formulation or composition, is administered to a subject with a TNFα-related disorder. Compositions and methods of preparing stabilized formulations of whole antibody crystals or antibody fragment crystals are also described in WO 02/072636, which is incorporated by reference herein. In one embodiment, a formulation comprising the crystallized antibody fragments described in Examples 37 and 38 are used to treat a TNFa-related disorder.

In certain embodiments, an antibody, antibody portion, or other TNFα inhibitor of the invention may be orally administered, for example, with an inert diluent or an assimilable edible carrier. The compound (and other ingredients, if desired) may also be enclosed in a hard or soft shell gelatin capsule, compressed into tablets, or incorporated directly into the subject's diet For oral therapeutic administration, the compounds may be incorporated with excipients and used in the form of ingestible tablets, buccal tablets, troches, capsules, elixirs, suspensions, syrups, wafers, and the like. To administer a compound of the invention by other than parenteral administration, it may be necessary to coat the compound with, or co-administer the compound with, a material to prevent its inactivation.

The pharmaceutical compositions of the invention may include a "therapeutically effective amount" or a "prophylactically effective amount" of an antibody or antibody portion of the invention. A "therapeutically effective amount" refers to an amount effective, at dosages and for periods of time necessary, to achieve the desired therapeutic result A therapeutically effective amount of the antibody, antibody portion, or other TNFα inhibitor may vary according to factors such as the disease state, age, sex, and weight of the individual, and the ability of the antibody, antibody portion, other TNFα inhibitor to elicit a desired response in the individual. A therapeutically effective amount is also one in which any toxic or detrimental effects of the antibody, antibody portion, or other TNFα inhibitor are outweighed by the therapeutically beneficial effects. A "prophylactically effective amount" refers to an amount effective, at dosages and for periods of time necessary, to achieve the desired prophylactic result. Typically, since a prophylactic dose is used in subjects prior to or at an earlier stage of disease, the prophylactically effective amount will be less than the therapeutically effective amount.

Dosage regimens may be adjusted to provide the optimum desired response (*e.g.*, a therapeutic or prophylactic response). For example, a single bolus may be administered, several divided doses may be administered over time or the dose may be proportionally reduced or increased as indicated by the exigencies of the therapeutic situation. It is especially advantageous to formulate parenteral compositions in dosage unit form for ease of administration and uniformity of dosage. Dosage unit form as used herein refers to physically discrete units suited as unitary dosages for the mammalian subjects to be treated; each unit containing a predetermined quantity of active compound calculated to produce the desired therapeutic effect in association with the required pharmaceutical carrier. The specification for the dosage unit forms of the invention are dictated by and directly dependent on (a) the unique characteristics of the active compound and the particular therapeutic or prophylactic effect to be achieved, and (b) the limitations inherent in the art of compounding such an active compound for the treatment of sensitivity in individuals.

An exemplary, non-limiting range for a therapeutically or prophylactically effective amount of an antibody or antibody portion of the invention is 10-150 mg, more preferably 20-80 mg and most preferably about 40 mg. It is to be noted that dosage values may vary with the type and severity of the condition to be alleviated. It is to be further understood that for any particular subject, specific dosage regimens should be adjusted over time according to the individual need and the professional judgment of the person administering or supervising the administration of the compositions, and that dosage ranges set forth herein are exemplary only and are not intended to limit the scope or practice of the claimed composition. Ranges intermediate to the above recited concentrations, *e.g.,* about 6-144 mg/ml, are also intended to be part of this invention. For example, ranges of values using a combination of any of the above recited values as upper and/or lower limits are intended to be included.

The invention also pertains to packaged pharmaceutical compositions which comprise a TNFα inhibitor of the invention and instructions for using the inhibitor to treat TNFα-related disorders, as described above.

Another aspect of the invention pertains to kits containing a pharmaceutical composition comprising an anti-TNFα antibody and a pharmaceutically acceptable carrier and one or more pharmaceutical compositions each comprising a drug useful for treating a TNFα-related disorder and a pharmaceutically acceptable carrier. Alternatively, the kit comprises a single pharmaceutical composition comprising an anti-TNFα antibody, one or more drugs useful for treating a TNFα-related disorder and a pharmaceutically acceptable carrier. The kits contain instructions for dosing of the pharmaceutical compositions for the treatment of a TNFα-related disorder in which the administration of an anti-TNFα antibody is beneficial, such as lupus.

The invention also pertains to packaged pharmaceutical compositions or kits which comprise a TNFα inhibitor of the invention and instructions for using the inhibitor to treat a particular disorder in which TNFα activity is detrimental, as described above. The package or kit alternatively can contain the TNFα inhibitor and it can be promoted for use, either within the package or through accompanying information, for the uses or treatment of the disorders described herein. The packaged pharmaceuticals or kits further can include a second agent (as described herein) packaged with or copromoted with instructions for using the second agent with a first agent (as described herein).

B. *Additional therapeutic agents*

The invention pertains to pharmaceutical compositions and methods of use thereof for the treatment of a TNFα-related disorder. The pharmaceutical compositions comprise a first agent that prevents or inhibits a TNFα-related disorder. The pharmaceutical composition also may comprise a second agent that is an active pharmaceutical ingredient; that is, the second agent is therapeutic and its function is beyond that of an inactive ingredient, such as a pharmaceutical carrier, preservative, diluent, or buffer. The second agent may be useful in treating or preventing TNFα-related disorders. The second agent may diminish or treat at least one symptom(s) associated with the targeted disease. The first and second agents may exert their biological effects by similar or unrelated mechanisms of action; or either one or both of the first and second agents may exert their biological effects by a multiplicity of mechanisms of action. A pharmaceutical composition may also comprise a third compound, or even more yet, wherein the third (and fourth, etc.) compound has the same characteristics of a second agent.

It should be understood that the pharmaceutical compositions described herein may have the first and second, third, or additional agents in the same pharmaceutically acceptable carrier or in a different pharmaceutically acceptable carrier for each described embodiment. It further should be understood that the first, second, third and additional agent may be administered simultaneously or sequentially within described embodiments. Alternatively, a first and second agent may be administered simultaneously, and a third or additional agent may be administered before or after the first two agents.

The combination of agents used within the methods and pharmaceutical compositions described herein may have a therapeutic additive or synergistic effect on the condition(s) or disease(s) targeted for treatment. The combination of agents used within the methods or pharmaceutical compositions described herein also may reduce a detrimental effect associated with at least one of the agents when administered alone or without the other agent(s) of the particular pharmaceutical composition. For example, the toxicity of side effects of one agent may be attenuated by another agent of the composition, thus allowing a higher dosage, improving patient compliance, and improving therapeutic outcome. The additive or synergistic effects, benefits, and advantages of.the compositions apply to classes of therapeutic agents, either structural or functional classes, or to individual compounds themselves.

Supplementary active compounds can also be incorporated into the compositions. In certain embodiments, an antibody or antibody portion of the invention is coformulated with and/or coadministered with one or more additional therapeutic agents that are useful for treating TNFα-related disorder in which TNFα activity is detrimental. For example, an anti-hTNFα antibody, antibody portion, or other TNFα inhibitor of the invention may be coformulated and/or coadministered with one or more additional antibodies that bind other targets (*e.g.,* antibodies that bind other cytokines or that bind cell surface molecules), one or more cytokines, soluble TNFα receptor (see *e.g.,* PCT Publication No. WO 94/06476) and/or one or more chemical agents that inhibit hTNFα production or activity (such as cyclohexane-ylidene derivatives as described in PCT Publication No. WO 93/19751). Furthermore, one or more antibodies or other TNFα inhibitors of the invention may be used in combination with two or more of the foregoing therapeutic agents. Such combination therapies may advantageously utilize lower dosages of the administered therapeutic agents, thus avoiding possible toxicities or complications associated with the various monotherapies. Specific therapeutic agent(s) are generally selected based on the particular TNFα-related disorder being treated, as discussed below.

Nonlimiting examples of therapeutic agents with which an antibody, antibody portion, or other TNFα inhibitor of the invention can be combined include the following: non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2/infliximab (chimeric anti-TNFα antibody; Centocor); 75 kdTNFR-IgG/etanercept (75 kD TNF receptor-IgG fusion protein; Immunex; see *e.g.,* Arthritis & Rheumatism (1994) Vol. 37, S295; J. Invest. Med. (1996) Vol. 44, 235A); 55 kdTNF-IgG (55 kD TNF receptor-IgG fusion protein; Hoffmann-LaRoche); IDEC-CE9.1/SB 210396 (non-depleting primatized anti-CD4 antibody; IDEC/SmithKline; see *e.g.,* Arthritis & Rheumatism (1995) Vol. 38, S185); DAB 486-IL-2 and/or DAB 389-IL-2 (IL-2 fusion proteins; Seragen; see *e.g.,* Arthritis & Rheumatism (1993) Vol. 36, 1223); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (*e.g.,* agonist antibodies); IL-IRA (IL-1 receptor antagonist; Synergen/Amgen); TNF-bp/s-TNF (soluble TNF binding protein; see *e.g.,* Anthritis & Rheumatism (1996) Vol. 39, No. 9 (supplement), S284; Amer. J. Physiol. - Heart and Circulatory Physiology (1995) Vol. 268, pp. 37-42); R973401 (phosphodiesterase Type IV inhibitor; see *e.g.,* Arthritis & Rheumatism (1996) Vol. 39, No. 9 (supplement), S282); MK-966 (COX-2 Inhibitor; see *eg.,* Arthritis & Rheumatism (1996) Vol. 39, No. 9 (supplement), S81); Iloprost (see *e.g.,* Arthritis & Rheumatism (1996) Vol. 39, No. 9 (supplement), S82); methotrexate; thalidomide (see *e.g.,* Arthritis & Rheumatism (1996) Vol. 39, No. 9 (supplement), S282) and thalidomide-related drugs (*e.g.*, Celgen); leflunomide (anti-inflammatory and cytokine inhibitor; see *e.g.,* Arthritis & Rheumatism (1996) Vol. 39, No. 9 (supplement), S131; Inflammation Research (1996) Vol. 45, pp. 103-107); tranexamic acid (inhibitor of plasminogen activation; see *e.g.,* Arthritis & Rheumatism (1996) Vol. 39, No. 9 (supplentent), S284); T-614 (cytokine inhibitor; see *e.g.,* Arthritis & Rheumatism (1996) Vol. 39, No. 9 (supplement), S282); prostaglandin E1 (see *e.g.,* Arthritis & Rheumatism (1996) Vol. 39, No. 9 (supplement), S282); Tenidap (non-steroidal anti-inflammatory drug; see *e.g.,* Arthritis & Rheumatism (1996) Vol. 39, No. 9 (supplement), S280); Naproxen (non-steroidal anti-inflammatory drug; see *e.g.,* Neuro Report (1996) Vol. 7, pp. 1209-1213); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine (see *e.g.,* Arthritis & Rheumatism (1996) Vol. 39, No. 9 (supplement), S281); Azathioprine (see *e.g.,* Arthritis & Rheumatism (1996) Vol. 39, No. 9 (supplement), S281); ICE inhibitor (inhibitor of the enzyme interleukin-1β converting enzyme); zap-70 and/or lck inhibitor (inhibitor of the tyrosine kinase zap-70 or lck); VEGF inhibitor and/or VEGF-R inhibitor (inhibitos of vascular endothelial cell growth factor or vascular endothelial cell growth factor receptor; inhibitors of angiogenesis); corticosteroid anti-inflammatory drugs (*e.g.,* SB203580); TNF-convertase inhibitors; anti-IL-12 antibodies; anti-IL-18 antibodies; interleukin-11 (see *e.g.,* Arthritis & Rheumatism (1996) Vol. 39, No. 9 (supplement), S296); interleukin-13 (see *e.g.,* Arthritis & Rheumatism (1996) Vol. 39, No. 9 (supplement), S308); interleukin-17 inhibitors (see *e.g.,* Arthritis & Rheumatism (1996) Vol. 39, No. 9 (supplement), S120); gold; penicillamine; chloroquine; hydroxychloroquine; chlorambucil; cyclophosphamide; cyclosporine; total lymphoid irradiation; anti-thymocyte globulin; anti-CD4 antibodies; CD5-toxins; orally-administered peptides and collagen; lobenzarit disodium; Cytokine Regulating Agents (CRAs) HP228 and HP466 (Houghten Pharmaceuticals, Inc.); ICAM-1 antisense phosphorothioate oligodeoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids; see *e.g.*, DeLuca et al. (1995) Rheum. Dis. Clin. North Am. 21:759-777); auranofin; phenylbutazone; meclofenamic acid; flufenamic acid; intravenous immune globulin; zileuton; mycophenolic acid (RS-61443); tacrolimus (FK-506); sirolimus (rapamycin); amiprilose (therafectin); cladribine (2-chlorodeoxyadenosine); azaribine; methotrexate; antivirals; and immune modulating agents. Any of the above-mentioned agents can be administered in combination with the TNFα antibody of the invention to treat an TNFα-related disorder.

In one embodiment, the TNFα antibody of the invention is administered in combination with one of the following agents for the treatment of rheumatoid arthritis: small molecule inhibitor of KDR (ABT-123), small molecule inhibitor of Tie-2; methotrexate; prednisone; celecoxib; folic acid; hydroxychloroquine sulfate; rofecoxib; etanercept; infliximab; leflunomide; naproxen; valdecoxib; sulfasalazine; methylprednisolone; ibuprofen; meloxicam; methylprednisolone acetate; gold sodium thiomalate; aspirin; azathioprine; triamcinolone acetonide; propxyphene napsylate/apap; folate; nabumetone; diclofenac; piroxicam; etodolac; diclofenac sodium; oxaprozin; oxycodone hcl; hydrocodone bitartrate/apap; diclofenac sodium/misoprostol; fentanyl; anakinra, human recombinant; tramadol hcl; salsalate; sulindac; cyanocobalamin/fa/pyridoxine; acetaminophen; alendronate sodium; prednisolone; morphine sulfate; lidocaine hydrochloride; indomethacin; glucosamine sulfate/chondroitin; cyclosporine; amitriptyline hcl; sulfadiazine; oxycodone hcl/acetaminophen; olopatadine hcl; misoprostol; naproxen sodium; omeprazole; mycophenolate mofetil; cyclophosphamide; rituximab; IL-1 TRAP; MRA; CTLA4-IG; IL-18 BP; ABT-874; ABT-325 (anti-IL 18); anti-IL 15; BIRB-796; SCIO-469; VX-702; AMG-548; VX-740; Roflumilast; IC-485; CDC-801; and mesopram. In another embodiment, the TNFα antibody of the invention is administered for the treatment of a TNFα related disorder in combination with one of the above mentioned agents for the treatment of rheumatoid arthritis.

In one embodiment, the TNFα antibody of the invention is administered in combination with one of the following agents for the treatment of a TNFα-related disorder in which TNFα activity is detrimental: anti-IL12 antibody (ABT 874); anti-IL18 antibody (ABT 325); small molecule inhibitor of LCK; small molecule inhibitor of COT; anti-IL1 antibody; small molecule inhibitor of MK2; anti-CD19 antibody; small molecule inhibitor of CXCR3; small molecule inhibitor of CCR5;

small molecule inhibitor of CCR1l anti-E/L selectin antibody; small molecule inhibitor of P2X7; small molecule inhibitor of IRAK-4; small molecule agonist of glucocorticoid receptor; antiC5a receptor antibody; small molecule inhibitor of C5a receptor; anti-CD32 antibody; and CD32 as a therapeutic protein.

In yet another embodiment, the TNFα antibody of the invention is administered in combination with an antibiotic or antiinfective agent. Antiinfective agents include those agents known in the art to treat viral, fungal, parasitic or bacterial infections. The term, "antibiotic," as used herein, refers to a chemical substance that inhibits the growth of, or kills, microorganisms. Encompassed by this term are antibiotic produced by a microorganism, as well as synthetic antibiotics (e.g., analogs) known in the art. Antibiotics include, but are not limited to, clarithromycin (Biaxin®), ciprofloxacin (Cipro®), and metronidazole (Flagyl®).

In another embodiment, the TNFα antibody of the invention is administered in combination with an additional therapeutic agent to treat sciatica or pain. Examples of agents which can be used to reduce or inhibit the symptoms of sciatica or pain include hydrocodone bitartrate/apap, rofecoxib, cyclobenzaprine hcl, methylprednisolone, naproxen, ibuprofen, oxycodone hcl/acetaminophen, celecoxib, valdecoxib, methylprednisolone acetate, prednisone, codeine phosphate/apap, tramadol hcl/acetaminophen, metaxalone, meloxicam, methocarbamol, lidocaine hydrochloride, diclofenac sodium, gabapentin, dexamethasone, carisoprodol, ketorolac tromethamine, indomethacin, acetaminophen, diazepam, nabumetone, oxycodone hcl, tizanidine hcl, diclofenac sodium/misoprostol, propoxyphene napsylate/apap, asa/oxycod/oxycodone ter, ibuprofen/hydrocodone bit, tramadol hcl, etodolac, propoxyphene hcl, amitriptyline hcl, carisoprodol/codeine phos/asa, morphine sulfate, multivitamins, naproxen sodium, orphenadrine citrate, and temazepam.

In yet another embodiment, the TNFα-related disorder is treated with the TNFα antibody of the invention in combination with hemodialysis.

In another embodiment, the TNFα antibody of the invention is used in combination with a drug used to treat Crohn's disease or a Crohn's-related disorder. Examples of therapeutic agents which can be used to treat Crohn's include mesalamine, prednisone, azathioprine, mercaptopurine, infliximab, budesonide, sulfasalazine, methylprednisolone sod succ, diphenoxylate/atrop sulf, loperamide hydrochloride, methotrexate, omeprazole, folate, ciprofloxacin/dextrose-water, hydrocodone bitartrate/apap, tetracycline hydrochloride, fluocinonide, metronidazole, thimerosal/boric acid, cholestyramine/sucrose, ciprofloxacin hydrochloride, hyoscyamine sulfate, meperidine hydrochloride, midazolam hydrochloride, oxycodone hcl/acetaminophen, promethazine hydrochloride, sodium phosphate, sulfamethoxazoletrimethoprim, celecoxib, polycarbophil, propoxyphene napsylate, hydrocortisone, multivitamins, balsalazide disodium, codeine phosphate/apap, colesevelam hcl, cyanocobalamin, folic acid, levofloxacin, methylprednisolone, natalizumab, and interferon-gamma

In another embodiment, the TNFα antibody of the invention is administered in combination with an additional therapeutic agent to treat asthma. Examples of agents which can be used to reduce or inhibit the symptoms of asthma include the following: albuterol; salmeterol/fluticasone; sodium; fluticasone propionate; budesonide; prednisone; salmeterol xinafoate; levalbuterol hcl; sulfate/ipratropium; prednisolone sodium phosphate; triamcinolone acetonide; beclomethasone clipropionate; ipratropium bromide; Azithromycin; pirbuterol acetate, prednisolone, theophylline anhydrous, methylprednisolone sod succ, clarithromycin, zafirlukast, formoterol fumarate, influenza virus vaccine, methylprednisolone, trihydrate, flunisolide, allergy injection, cromolyn sodium, fexofenadine hydrochloride, flunisolide/menthol, amoxicillin/clavulanate, levofloxacin, inhaler assist device, guaifenesin, dexamethasone sod phosphate; moxifloxacin hcl; hyclate; guaifenesin/d-methorphan; pephedrine/cod/chlorphenir; gatifloxacin; cetirizine hydrochloride; mometasone furoate; salmeterol xinafoate; benzonatate; cephalexin; pe/hydrocodone/chlorphenir; cetirizine hcl/pseudoephed; phenylephrine/cod/promethazine; codeine/promethazine; cefprozil; dexamethasone; guaifenesin/pseudoephedrine, chlorpheniramine/hydrocodone, nedocromil sodium, terbutaline sulfate, epinephrine and methylprednisolone, metaproterenol sulfate.

In another embodiment, the TNFα antibody of the invention is administered in combination with an additional therapeutic agent to treat COPD. Examples of agents which can be used to reduce or inhibit the symptoms of COPD include, albuterol sulfate/ipratropium; ipratropium bromide; salmeterol/fluticasone; albuterol; salmeterol; xinafoate; fluticasone propionate; prednisone; theophylline anhydrous; methylprednisolone sod succ; montelukast sodium; budesonide; formoterol fumarate; triamcinolone acetonide; levofloxacin; guaifenesin; azithromycin; beclomethasone; dipropionate; levalbuterol hcl; flunisolide; sodium; trihydrate; gatifloxacin; zafirlukast; amoxicillin/clavulanate; flunisolide/menthol; chlorpheniramine/hydrocodone; metaproterenol sulfate; mathylprednisolone; furoate; -ephedrine/cod/chlorphenir; pirbuterol acetate; -ephedrine/loratadine; terbutaline sulfate; tiotropium bromide; (R,R)-formoterol; TgAAT; Cilomilast and Roflumilast

In another embodiment, the TNFα antibody of the invention is administered in combination with an additional therapeutic agent to treat IPF. Examples of agents which can be used to reduce or inhibit the symptoms of IPF include prednisone; azathioprine; albuterol; colchicines; sulfate; digoxin; gamma interferon; methylprednisolone sod succ; furosemide; lisinopril; nitroglycerin; spironolactone; cyclophosphamide; ipratropium bromide; actinomycin d; alteplase; fluticasone propionate; levofloxacin; metaproterenol sulfate; morphine sulfate; oxycodone hcl; potassium chloride; triamcinolone acetonide; tacrolimus anhydrous; calcium; interferon-alpha; methotrexate; mycophenolate mofetil.

In one embodiment of the invention, a TNFα antibody is administered in combination with an agent which is commonly used to treat spondyloarthropathies. Examples of such agents include nonsteroidal, anti-inflammatory drugs (NSAIDs), COX 2 inhibitors, including Celebrex®, Vioxx®, and Bextra®, aand etoricoxib. Physiotherapy is also commonly used to treat spondyloarthropathies, usually in conjunction with non-steoidal inflammatory drugs.

In another embodiment, the TNFα antibody of the invention is administered in combination with an additional therapeutic agent to treat ankylosing spondylitis. Examples of agents which can be used to reduce or inhibit the symptoms of ankylosing spondylitis include ibuprofen, diclofenac and misoprostol, naproxen, meloxicam, indomethacin, diclofenac, celecoxib, rofecoxib, sulfasalazine, prednisone, methotrexate, azathioprine, minocyclin, prednisone, etanercept, and infliximab.

In another embodiment, the TNFα antibody of the invention is administered in combination with an additional therapeutic agent to treat psoriatic arthritis. Examples of agents which can be used to reduce or inhibit the symptoms of psoriatic arthritis include methotrexate; etanercept; rofecoxib; celecoxib; folic acid; sulfasalazine; naproxen; leflunomide; methylprednisolone acetate; indomethacin; hydroxychloroquine sulfate; sulindac; prednisone; betamethasone diprop augmented; infliximab; methotrexate; folate; triamcinolone acetonide; diclofenac; dimethylsulfoxide; piroxicam; diclofenac sodium; ketoprofen; meloxicam; prednisone; methylprednisolone; nabumetone; tolmetin sodium; calcipotriene; cyclosporine; diclofenac; sodium/misoprostol; fluocinonide; glucosamine sulfate; gold sodium thiomalate; hydrocodone; bitartrate/apap; ibuprofen; risedronate sodium; sulfadiazine; thioguanine; valdecoxib; alefacept; and efalizumab.

In one embodiment the TNFα inhibitor is administered following an initial procedure for treating coronary heart disease. Examples of such procedures include, but are not limited to coronary artery bypass grafting (CABG) and Percutaneous transluminal coronary balloon angioplasty (PTCA) or angioplasty. In one embodiment, the TNFα inhibitor is administered in order to prevent stenosis from re-occurring. In another embodiment of the invention, the TNFα inhibitor is administered in order to prevent or treat restenosis. The invention also provides a method of treatment, wherein the TNFα inhibitor is administered prior to, in conjunction with, or following the insertion of a stent in the artery of a subject receiving a procedure for treating coronary heart disease. In one embodiment the stent is administered following CABG or PTCA. A wide variety of stent grafts may be utilized within the context of the present invention, depending on the site and nature of treatment desired. Stent grafts may be, for example, bifurcated or tube grafts, cylindrical or tapered, self-expandable or balloon-expandable, unibody, or, modular. Moreover, the stent graft may be adapted to release the drug at only the distal ends, or along the entire body of the stent graft. The TNFα inhibitor of the invention can also be administered on a stent. In one embodiment, the TNFα antibody of the invention, including, for example, D2E7/HUMIRA® is administered by a drug-eluting stent.

The TNFα antibody of the invention can be administered in combination with an additional therapeutic agent to treat restenosis. Examples of agents which can be used to treat or prevent restenosis include sirolimus, paclitaxel, everolimus, tacrolimus, ABT-578, and acetaminophen.

The TNFα antibody of the invention can be administered in combination with an additional therapeutic agent to treat myocardial infarction. Examples of agents which can be used to treat or prevent myocardial infarction include aspirin, nitroglycerin, metoprolol tartrate, enoxaparin sodium, heparin sodium, clopidogrel bisulfate, carvedilol, atenolol, morphine sulfate, metoprolol succinate, warfarin sodium, lisinopril, isosorbide mononitrate, digoxin, furosemide, simvastatin, ramipril, tenecteplase, enalapril maleate, torsemide, retavase, losartan potassium, quinapril hcl/mag carb, bumetanide, alteplase, enalaprilat, amiodarone hydrochloride, tirofiban hcl m-hydrate, diltiazem hydrochloride, captopril, irbesartan, valsartan, propranolol hydrochloride, fosinopril sodium, lidocaine hydrochloride, eptifibatide, cefazolin sodium, atropine sulfate, aminocaproic acid, spironolactone, interferon, sotalol hydrochloride, potassium chloride, docusate sodium, dobutamine hcl, alprazolam, pravastatin sodium, atorvastatin calcium, midazolam hydrochloride, meperidine hydrochloride, isosorbide dinitrate, epinephrine, dopamine hydrochloride, bivalirudin, rosuvastatin, ezetimibe/simvastatin, avasimibe, abciximab, and cariporide.

The TNFα antibody of the invention can be administered in combination with an additional therapeutic agent to treat angina. Examples of agents which can be used to treat or prevent angina include: aspirin; nitroglycerin; isosorbide mononitrate; metoprolol succinate; atenolol; metoprolol tartrate; amlodipine besylate, dilitiazem hydropchloride, isosorbide dinitrate; clopidogrel bisulfate; nifedipine; atorvastatin calcium; potassium chloride; furosemide; simvastatin; verapamil hcl; digoxin; propranolol hcl; carvedilo; lisinopril; sprionolactone; hydrochlorothiazide; enalapril maleate; madolol; ramipril; enoxaparin sodium; heparin sodium; valsartan; sotalol hydrochloride; fenofibrate; ezetimibe; bumetanide; losartan potassium; lisinopril/hydrochlorothiazide; felodipine; captopril; and bisoprolol fumarate.

In one embodiment of the invention, a TNFα antibody is administered in combination with an agent which is commonly used to treat hepatitis C virus. Examples of such agents include Interferon-aplha-2a, Interferon-alpha-2b, Interferon-alpha con1, Interfero-aopha-n1, Pegylated interferon-alpha-2a, Pegylated interferon-alpha-2b, Ribavirin, Peginterferon alfa-2b and ribavirin, Ursodeoxycholic Acid, Glycyrrhizic Acid, Thymalfasin, Maxamine, and VX-497.

The TNFα antibody of the invention is administered in combination with topical corticosteroids, vitamin D analogs, and topical or oral retinoids, or combinations thereof, for the treatment of psoriasis. In addition, the TNFα antibody of the invention is administered in combination with one of the following agents for the treatment of psoriasis: small molecule inhibitor of KDR (ABT-123), small molecule inhibitor of Tie-2, calcipotriene, clobetasol propionate, triamcinolone acetonide, halobetasol propionate, tazarotene, methotrexate, fluocinonide, betamethasone diprop augmented, fluocinolone, acetonide, acitretin, tar shampoo, betamethasone valerate, mometasone furoate, ketoconazole, pramoxine/fluocinolone, hydrocortisone valerate, flurandrenolide, urea, betamethasone, clobetasol propionate/emoll, fluticasone propionate, azitbromycin, hydrocortisone, moisturizing formula, folic acid, desonide, coal tar, diflorasone diacetate, etanercept, folate, lactic acid, methoxsalen, hc/bismuth subgal/znox/resor, methylprednisolone acetate, prednisone, sunscreen, salicylic acid, halcinonide, anthralin, clocortolone pivalate, coal extract, coal tar/salicylic acid, coal tar/salicylic acid/sulfur, desoximetasone, diazepam, emollient, pimecrolimus emollient, fluocinonide/emollient, mineral oil/castor oil/na lact, mineral oil/peanut oil, petroleum/isopropyl myristate, psoralen, salicylic acid, soap/tribromsalan, thimerosal/boric acid, celecoxib, infliximab, alefacept, efalizumab, tacrolimus, pimecrolimus, PUVA, UVB, and sulfasalazine.

An antibody, antibody portion, or other TNFα inhibitor of the invention can be used in combination with other agents to treat skin conditions. For example, an antibody, antibody portion, or other TNFα inhibitor of the invention is combined with PUVA therapy. PUVA is a combination of psoralen (P) and long-wave ultraviolet radiation (UVA) that is used to treat many different skin conditions. The antibodies, antibody portions, or other TNFα inhibitors of the invention can also be combined with pimecrolimus. In another embodiment, the antibodies of the invention are used to treat psoriasis, wherein the antibodies are administered in combination with tacrolimus. In a further embodiment, tacrolimus and TNFα inhibitors are administered in combination with methotrexate and/or cyclosporine. In still another embodiment, the TNFα inhibitor of the invention is administered with excimer laser treatment for treating psoriasis.

Nonlimiting examples of other therapeutic agents with which a TNFα inhibitor can be combined to treat a skin or nail disorder include UVA and UVB phototherapy. Other nonlimiting examples which can be used in combination with a TNFα inhibitor include anti-IL-12 and anti-IL-18 therapeutic agents, including antibodies.

In one embodiment, the TNFα antibody of the invention is administered in combination with an additional therapeutic agent in the treatment of Behcet's disease. Additional therapeutic agents which can be used to treat Behcet's disease include, but are not limited to, prednisone, cyclophosphamide (Cytoxan), Azathioprine (also called imuran, methotrexate, timethoprim/sulfamethoxazole (also called bactrim or septra) and folic acid.

Any one of the above-mentioned therapeutic agents, alone or in combination therewith, can be administered to a subject suffering from a TNFα-related disorder in which TNFα is detrimental, in combination with the TNFα antibody of the invention. In one embodiment, any one of the above-mentioned therapeutic agents, alone or in combination therewith, can be administered to a subject suffering from rheumatoid arthritis in addition to a TNFα antibody to treat a TNFα-related disorder.

This invention is further illustrated by the following examples which should not be construed as limiting. The contents of all references, patents and published patent applications cited throughout this application are incorporated herein by reference

## EXAMPLES

### Example 1: TNFα Inhibitor in Rat Model for Ankylosing Spondylitis

*Administration of TNF antibody to human leukocyte antigen-B27(HLA-B27) rats to test inhibition of progressive ankylosis*

Fisher 344 rats genetically engineered to carry high-copy numbers of the human major histocompatibility complex class 1 allele B27 and the $\beta_2$-microglobulin genes exhibit symptoms similar to human spondyloarthopathies particularly ankylosing spondylitis (AS) (Zhang et al. Curr Rheumatol Rep. 2002: 4:507). Male transgenic human leuokocyte antigen-B27 (HLA-B27) rats are obtained at 10 weeks of age and are housed in an animal facility until they are 40 weeks of age. A group of Fisher 344 rats are obtained and serve as nontransgenic controls. The control rats are purchased at 36 weeks and are housed in the animal facility under the same conditions for an additional 3 to 4 weeks.

Prior to the experimental treatment, body weights are measured for both the HLA-B27 transgenic rats, and the control rats to make sure there is no significant difference between the two. The rats are then administered intraperitoneally (i.p.) doses of either a placebo or a monoclonal anti-TNFα antibody that is known to bind and neutralize rat TNFα, *e.g.*, antibody TN3 (TN3-19.12) (see Marzi et al. (1995) Shock 3:27; Williams et al. (1992) Proc Natl Acad Sci USA. 89:9784; BD Biosciences Pharmingen). Rats are evaluated for symptoms of AS using the following tests beginning at roughly 36 weeks of age and continuing throughout the study: weight, forepaw grasp of a wire grid, ability to cling to an inverted wire grid, gait, thorax flexibility, spinal mobility, and appearance of eyes, skin, nails, genitals, and peripheral and axial skeletal joints with respect to redness and swelling, joint deformity, and mobility. Rats are also examined for evidence of arthritis, particularly decreases in AS symptoms in the treated rats, and are closely observed for growth characteristics and changes in skin and nails. At 4, 6, 8, 10, 12, 16, and 20 weeks, rats are sacrificed for radiographic and microscopic analysis.

**Example 2: TNF Inhibitor Effects on AS Symptoms**

*Ankylosing Spondylitis- Clinical Considerations*

Patients who exhibit symptoms commonly associated with AS are examined and tested to determine if they suffer from AS, and thus qualify for the study. Symptoms commonly associated with AS are low back pain that is worse after inactivity, stiffness and limited motion in the low back, hip pain and stiffness, limited expansion of the chest, limited range of motion (especially involving spine and hips), joint pain and joint swelling in the shoulders, knees, and ankles, neck pain, heel pain, chronic stooping to relieve symptoms, fatigue, fever, low grade, loss of appetite, weight loss, and/or eye inflammation. Patients are given a physical examination to determine whether or not they exhibit any of the characteristic symptoms indicative of limited spine motion or chest expansion associated with AS. Examples of tests which indicate AS include X-rays of sacroiliac joints and vertebrae a which show characteristic findings associated with AS.

Ankylosing spondylitis is diagnosed using the modified New York criteria (Moll et al. (1973) Ann Rheum Dis 32:354; Van der Linden et al. (1984) Arthritis Rheum 27:361). The New York criteria for ankylosing spondylitis is a modification of the Rome criteria as proposed at the CIOMS Symposium in New York during 1966. It combines both clinical criteria and radiographic findings of the sacroiliac joint. Clinical criteria of New York criteria:

(a) Limitation of motion of the lumbar spine in all 3 planes (anterior flexion lateral flexion extension). Skin markings to aid in the examination are shown in Moll, *supra*;
(b) A history of pain or the presence of pain at the dorsolumbar junction or in the lumbar spine; and
(c) Limitation of chest expansion to 1 inch (2.5 cm) or less measured at the level of the fourth intercostal space.

Scoring Index for New York Criteria

| Radiographic Changes in the Sacroiliac Joint(s) | Grade |
| --- | --- |
| normal | 0 |
| suspicious | 1 |
| minimal sacroiliitis | 2 |
| moderate sacroiliitis | 3 |
| ankylosis | 4 |

The clinical course of AS is measured by using any number of instruments to evaluate various AS symptoms. Some of the commonly used scales include the Assessment in Ankylosing Spondylitis (ASAS), the Bath Ankylosing Spondylitis Disease Activity Index (BASDAI) (Garrett et al. (1994) J Rheumatol 21:2286), the Bath Ankylosing Spondylitis Metrology Index (BASMI) (Jenkinson et al. (1994) J Rheumatol 21:1694), and the Bath Ankylosing Spondylitis Functional Index (BASFI) (Calin et al. (1994) J Rheumatol 21:2281). These indices can be used to monitor a patient over time and to determine improvement. Each of these scales is described further below:

Criteria for Measuring the Clinical Course of AS

1. The Assessment in Ankylosing Spondylitis (ASAS20) is the primary endpoint in the pivotal Phase 3 AS studies. A≥20% improvement and absolute improvement of ≥10 units (scale of 0-100) in ≥3 of 4 domains: Subject Global Assessment, Pain, Function, and Inflammation. There must be an absence of deterioration in the potential remaining domain (deterioration is defined as a change for the worse of ≥20% and a net worsening of ≥10 units (scale of 0-100).

2. The Bath Ankylosing Spondylitis Disease Activity Index (BASDAI) can be used to evaluate the level of disease activity in a patient with AS. BASDAI focuses upon signs and symptoms of the inflammatory aspects of AS, nocturnal and total back pain, the patient's global assessment and actual physical measurements of spinal mobility such as the Schober's test, chest expansion score and occiput to wall measurement. BASDAI measures disease activity on the basis of six questions relating to fatigue, spinal pain, peripheral arthritis, enthesitis (inflammation at the points where tendons/ligaments/joint capsule enter the bone), and morning stiffness. These questions are answered on a 10-cm horizontal visual analog scale measuring severity of fatigue, spinal and peripheral joint pain, localized tenderness, and morning stiffness (both qualitative and quantitative). The final BASDAI score has a range of 0 to 10.

3. The Bath Ankylosing Spondylitis Functional Index (BASFI) measures the physical function impairment caused by AS, and is a self-assessment instrument that consists of 8 specific questions regarding function in AS, and 2 questions reflecting the patient's ability to cope with everyday life. Each question is answered on a 10-cm horizontal visual analog scale, the mean of which gives the BASFI score (0-10).

4. The Bath Ankylosing Spondylitis Metrology Index (BASMI) consists of 5 simple clinical measurements that provide a composite index and define disease status in AS. Analysis of metrology (20 measurements) identified these 5 measurements as most accurately reflecting axial status: cervical rotation, tragus to wall distance, lateral flexion, modified Schober's test, and intermalleolar distance. The BASMI is quick (7 minutes), reproducible, and sensitive to change across the entire spectrum of disease. The BASMI index comprises 5 measures of hip and spinal mobility in AS. The five BASMI measures, scaled 0 (mild) to 10 (severe), include tragus to wall distance, cervical rotation, lumbar flexion, lumbar side flexion, and intermolleolar distance.

Combinations of the above-mentioned criteria are used to evaluate patients. In addition, radiographic, MRI, and bone and cartilage degradation markers can be used to determine disease activity in AS patients.

*Clinical studies examining D2E7 in human subjects with active AS*

Patients are administered a dose of D2B7 s.c in a placebo-controlled clinical trial over a period of weeks, and re-examined every 2-6 weeks for the next year to determine if AS symptoms are reduced or treated. A dose of 40 mg every other week, which is effective and safe in treating rheumatoid arthritis, is used in the study. Only patients who have a confirmed diagnosis of active AS, as defined by having 2 of the following 3 criteria- BASDAI index, a visual analog scale (VAS) for pain and the presence of morning stiffness- are chosen for the study. The BASDAI index is described in more detail above. In order to enroll in this study, patients must have significant pain at screening and at baseline, , a pain score of > 4 on a 10-cm VAS, and a BASDAI score of $\geq$ 4.
Disease-modifying antirheumatic drugs (DMARDS) or other immunosuppressive agents are allowed in the study. Patients are allowed to enroll if they are on an equivalent dose of < 10 mg of prednisone per day.
Screening examinations are performed prior to the study enrollment in order to document each patient's medical history and current findings. The following information is obtained from each patient: morning stiffness (duration and severity), occurrence of anterior uveitis (number of episodes and duration), and the number of inflamed peripheral joints. For each patient, radiographs of the vertebral column and the sacroiliac joints are obtained. Magnetic resonance imaging can also be used to document the spinal column of the patients enrolled
Patients are randomly divided into experimental and placebo groups, and are administered either D2E7 or the placebo once every two weeks in a blinded fashion until week 12 or week 24. D2E7 has been administered at doses of 20 to 80 mg that have been used effectively to treat rheumatoid arthritis; a 40 mg dose was determined to be effective. A higher dose might be necessary to treat spinal inflammation, so a higher dose (40 mg weekly in those patients who are nonresponders and who are not on methotrexate) is used in the study. The percentage of patients who achieve an ASAS20 is calculated.

## Example 3: TNF Inhibitor in Clinical Study for Psoriatic Arthritis

*D2E7 in human subjects with psoriatic arthrits*

Patients with moderate to severe psoriatic arthritis of any subtype (arthritis of the distal interphalangeal joints, arthritis mutilans, symmetric polyarthritis, asymmetric oligoarthritis and/or spoyloarthropathy) are selected for the study. Patients have either failed or exhibited intolerance to non-steroidal antiinflamatory drugs (NSAIDs) or disease modifying anti-rheumatic drugs (DMARDs). Therapy is given alone and/or in combination with NSAIDs and DMARDs.
Dosage ranges being evaluated include 40 mg every other week, which is the D2E7 dose which has been found to be most effective at treating rheumatoid arthritis in patients. Higher dose (40 mg every week) is also being studied. Studies are a comparison to placebo for 12 to 24 weeks followed by open label therapy to determine long term safety and efficacy.
Patients are examined clinically at screening, baseline, and frequently during treatment. The primary efficacy for signs and symptoms is measured via American College of Rheumatology preliminary criteria for improvement (ACR20) at 12 weeks. An additional primary endpoint includes evaluation of radiologic changes over 6 to 12 months to assess changes in structural damage. Multiple other evaluations are performed during treatment including Psoriatic Arthritis Response Criteria (PsARC), quality of life measurements, and skin evaluations to determine efficacy on psoriasis lesions (psorasis area severity index (PASI) and target lesion evaluations).

**Example 4: TNFα Inhibitor in Mouse Model for Asthma**

*TNF antibody study using ovalbumin (OVA)-inditced allergic asthma mice*

The mouse OVA model of allergic asthma (Hessel, E.M., et al. (1995) Eur. J. Pharmacol. 293:401; Daphne, T., et al. (2001) Am. J. Respir. Cell Mol. Biol. 25:751, is used in the following study for treating allergic asthma.

All mice are sensitized to OVA (chicken egg albumin, crude grade V; Sigma, St. Louis, MO). Active sensitization is performed without an adjuvant by giving seven intraperitoneal injections of 10 μg OVA in 0.5 ml pyrogen-free saline on alternate days (one injection per day). Three weeks after the last sensitization, mice are exposed to either 16 OVA challenges (2 mg/ml in pyrogen-free saline) or 16 saline aerosol challenges for 5 min on consecutive days (one aerosol per day). An additional group of mice first receive eight OVA aerosols, followed by eight saline aerosols (OVA/saline, spontaneous resolution group).

For the experiment in the more severe ongoing model of allergic asthma, all mice are sensitized to OVA by active sensitization with two intraperitoneal injections (7 d apart) of 0.1 ml alum-precipitated antigen, comprising 10 μg OVA adsorbed onto 2.25 mg alum (AlumImject; Pierce, Rockford, IL). Two weeks after the second sensitization, mice are exposed to either six OVA challenges (10 mg/ml in pyrogen-free saline) or six saline aerosol challenges for 20 min every third day (one aerosol every third day). An additional group of mice first receive three OVA aerosols, followed by three saline aerosols (OVA/saline, spontaneous resolution group).

The aerosol treatment is performed in a plexiglas exposure chamber (5 liter) coupled to a Pari LC Star nebulizer (PARI Respiratory Equipment, Richmond, VA; particle size 2.5-3.1 μm) driven by compressed air at a flow rate of 6 liters/min. Aerosol is given in groups composed of no more than eight animals.

A monoclonal anti-TNFα antibody which is known to bind and neutralize mouse TNFα, *e.g.*, antibody TN3 (TN3-19.12) (see Marzi et al. (1995) Shock 3:27; Williams et al. (1992) Proc Natl Acad Sci U S A. 89:9784; BD Biosciences Pharmingen) is administered to the OVA sensitized mice in a range of doses after the second sensitization according to standard protocols known in the art. Appropriate placebo controls are also administered.

Airway responsiveness is measured in conscious, unrestrained mice using barometric whole-body plethysmography by recording respiratory pressure curves in response to inhaled methacholine (acetyl-β-methylcholine chloride; Sigma). Briefly, mice are placed in a whole-body chamber, and basal readings are obtained and averaged for 3 min. Aerosolized saline, followed by doubling concentrations of methacholine (ranging from 1.6-50 mg/ml saline), are nebulized for 3 min, and readings are taken and averaged for 3 min after each nebulization. Dose-response curves (DRCs) to methacholine are statistically analyzed by a general linear model of repeated measurements followed by *post-hoc* comparison between groups. Data are LOG transformed before analysis to equalize variances in all groups.

After measurement of *in vivo* airway responsiveness, mice are sacrificed by intraperitoneal injection of 1 ml 10% urethane in pyrogen-free saline (Sigma). Subsequently, mice are bled by cardiac puncture, and OVA-specific IgE is measured by ELISA. Briefly, microtiter plates (Nunc A/S, Roskilde, Denmark) are coated overnight at 4°C with 2 μg/ml rat anti-mouse IgE (clone EM95) diluted in phosphate-buffered saline (PBS). The next day, the ELISA is performed at room temperature. After blocking with ELISA buffer (PBS containing 0.5% bovine serum albumin [Sigma], 2 mM EDTA, 136.9 mM NaCl, 50 mM Tris, 0.05% Tween-20 [Merck, Whitehouse Station, NJ] pH 7.2) for 1 h, serum samples and a duplicate standard curve (starting 1:10), diluted in ELISA buffer, are added for 2 h. An OVA-specific IgE reference standard is obtained by intraperitoneal immunization with OVA and arbitrarily assigned a value of 10,000 experimental units/ml (EU/ml). After incubation, 1 μg/ml of OVA coupled to digoxigenin (DIG), which is prepared from a kit containing DIG-3-*o*-methylcarbonyl-ε-aminocaproic acid-N-hydroxy-succinimide-ester (Roche Diagnostics, Basel, Switzerland) in ELISA buffer, is added for 1.5 h, followed by incubation with anti-DIG Fab fragments coupled to horseradish peroxidase (Roche Diagnostics) diluted 1:500 in ELISA buffer for 1 hour. Color development is performed with *o*-phenylenediamine-dichloride substrate (0.4 mg/ml, Sigma) and 4 mM $H_2O_2$ in PBS and stopped by adding 4 M $H_2SO_4$. The optical density is read at 492 nm, using a Benchmark microplate reader (Bio-Rad Laboratories, Hercules, CA). The detection limit of the ELISA is 0.5 EU/ml IgE.

Bronchial alveolar lavage (BAL) is performed immediately after bleeding of the mice. Briefly, the airways are lavaged five times through a tracheal cannula with 1-ml aliquots of pyrogen-free saline warmed to 37°C. The recovered lavage fluid is pooled, and cells are pelleted ($32 \times g$, 4°C, 5 min) and resuspended in 150 μl cold PBS. The total number of cells in the BALF is determined using a Bürker-Türk counting-chamber (Karl Hecht Assistent KG, Sondheim/Röhm, Germany). For differential BALF cell counts, cytospin preparations are made and stained with Diff-Quick (Dade AG, Düdingen, Switzerland). Per cytospin, 400 cells are counted and differentiated into mononuclear cells (monocytes, macrophages, and lymphocytes), eosinophils, and neutrophils by standard morphology. Statistical analysis is performed using the nonparametric Mann-Whitney *U* test.

Cytokine production by antigen-restimulated T cells in lung tissue is determined as described previously (Hofstra, C.L., et al. (1999) Inflamm. Res. 48:602). Briefly, the lungs are lavaged as described above and perfused via the right ventricle with 4 ml saline containing 100 U/ml heparin to remove any blood and intravascular leukocytes. Complete lung tissue is removed and transferred to cold sterile PBS. Lungs are then minced and digested in 3 ml RPMI 1640 containing 2.4 mg/ml collagenase A and DNase I (grade II) (both from RocheDiagnostics) for 30 min at 37°C. Collagenase activity is stopped by adding fetal calf serum (FCS). The lung tissue digest is filtered through a 70-$\mu$m nylon cell strainer (Becton Dickinson Labware, Franklin Lakes, NJ) with 10 ml RPMI 1640 to obtain a single-cell suspension. The lung-cell suspension is washed, resuspended in culture medium (RPMI 1640 containing 10% FCS, 1% glutamax I, and gentamicin [all from Life Technologies, Gaithersburg, MD]) and 50 mM P-mercaptoethanol (Sigma), and the total number of lung cells is determined using a Bürker-Türk counting-chamber. Lung cells ($8 \times 10^5$ lung cells/well) are cultured in round-bottom 96-well plates (Greiner Bio-One GmbH, Kremsmuenster, Austria) in the presence of OVA (10 $\mu$g/ml) or medium only. As a positive control, cells are cultured in the presence of plate-bound rat anti-mouse CD3 (clone 17A2, 50 $\mu$g/ml, coated overnight at 4°C). Each *in vitro* stimulation is performed in triplicate. After 5 days of culture at 37°C, the supernatant is harvested, pooled per stimulation, and stored at -20°C until cytokine levels were determined by ELISA.

The IFN-$\gamma$, IL-4, IL-5, IL-10, and IL-13 ELISAs are performed according to the manufacturer's instructions (PharMingen, SanDiego, CA). The detection limits of the ELISAs are 160 pg/ml for IFN-$\gamma$, 16 pg/ml for IL-4, 32 pg/ml for IL-5, and 100 pg/ml for IL-10 and IL-13.

In all experiments, airway responsiveness to methacholine, OVA-specific IgE levels in serum, cellular infiltration in the BALF, and T-cell responses in lung tissue are measured 24 hours after the last challenge in each mouse.

Improvements in asthma in the experimental mice are marked by a decrease in the number of mononuclear cells (including monocytes, macrophages, and lymphocytes), eosinophils, and neutrophils in the BALF, a decrease in the airway hyperresponsiveness, and a decrease in the cytokine production by antigen-restimulated T cells in the lung tissue.

## Example 5: TNF$\alpha$ Inhibitor in Mouse Model of Chronic Ostructive Pulmonary Disease (COPD)

*Study examining treatment for alveolar enlargement and inflammation*

The following study is performed using a cigarette smoke induced COPD mouse model (Keast, D.et al. (1981) J. Pathol. 135:249; Hautmaki, R.D., et al. (1997) Science 277:2002). In response to cigarette smoke, inflammatory cell recruitment into the lungs followed by pathologic changes characteristic of emphysema have been observed. Previous studies have shown that progressive inflammatory cell recruitment begins within the first month of smoking followed by air space enlargement after 3 to 4 months of cigarette exposure (Hautmaki et al. (1997) Science 277:2002).

Mice are exposed to smoke from two non-filtered cigarettes per day, 6 days per week, for 6 months, with the use of a smoking apparatus with the chamber adapted for mice. Nonsmoking, age-matched animals are used as controls. After 6 months of exposure to smoke as described above, a monoclonal anti-TNF$\alpha$ antibody which is known to bind and neutralize mouse TNF$\alpha$, *e.g.,* antibody TN3 (TN3-19.12) (see Marzi et al. (1995) Shock 3:27; Williams et al. (1992) Proc Natl Acad Sci USA. 89:9784; BD Biosciences Pharmingen) is administered in a range of doses according to standard protocols known in the art. An appropriate placebo control is also administered. Mice are administered the antibody treatment for a period of 21 days. Mice are sacrificed, followed by examination of lung volume and compliance, cytokine measurement, histological mucus index measurement, alveolar duct enlargement, air space measurement, alveolar and interstitial macrophage counts and alveolar size, as described below.

Following antibody treatment, bronchiolar lavage is performed on euthamized animals; the trachea is isolated by blunt dissection, and small caliber tubing is inserted and secured in the airway. Two volumes of 1.0 ml ofPBS with 0.1% BSA are instilled, gently aspirated, and pooled. Each BAL fluid sample is centrifuged, and the supernatants are stored in -70° until used. Cytoldne measurements are as described in Example 5.

To determine lung volume and compliance, animals are anesthetized, the trachea is cannulated, and the lungs are ventilated with 100% $O_2$ via a "T" piece attachment. The trachea is then clamped and oxygen absorbed in the face of ongoing pulmonary perfusion. At the end of this degassing, the lungs and heart are removed en bloc and inflated with PBS at gradually increasing pressures from 0 to 30 cm. The size of the lung at each 5-cm interval is evaluated via volume displacement. An increase in the lung volume of treated animals compared to placebo treated control animals indicates an improvement in COPD.

For histological analysis, animals are sacrificed and a median sternotomy is performed, and right heart perfusion is accomplished with calcium- and magnesium-free PBS to clear the pulmonary intravascular space. The lungs are then fixed to pressure (25 cm) with neutral buffered 10% formalin, fixed overnight in 10% formalin, embedded in paraffin, sectioned at 5 $\mu$m and stained with Hematoxylin and eosin (H&E) and periodic acid-Schiff with diastase (D-PAS).

The histological mucus index (HMI) provides a measurement of the percentage of epithelial cells that are D-PAS[+] per unit airway basement membrane. It is calculated from D-PAS-stained sections (Cohn, L., et al. (1997) J. Exp. Med. 186:1737). A decrease in the HMI of treated animals compared to placebo treated control animals indicates an improvement in COPD.

*Lm*, an indicator of air space size, is calculated for each mouse from 15 random fields at $\times$20D by means of a 50-line counting grid (10-mm total length). The results are the average of measurements of two independent investigators. An increase in air space size of treated animals compared to placebo treated control animals indicates an improvement in COPD.

To determine alveolar duct enlargement, the proximal surface areas from the terminal bronchiole-alveolar duct transition extending 250 $\mu$m into the duct using Optimus 5.2 image analysis software (Optimus, Bothell, WA) is measured. A decrease in alveolar duct size of treated animals compared to placebo treated control animals indicates an improvement in COPD.

Alveolar and interstitial macrophages are quantitated by counting macrophages identified by murine Mac-3 (rat antibody to mouse (0.5 mg/ml), used at 1:4000 dilution; PharMingen, San Diego, CAO immunostaining using the avidin-biotin alkaline. A decrease in the number of alveolar and interstitial macrophages of treated animals compared to placebo treated control animals indicates an improvement in COPD.

Alveolar size is estimated from the mean cord length of the airspace (Ray, P., et al. (1997) J. Clin. Invest. 100:2501). This measurement is similar to the mean linear intercept, a standard measure of air space size, but has the advantage that it is independent of alveolar septal thickness. Sections are prepared as described above. To obtain images at random for analysis, each glass slide is placed on a printed rectangular grid and a series of dots placed on the coverslip at the intersection of the grid lines, i.e., at intervals of approximately 1 mm. Fields as close as possible to each dot are acquired by systematically scanning at 2-mm intervals. Fields containing identifiable artifacts or non-alveolated structures such as bronchovascular bundles or pleura are discarded.

A minimum often fields from each mouse lung are acquired into a Macintosh G3 computer (Apple Computer Inc., Cupertino, California, USA) through a framegrabber board. Images are acquired in 8-bit gray-scale at a final magnification of 1.5 pixels per micron. The images are analyzed on a Macintosh computer using the public domain NIH. Image program written by Wayne Rasband at NIH using a custom-written macro available from the web site (http://rsb.info.nih.gov/nih-image). Images are manually thresholded and then smoothed and inverted. The image is then subject to sequential logical image match "and" operations with a horizontal and then vertical grid. At least 300 measurements per field are made for each animal. The overlying air space air is averaged as the mean chord length. Chord length increases with alveolar enlargement. An increase in alveolar size of treated animals compared to placebo treated control animals indicates an improvement in COPD.

**Example 6: TNF$\alpha$ Inhibitor in Idiopathic Pulmonary Fibrosis (IPF) Mouse Model.**

*Study of IPF treatment using bleomycin induced lung fibrosis mouse model*

The following study is performed using the bleomycin induced lung fibrosis mouse model (reviewed in Bowden, D.H. (1984) Lab. Invest. 50:487; Tokuda, A., et al. (2000) J. Immunol. 164:2745).

Bleomycin sulfate is administered to C57BL/6J female mice aged 8-10 weeks. Briefly, C57BL/6J mice are anesthetized with 200 $\mu$l of 5 mg/ml pentobarbital injected. i.p., followed by intratracheal instillation of 3 mg/kg bleomycin sulfate in 50 $\mu$l sterile saline.

A monoclonal anti-TNF$\alpha$ antibody which is known to bind and neutralize mouse TNF$\alpha$, e.g., antibody TN3 (TN3-19.12) (see Marzi et al. (1995) Shock 3:27; Williams et al. (1992) Proc Natl Acad Sci USA. 89:9784; BD Biosciences Pharmingen) is administered to the bleomycin induced lung fibrosis mice in a range of doses, after intratracheal instillation of bleomycin as described above. An appropriate placebo control is also administered. Mice ar treated twice daily for 14 days.

Mice are sacrificed 20 and 60 days following bleomycin treatment. Tissues are fixed in 10% buffered formalin and embedded in paraffin. Sections are stained with hematoxylin and eosin and examined by light microscopy. Lung-infiltrating leukocyte counts, cytokine measurements, and total lung collagen content is determined as described below.

BAL cells and lung-infiltrating leukocytes are prepared as described in Smith et al. (1994) J. Immunol. 153:4704. In brief, following anesthesia, 1 ml PBS is instilled and withdrawn five times from the lung via an intratracheal cannula. The BAL fluids are collected and after RBC lysis total leukocyte counts are determined. Cell differentials are determined after cytospin centrifuge. Specimens are stained with Diff-Quik products (Baxter, Miami, FL).

To isolate lung-infiltrating leukocytes, lungs are perfused with saline, dissected from the chest cavity, and then minced with scissors. Each sample is incubated for 30 minutes at 37°C on a rocker in 15 ml digesting buffer (10% FCS in RPMI 1640 with 1% collagenase; Wako Pure Chemical, Osaka, Japan). Next, the sample is pressed through nylon mesh and suspended in 10%FCS-RPMI 1640 after being rinsed. The cell suspension is treated with Histopaque-1119 (Sigma, St. Louis, MO) and centrifuged at 2000 rpm for 20 min to remove lung parenchymal cells and RBC. The pellet is resuspended in 2.5% FCS-PBS after being rinsed. After cell counts are performed, flow cytometric immunofluorescence analyses are conducted.

Immunofluorescence analyses of peripheral blood leukocytes and lung-infiltrating leukocytes are performed with the use of an Epics Elite cell sorter (Coulter Electronics, Hialeah, FL) as described previously (Yoneyama et al. (1998) J. Clin. Invest. 102:1933; Murai et al. (1999) J. Clin. Invest. 104:49). Peripheral blood leukocytes are prepared from normal mice with RBC lysis buffer. After incubation with Fc Block (anti-CD16/32; PharMingen, San Diego, CA) for 10 min, cells are stained with PE-conjugated mAb against CD3, CD4, CD8, CD11b, CD11c, and Gr-1 (PharMingen), and also stained with 20 µg/ml of rabbit anti-CCR1 polyclonal Ab followed by staining with FITC-conjugated goat anti-rabbit IgG (Leinco Technologies, St. Louis, MO). Before analyses propidium iodide (Sigma) staining is performed to remove the dead cells. A decrease in the number of lung-infiltrating leukocytes of treated animals compared to placebo treated control animals indicates an improvement in IPF.

Immunohistochemistry of lung samples is carried out as follows: lung specimens are prepared as described previously (Yoneyama et al. (1998) J. Clin. Invest. 102:1933; Murai et al. (1999) J. Clin. Invest. 104:49). Briefly, lung specimens are fixed in periodate-lysine-paraformaldehyde, washed with PBS containing sucrose, embedded in Tissue-Tek OCT compound (Miles, Elkhart, IN), frozen in liquid nitrogen, and cut into 7-µm-thick sections with a cryostat. After inhibition of endogenous peroxidase activity, the sections are incubated with the first Ab. The Abs used are rabbit anti-CCR1 Ab, rat anti-F4/80 (BMA Biomedicals, Geneva, Switzerland), rat anti-CD4,rat anti-CD8, rat anti-Gr-1 (PharMingen), rat anti-nonlymphoid dendritic cell (NLDC)-145, and rat anti-MHC class II (BMA Biomedicals). As a negative control either a rabbit IgG or a rat IgG is used, respectively. They are treated sequentially with either HRP-conjugated goat anti-rabbit IgG (Cedarlane Laboratories, Hornby, Ontario, Canada) or a HRP-conjugated goat anti-rat IgG (BioSource International, Camarillo, CA). After staining with 3,3'-diaminobenzidine (Wako Pure Chemical) or 3-amino-9-ethylcarbazole substrate kit (Vector Laboratories, Burlingame, CA), samples are counterstained with Mayer's hematoxylin. A decrease in CCR1, and decreases in the number of CD4+ T cells, , CD8+ T cells, nonlymphoid dendritic cell (NLDC), and MHC class II bearing cells of treated animals compared to placebo treated control animals indicates an improvement in IPF

Total lung collagen content is determined by assaying total soluble collagen using the Sircol Collagen Assay kit (Biocolor, Northern Ireland) according to the manufacturer's instructions. Briefly, lungs are harvested at day14 after bleomycin administration and homogenized in 10 ml 0.5 M acetic acid containing about 1 mg pepsin/10 mg tissue residue. Each sample is incubated for 24 h at 4°C with stirring. After centrifugation, 200 µl of each supernatant is assayed. One milliliter of Sircol dye reagent that binds to collagen is added to each sample and then mixed for 30 min. After centrifugation, the pellet is suspended in 1 ml of the alkali reagent included in the kit and read at 540 nm by a spectrophotometer. Collagen standard solutions are utilized to construct a standard curve. Collagens contain about 14% hydroxyproline by weight, and collagen contents obtained with this method correlate well with the hydroxyproline content according to the manufacturer's data. A decrease on lung collagen content of treated animals compared to placebo treated control animals indicates an improvement in IPF

Using the bleomycin induced lung fibrosis mouse model, mice are examined for a decrease in the BAL cell counts, a decrease in the peripheral blood leukocytes and lung infiltrating leukocytes. Mice are also examined for a decrease in the total lung collagen content in D2E7 treated mice as compared to placebo treated mice.

## Example 7: TNFα Inhibitor in Treatment of Asthma

*Clinical study of D2E7 in human subjects with asthma*

Patients 12 to 65 years of age are eligible for the study if they have had a documented diagnosis of asthma of at least 2 years duration and have also had demonstrable reversible bronchospasm with an increase in FEV1 of 15% or greater after the administration of albuterol within the previous six months. Additional inclusion criteria include, a baseline FEV1 between 50% and 80% of predicted normal, absence of any clinically significant disease other than asthma, a history of daily use of inhaled corticosteroids and cessation of all β2-agonist use 30 days prior to the beginning of the study.

A baseline visit occurs within 7 days after the screening visit. All patients undergo evaluation of FEV1 and have a complete physical examination. Pulmonary auscultation and oropharyngeal examinations are performed, and asthma symptoms are assesses. Patients who qualify are randomly assigned to a treatment group including a placebo group.

Following baseline measurements, patients begin receiving treatment. They are randomized and treated with either D2E7 or placebo in a blinded fashion. At days 15 and 29, all examinations performed at the baseline visit are repeated. A 12-lead ECG is also performed. Diary cards are reviewed with patients regarding the use of other medications and any adverse events.

Improvements are determined on spirometry tests measured at each visit. These include FEV1, peak expiratory flow rate (PEFR), Forced Vital Capacity (FCV), and forced expiratory flow at 25% to 75% of FVC. FEV1 at the final visit is regarded as the primary measure of efficacy. Twice-daily PEFR tests performed by the patient are compared and the number of inhalations of rescue medication is calculated. Patient/physician evaluations of asthma symptoms (wheezing, tightness in the chest, shortness of breath and cough) are characterized by severity. Compliance is assessed by review of the patient's diary cards and by collecting unused study medication.

**Example 8: TNFα Inhibitor in Treatment of COPD**

*Clinical study examining D2E7 in human subjects with COPD*

The study population is male and female subjects who are 40 to 80 years of age with a diagnosis of COPD. Subjects must have a best FEVi/FVC ratio ≤0.70 liters, fixed airway obstruction, defined by ≤15% or ≤200 ml (or both) increase in FEV1 after the administration of albuterol and a post-albuterol FEV1 between 30 and 70% of predicted. Subjects must also be current or previous smokers with a history of smoking ≥10 pack years.

Following baseline measurements, patients begin receiving treatment. They are randomized and treated with either D2E7 or placebo in a blinded fashion.

Improvements are marked by an increase from predose baseline after study medication in pre-bronchodilator FEV1 and change from baseline in total score of the St. George's Respiratory Questionnaire (Jones, P.W., et al. (1991) Resp. Med. 85(suppl):25) which indicates an improvement in the patients' quality of life. Improvements are also seen as an increase from baseline FVC at trough, an increase in time to first COPD exacerbation, and a decrease from baseline in post-exercise breathlessness (modified Borg Scale; Stulbarg, M., Adams, L. Dyspnea. In: Murray J, Nadel J, eds. Textbook of Respiratory Medicine. Philadelphia, PA: WB Saunders, 2000; 541-552). Measures of safety are adverse events, vital signs, electrocardiogram at all double-blind visits, and laboratory assessments.

**Example 9: TNFα Inhibitor in Treatment of IPF**

*Clinical study of D2E7 in human subjects with IPF.*

A multi-center, double-blind, placebo-controlled study comparing treatment of IPF patients with D2E7 versus treatment with placebo is performed. Patients are eligible for the study if they have histologically verified IPF and have a decline in lung function of at least 10% during the 12 months prior to the beginning of the study, despite continuous or repeated treatment with glucocorticoids or other immunosuppressive agents or both for at least 6 months. The main histological feature used to identify IPF is the presence of subpleural and periacinar fibrotic lesions with only minor cellular infiltration. The absence of bilateral patchy infiltrates on high-resolution computed tomography and the demonstration of predominantly peripheral distribution of lesions are the radiological criteria for identifying the disease. Patients with a history of exposure to organic or inorganic dust or drugs known to cause pulmonary fibrosis and those with connective-tissue disease or other chronic lung diseases are excluded. Patients with end-stage IPF as identified on the basis of a total lung capacity of less than 45% of the predicted normal are also excluded. Baseline values for repeat pulmonary function tests, FVC, total lung capacity (TLC), and oxygen saturation are taken.

Following baseline measurements, patients begin receiving treatment They are randomized and treated with either D2E7 or placebo in a blinded fashion.

Improvements in IPF patients include an increase in the overall survival rate of patients in the study, and improvements in FVC, total lung capacity (TLC) and oxygen saturation. Improvement in pulmonary function is defined as a 10% or greater increase in predicted value of FVC or TLC, or a 3% or greater increase in oxygen saturation with the same fraction of expired air, resting or exertional. A decrease of similar manner for each measure is considered a deterioration. Patients who do not demonstrate improvement or deterioration are considered stable.

**Example 10: TNFα Inhibitor In Reducing Inflammation and Restenosis**

*Study of restenosis using mouse carotid artery model*

The following study of restenosis is performed using the mouse carotid artery model (Kumar and Lindner (1997) Arterioscler. Thromb. Vasc. Biol. 17:2238; de Waard et al. (2002) Arterioscler. Thromb. Vasc. Biol. 22:1978). Mice, ranging in age from two to four months, are anesthetized by intraperitoneal (i.p.) injection of a solution of xylazine. The left common carotid artery is dissected and ligated near the carotid bifurcation. Mice are then allowed to recover.

A monoclonal anti-TNFa antibody which is known to bind and neutralize mouse TNFα, *e.g.,* antibody TN3 (TN3-19.12) (see Marzi et al. (1995) Shock 3:27; Williams et al. (1992) Proc Natl Acad Sci USA. 89:9784; BD Biosciences Pharmingen) is administered to the experimental group. Mice receive daily subcutaneous injections per week of either the anti-TNF antibody or a placebo. At either 2.5 or 4 weeks after the ligation of the carotid artery, mice are sacrificed and subsequently fixed by perfusion with 4% paraformaldehyde in PBS. The carotid arteries are excised, immersed in 70% (v/v) ethanol, and embedded in paraffin. The nonligated right carotid artery serves as an internal control for both the D2E7 injected and placebo injected mice. Serial sections are cut for morphometric analysis, as described in de Waard *et al., supra.*

Morphometric analysis provides a measurement of the total vessel area for the treated and untreated ligated carotids at certain set distances from a common physical reference point. It has previously been shown that the ligation results in the narrowing of the arteries (constructive remodeling) (Kumar and Lindner, *supra;* Kumar et al. (1997) Circulation 96:4333). Cross sections of the carotids are mounted on microscopic slides and stained with hematoxylin and eosin. Images of the carotid arteries are obtained using microscopic digital photography and the cross sectional areas of the intimal and the media are measured for a decrease in arterial narrowing (i.e, larger vessel diameter) as compared to placebo injected mice.

**Example 11 : TNFα Inhibitor in Monkey Model of Atherosclerosis**

*Effect of D2E7 in monkey model of atherosclerosis*.

The following study is performed using a diet-induced monkey model of atherosclerosis (Lentz SR et al. (2002) Circulation 106(7):842-6; Sundell CL et al. (2003)305(3):1116-23).

Adult cynomolgus monkeys (*Macaca fascicularis)* are fed an atherogenic diet that contains 0.7% cholesterol and 43% total calories as fat After $44 \pm 1$ months on the atherogenic diet, animals are sedated with ketamine hydrochloride (20mg/kb IM) and anesthetized with sodium pentobarbital (20mg/kg IV). A nonobstructive catheter is inserted into an axillary artery for blood sampling, and the axillary vein is cannulated for administration of either D2B7 or placebo and supplemental anesthesia (sodium pentobarbital 5mg/kg per hour). D2E7 has been shown to effectively inhibit TNFα activity in a variety of species, including cynomolgus monkeys (see U.S. Patent No. 6,258,562).

Prior to infusion of D2E7 or placebo, blood is collected from the axillary artery catheter directly into a 1/10 volume of 3.8% sodium citrate for hemostatic assaying. After collection, blood samples are placed immediately on ice, and plasma is isolated by centrifugation at 2500g for 30 minutes at 4°C. Additional blood samples are collected into serum separator tubes for determination of cholesterol or into serum separator tubes prepared with 3.4 mmol/L EDTA for determination of total plasma homocysteine (tHCY).

D2E7 or placebo is infused in 10ml of saline over 10 minutes through the axillary vein catheter. After infusion, blood samples are collected regularly.

The degree to which the animals are suffering atherosclerosis after treatment is assessed in various ways. Serum samples are regularly taken from the monkeys and assayed for total cholesterol, HDL cholesterol, LDL cholesterol, tHCY and triglycerides. Treated monkeys are examined to determine if total cholesterol, and LDL cholesterol, and tHCY levels are lower as compared to placebo treated monkeys, and whether HDL levels are higher.

**Example 12: TNFα Inhibitor on Treating Restenosis in Patients**

*Study of D2E7 in human subjects with restenosis*

Patients who have undergone balloon angioplasty are chosen for the study, as they have an increased chance of restenosis occurring within the first six months following angioplasty.

Prior to treatment, estimates of vessel and lesion parameters are made with reference to the guiding catheter. Estimates include reference vessel diameter (RVD), pretreatment minimal luminal diameter (MLD, which is determined by (RVD X [1-preprocedural percent diameter stenosis]), postprocedural MLD (which is determined by (RVD X [1 - postprocedural percent diameter stenosis]), acute gain (postprocedural MLD - preprocedural MLD), number of diseased vessels and number of traded vessels.

Experimental group of patients are administered either D2E7 in biweekly and weekly doses of 40 mg or a placebo. Dosages may be adjusted by an ordinarily skilled artisan knowledgeable in restenosis. Patients are following and assessed at six months post-angioplasty to determine whether restenosis has occurred. Patients are also assessed at 9 months and long-term to determine the effect of delayed restenosis in those groups where restenosis was prevented or reduced due to treatment. Estimates of vessel and lesion parameters are recorded following D2E7 treatment. Statistical analysis is performed to compare the extent of restenosis in the patients. (Jackson et al. (2003) Am Heart J 145:875).

**Example 13: TNFα Inhibitor on Treating Heart Failure**

*Clinical study of D2E7 in human subjects with heart failure*

Patients with stable New York Association (NYHA) class II or IV heart failure and left ventricular ejection fraction of less than 35% are chosen for the study. Under the NYHA standard, class III patients are defined as those with marked limitation of activity, i.e., they are comfortable only at rest, and class IV patients are defined as those who should be at complete rest, *i.e.,* confined to bed or chair, or where any physical activity brings on discomfort and symptoms occur at rest. As described in Burns *et al.,* left ventricular ejection fraction is associated with six-month mortality (Burns et al.

(2002) J Am Coll Cardiol. 39:30).

Patients receive biweekly doses of D2E7 at 40 mg, or a dosage adjusted by an ordinarily skilled artisan knowledgeable in heart failure. The control group is given a placebo. Patients undergo examinations at 1, 2, 6, 10, 14, 20, and 18 weeks. At each visit, each patient is examined and given an assessment of their overall heart failure status, relative to their status at the onset of the study, *i.e.,* their NYHA class is assessed. At the end of the heart failure study, the patient's final NYHA class is compared to the initial NHY A class.

## Example 14: TNFα Inhibitor in Mouse Model for Diabetes

*Study of TNF antibody in NOD mouse model*

The following study is performed using the nonobese diabetic (NOD) mouse model for type 1 diabetes. At the onset of the study, insulin levels are established by testing glucose levels in the blood of the NOD mice. Baseline insulin levels are established by fasting the mice overnight (17 hours). The blood glucose level is checked, and checked again 4 minutes after administering glucose. Blood glucose is determined with a reflectance meter. Glucose (200 mg/mL in 0.85% sodium chloride) in 1 mL syringes were prewarmed to 40°C and mice injected ip at 3 g/kg body weight. The second blood glucose measurement is determined 4 minutes after administering the glucose. Samples of the second blood measurement are used to determine the blood glucose level using the Glucometer Elite. The remaining sample of blood is collected into microfuge tube and used to separate the serum for insulin or C-peptide determination. Insulin levels are determined using a rodent radioimmunassay (RIA) kit per manufacturers' instruction or an enzyme-linked immunoassay (ELISA).

Diabetic mice are chosen based on the criteria that they have blood glucose readings greater than 300 mg/dL. Non-diabetic mice are chosen such that their glucose readings are under 200 mg/dL by glucose meter. NOD mice (those which displayed the glucose reading described above) are allowed to develop diabetes, and are administered doses of a placebo or a monoclonal anti-TNFα antibody which is known to bind and neutralize mouse TNFα, *e.g.,* antibody TN3 (TN3-19.12) (*see* Marzi et al. (1995) Shock 3:27; Williams et al. (1992) Proc Natl Acad Sci USA. 89:9784; BD Biosciences Pharmingen). The mice receive daily subcutaneous injections of the TNF antibody or a placebo. Insulin and glucose levels are measured at weekly increments to determined whether there is a decrease in blood glucose levels.

## Example 15: TNFα Inhibitor in Mouse Model of Diabetes

*Study of TNF antibody in type-2 diabetic mouse model*

The following study is performed using the NSY mouse model (type 2 diabetes) (Ueda et al, Diabetes Vol. 48, May 1999, 1168: 1174). The NSY mouse closely mimics human type 2 diabetes in that the onset is age-dependant, the animals are not severely obese, and both insulin resistance and impaired insulin response to glucose contribute to disease development. This study evaluates a number of phenotypic data, including glucose levels, insulin levels, height, and weight of the mouse.

Glucose is measured in the NSY mouse according to standard techniques, including by an intravenous glucose-tolerance test Baseline glucose resistance is measured prior to 12 weeks before the initiation of the study, and glucose, insulin, height, and weights are charted accordingly.

NSY mice are administered doses of either a placebo or a monoclonal anti-TNFα antibody which is known to bind and neutralize mouse TNFα, *e.g.*, antibody TN3 (TN3-19.12) (see Marzi et al. (1995) Shock 3:27; Williams et al. (1992) Proc Natl Acad Sci U S A. 89:9784; BD Biosciences Pharmingen). Mice receive daily subcutaneous injections of the anti-TNF antibody or a placebo. Glucose level measurements are taken 120 minutes after intraperitoneal glucose administration at 0, 12, 24, 36, and 48 weeks following the initiation of the study to examine whether there is a decrease in glucose intolerance.

## Example 16: TNFα Inhibitor in Obese Mouse Model

*Study of TNF antibody in mouse model for obesity*

The following study is performed using the obese mice (*ob/ob*) murine model. Mice are evaluated for weight loss and a reduction in their body mass index. Obese mice are characterized by marked obesity, hyperphagia, transient hyperglycemia and markedly elevated plasma insulin concentration associated with an increase in number and size of the beta cells of the islets of Langerhans (Coleman, *supra*). Obese mice (*ob/ob*) are phenotypically distinguished from their lean littermates (*ob/+* and *+/+*) at about 26 days of age on basis of body weight. Obese mice gain weight rapidly and have marked obesity at 5 weeks of age. Obese mice reach a maximum body weight of 60-70 grams at an age of 7-8 months, while lean littermates reach their maximal weight of 30-40 grams in 3-4 months (Coleman, *supra;* Westman (1968) Diabetologia 4:141; Bray & York (1971) Physiological reviews. 51:598).

Thirteen (13) week old *ob/ob* mice and matched wild-type control mice are weighed to establish a base line weight. The mice are administered doses of either a placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). Mice receive daily subcutaneous injections of the TNF antibody or a placebo. All mice are fed a high-fat diet (58% fat, Research Diets D12330) for 12 weeks. Body weights are recorded weekly. After 12 weeks, the mice are euthanized, and the fat pads are dissected and weighed, as well as the final weight of the animal to determine the final body mass index (BMI) and occurrence of obesity.

Alternatively, *ob/ob* mice can be treated with D2E7 beginning at birth, and fed a regular diet, *i.e.,* not low-fat, not high-fat diet. Treated and control mice (*ob/ob* littermates) are weighed weekly. Normally, at five weeks *ob/ob* mice exhibit a BMI which indicates that they are obese. Mice are examined at five weeks to determine if they have a lower BMI measurement than the controls.

**Example 17: TNFα Inhibitor in Treating Type 2 Diabetes in Humans**

*Study of D2E7 in human subjects with diabetes type 2*

Patients who are diagnosed with type 2 diabetic are selected for the study. The following inclusion criteria are used: 40-65 years of age, known duration of diabetes > 12 months, stable BMI < 35 kg/m2, supine blood pressure< 140/90 mm/Hg, serum creatinine <106 $\mu$mol/l, m24-h UAE between 20 and 200 $\mu$g/min in samples assessed weekly during the 3 months prior to the first evaluation and in the 15-day placebo run-in period, and no cardiovascular, hepatic, or systemic disease before the beginning of the study. The subjects do not take any additional drugs other than those for the treatment of their diabetes. For three days prior to and throughout the duration of the study, the patients follow an isocaloric diet (~0.13 mJ x kg -1 X day -1; 50% carbohydrates, 35% lipids, 15% proteins) with no restriction on sodium intake. Adherences to the dietary recommendations are checked at each visit

Patients are administered 40 mg of D2E7 in a biweekly dosing regiment, although this dose and the frequency of the dose can be adjusted by an ordinarily skilled artisan with knowledge of HCV treatments. Patients are monitored at least every week for twelve weeks, with repeated assays like those which were performed prior to the initiation of the D2E7 treatment and as described below.

For each patient's evaluation throughout the study, the following baseline examinations are performed: supine blood pressure measurements; BMI; the mean of three twenty four hour urine samples; blood glucose levels; twenty four hour urine glucose; serum creatine levels; creatinine clearance; and an electrocardiogram reading. Furthermore, each subject keeps a daily journal to monitor typical type 2 diabetic symptoms such as fatigue, excessive thirst, frequent urination, blurred vision, a high rate of infections, wounds that heal slowly, mood changes, and sexual problems. Patients are examined to determine if there is a reduction in blood glucose levels in D2E7, as well as reduction in symptoms typical to type II diabetes such as fatigue, excessive thirst, frequent urination, blurred vision, a high rate of infections, wounds that heal slowly, and mood changes.

**Example 18: TNFα Inhibitor in Iron Deficiency Anemia**

*Study of TNF antibody in rat model of iron deficiency*

The following study is performed using the rat animal model of iron deficiency anemia (Catani et al (2003) Braz. J. Med. Biol. Res. 36;693). Male Wistar-EPM rats (approximately three weeks old) are fed an AIN-93G (American Institute of Nutrition Rodent Diets) iron-free diet for a period of two weeks to induce iron deficiency anemia. Rats are administered doses of a placebo or a monoclonal anti-TNFα antibody which is known to bind and neutralize rat TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). Blood samples are taken pre and post treatment to determine hemoglobin and hematocrit values. For the analysis of hematocrit and hemoglobin concentration, blood is collected and mixed with 5ml of 0.5 M EDTA. Hematocrit is determined by centrifugation of blood in sealed heparinized capillaries. Hemoglobin concentrations are calculated from the absorbance of cyanmethemoglobin at 546 nm. Rats are examined to determine if there was an improved hematocrit measurement.

**Example 19: TNFα Inhibitor Study of Chronic Disease Anemia**

*Study of TNF antibody on anemia associated with chronic inflammatory disease*

The following study is performed using a rat model of anemia of chronic disease (Coccia et al, (2001) *Exp*. *Hematology* 29;1201). Eight to ten week old female Lewis rats are inoculated on day 0 with an intraperitoneal (i.p.) injection of peptidoglycan-polysaccharide polymers (PG-APS) suspended in 0.85% saline equilibrated to a dose of 15 μg rhamnose/kg. Blood is collected via tail veins into EDTA-coated Microtainer tubes and complete blood counts (CBC) are performed on an ADVA 120 Hematology System calibrated for rat blood. An additional blood sample is collected and separated on Microtainer serum separator centrifuge tubes and sera are analyzed for iron, bilirubin, and endogenous EPO concentrations. Rats are administered doses of a placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen), and examined for improved iron, bilirubin, and EPO concentration measurements.

**Example 20: TNFα Inhibitor on Anemia**

*Study of D2E7 antibody in human subjects with anemia*

Patients who exhibit symptoms commonly associated with anemia are examined and tested to determine if they suffer from anemia. Symptoms commonly associated with anemia are fatigue, chest pain, shortness of breath, pale complexion, and rapid heart rate. Examples of tests which indicate anemia are the complete blood count (CBC), reticulocyte count, and measurements of iron supply, including the serum iron, total iron-binding capacity, and serum ferritin. In patients with sever anemia and abnormalities in red blood cell morphology, a bone marrow aspirate and biopsy are important diagnostic tools. Patients who suffer from anemia are selected for the study.

In the CBC test, automated cell counters measure a number of parameters as part of the CBC, including the hemoglobin, red blood cell count, red blood cell volume distribution, platelet count, and white blood cell count The counter also calculates the hematocrit (based on the RBC count and volume), the mean cell volume (MCV) (based on volume distribution), mean cell hemoglobin (MCH)(hemoglobin divided by hematocrit), and the red cell distribution width (RDW). The red cell indices and RDW are used together with a direct inspection of the Wright-stained blood smear to evaluate red blood cell morphology.

Like the CBC test, an accurate measure of the reticulocyte count is key to the initial classification of any anemia. Reticulocytes are newborn red blood cells that contain sufficient residual RNA that they can be stained with a supravital dye and counted as a percent of the circulating red cell population. In the basal state, the normal reticulocyte count ranges from 1 to 2 percent according to the counting method. This correlates with the normal daily replacement of approximately 1 percent of the circulating red blood cell population. Increases in the reticulocyte count provide a reliable measure of the red blood cell production response to anemia.

To use the reticulocyte count as a production measure, it must first be corrected for changes in the patient's hematocrit and for the effect of erythropoietin on the early release of marrow reticulocytes into circulation. The hematocrit (HCT) correction converts the reicultocyte percentage to an absolute number:

$$\text{\% Reticulocytes X } \frac{\text{patient HCT}}{45\%} = \text{absolute \% reticulocytes}$$

The marrow reticulocyte ("shift") correction involves dividing the absolute percentage by a factor of 1.5 to 2.5 whenever there is prominent polychromasia on the peripheral blood smear. The shift correction should always be applied to any patient with anemia and a very high reticulocyte count to provide a true index of effective red blood cell production. A normal patient will respond to a hematocrit less than 30 percent with a two-to three-fold increase in the reticulocyte production index. This measure alone, therefore, will confirm the fact that the patient has an appropriate erythropoietin response, a normal erythroid marrow, and sufficient iron supply to meet the challenge. When the reticulocyte index falls below 2, a defect in marrow proliferation or precursor maturation must be present

Standard measures of iron supply include the serum iron, transferring iron binding capacity (TIBC), and the serum ferritin level. The normal serum iron ranges from 9 to 27 μmol/L (50 to 150 μg/dL), while the normal TIBC is 54 to 64 μmol/L (300 to 360 μg/dL). Therefore, in the basal state, only 30 to 50 percent of the transferring in circulation is saturated with iron. Important information is provided by each measurement as well as the calculated percent saturation. The serum ferritin is used to evaluate body iron stores. Adult males have serum ferritin levels of between 50 and 150 mg/L, corresponding to iron stores of from 600 to 1000 mg. Adult females have lower serum ferritin levels (15 to 50 mg/L) and smaller iron stores (0 to 300 mg). Lower serum ferritin levels are observed as iron stores are depleted; levels below 15mg/L indicate store exhaustion and iron deficiency.

A sample of bone marrow is readily obtained by needle aspirate or biopsy. It is of greatest value in patients who have a hypoproliferative anemia or a disorder of red blood cell maturation, providing valuable information as to marrow structure and cellularity, as well as precursor proliferation and maturation. The ratio of erythroid to granulocytic precursors (E/G ration) is used to asses the proliferative capacity of erythorid precursors. A patient with hypoproliferative anemia and a reticulocyte index <2 will demonstrate an E/G ratio ≤1:3 or 1:2. In contrast, the hemolytic anemia patient with a production index ≥3 to 5 will have an E/G ratio >1:1. Red cell precursor maturation defects are identified from the mismatch between the B/G ratio and reticulocyte production index. These individuals demonstrate and B/G ratio of greater than 1:1 together with a low reticulocyte index, typical of the ineffective erythorpoiesis of a maturation disorder.

Following baseline measurements, patients begin receiving treatment. They are randomized and treated with either D2E7 or placebo in a blinded fashion. Patients' complete blood count (CBC), reticulocyte count, and measurements of iron supply are monitored at least every two weeks.

## Example 21: TNFα Inhibitor in Animal Model of Neuropathic Pain

*TNF antibody in rat sciatic nerve ligation model*

The following study is performed using the rat sciatic nerve ligation model for neuropathic pain (Bennett and Zie (1988) Pain 33:87). Baseline behavioral measurements (response to mechanical allodynia and heat hyperalgesia, protocols are described below) are made prior to surgery. Heat hyperalgesia refers to the rat heat pain threshold, and mechanical allodynia refers to the response threshold to light tactile stimuli. Male Sprague-Dawley rats, weighing between 120-150 grams, are anesthetized and a sciatic nerve ligation procedure is performed on each. The sciatic nerve ligation procedure involves exposing the common sciatic nerve, which is then tied loosely with 4 ligatures with about 1 mm spacing. Rats are allowed to recover and are administered doses of either a placebo or a monoclonal anti-TNFα antibody which is known to bind and neutralize rat TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). The experimental groups receive daily subcutaneous injections per week of TNF antibody or a placebo.

Following the surgery, mechanical allodynia and heat hyperalgesia are performed on a weekly basis for 10 weeks. Analgesia testing examines responses to noxious heat and is determined by placing the rats in a chamber with a clear glass floor and aiming a radiant heat source from beneath the floor at the plantar surface of the affected foot. Withdrawal latency and duration are recorded. Increased latency to withdraw the hind paw after treatment is demonstrative of analgesic activity.

Responses to normally innocuous mechanical stimuli (mechanical allodynia measurement) are determined by placing the rats in a chamber with a screen floor and stimulating the plantar surface of the hind paw with graduated von Frey hairs which are calibrated by the grams of force required to bend them. Rats with sciatic nerve ligation respond to lower grams of mechanical stimulation by reflexive withdrawal of the foot than unoperated rats. This response to stimuli which are normally innocuous is termed allodynia. Increases in the grams of mechanical force required to produce foot withdrawal after treatment is demonstrative of antiallodynic activity and a decrease in neuropathic pain.

## Example 22: TNFα Inhibitor in Animal Model of Neuropathic Pain

*Study of TNF antibody in rat segmental spinal nerve ligation model*

The following study is performed using the rat segmental spinal nerve ligation model for neuropathic pain (Kim and Chung, Pain 50 (1992) 355-363.). Male Sprague-Dawley rats, weighing 120-150 grams, are anesthetized, and placed in a prone position. The left paraspinal muscles are separated from the spinous processes at the $L_4$ - $S_2$ levels. The left L5 and L6 nerve roots are exposed and tightly ligated with 6-0 surgical silk suture distal to the dorsal root ganglion. Rats are allowed to recover and are administered doses of either a placebo or a monoclonal anti-TNFα antibody which is known to bind and neutralize rat TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). The experimental groups receive daily subcutaneous injections per week of TNF antibody or a placebo. Baseline behavioral measurements (response to mechanical allodynia and heat hyperalgesia testing, as described above) are made prior to surgery. Following the surgery, mechanical allodynia and analgesia testing for neuropathic pain are performed on a weekly basis for 10 weeks.

**Example 23: TNFα Inhibitor in Treatment of Neuropathic Pain**

*Study examining D2E7 in human subjects with neuropathicpain*

Patients diagnosed with neuropathic pain are selected for the study. Clinical neuropathic pain is determined based on clinical grounds, including history, physical examination and appropriate investigation of symptoms and signs expressed by the patient. The definitions of diagnostic criteria defined in the International Association for the Study of Pain (IASP) Classification of Chronic Pain are used to support the clinical diagnosis of neuropathic pain. Patients are excluded based on criteria including, but not limited to, another pain problem of equal or greater severity that might impair the assessment of neuropathic pain; significant neurological or psychiatric disorders unrelated to causes of neuropathic pain which might impair the assessment of neuropathic pain; current drug or alcohol abuse; and clinically significant liver, renal or pulmonary disease.

Evaluations of patient neuropathic pain are made using standard pain assessment tools such as the Short Form-McGill Pain Questionnaire (SF-MPQ); a 100-mm vertical Visual Analog Scale (VAS) (0 = no pain, 100 = intolerable pain); and the Clinician Global Impression of Change (CGIC). Patient's may also use a daily diary to score their neuropathic pain. Each evening, patients rate the average intensity of their pain during the preceding 24 hours.

Following a week of baseline measurements, patients begin receiving treatment. They are randomized and treated with either D2E7 or placebo in a blinded fashion. Patients are monitored every two weeks, and examined for a reduction in the patient's neuropathic pain assessment and average intensity of pain, as charted in their daily diaries.

**Example 24: TNFα Inhibitor in Animal Model for Hepatitis C Infection**

*Study of D2E7 in HCV chimpanzee model*

The following study is performed using the chimpanzee hepatitis C virus (HCV) model (Shimizu et al. (1990) Proc. Natl. Acad Sci. USA 87:6441).

Chimpanzees are inoculated intravenously (i.v.) with 0.5ml of undiluted plasma obtained from a patient with posttransfusion acute non-A, non-B hepatitis. The inoculum contains, for example, $10^{6.5}$ chimpanzee 50% infectious doses per ml ($CID_{50}$/ml) of HCV. Serum samples and liver biopsy specimens are taken before inoculation and weekly after treatment. After inoculation, chimpanzees are administered doses of D2E7 or a placebo. D2E7 is effective at binding TNF in a high affinity manner across species, see U.S. Patent No. 6,258,562.

HCV levels following innoculation are monitored in a number of ways. Serum samples are regularly taken from the chimpanzees and assayed for alanine aminotransferase (ALT). The ALT assay is one of a group of tests known as liver function tests (or LFTs), and is used to monitor damage to the liver. Circulating antibody to HCV (anti-C100-3 antibody) is detected by the BCV antibody ELISA test system. In addition, HCV is detected in the serum samples, cDNA/PCR assays are performed as described in Weiner et al, (1990) Lancet 335;1. Frozen liver biopsy specimens are also tested for the cytoplasmic antigen by immunofluorescent staining with monoclonal antibody 48-1 according to the method described in Shimizu et al (1985) Proc. Natl. Acad. Sci. USA 82;2138. Treated chimpanzees are examined to determine if ALT levels and HCV serum levels are lower as compared to placebo treated chimpanzees.

**Example 25: TNFα Inhibitor in Human HCV Infection**

*Study of D2E7 in treating HCV in humans*

Men and women aged 18 to 70 years with compensated chronic HCV infection are selected. To qualify for the study, patients must test positive for anti-HCV (second-generation enzyme immunoassay) and HCV RNA by reverse transcription -polymerase chain reaction (RT-PCR). Patients also have a liver biopsy within a year of the study entry showing chronic hepatitis, and have elevated serum alanine transaminase (ALT) levels for at least 6 months before initiation of treatment. Entry leukocyte counts should be least 2,500/$\mu$L; the platelet counts should be greater than 70,000/$\mu$L. Exclusion criteria include but are not limited to any other cause of liver disease or other relevant disorders, including human immunodeficiency or hepatitis B virus coinfection; clinically significant cardiac or cardiovascular abnormalities, organ grafts, systemic bacterial or fungal infection; clinically significant bleeding disorders; alcohol or drug abuse within the previous year.

Pretreatment and post-treatment serum HCV RNA is quantified by a standardized RT-PCR assay. Qualitative detection of HCV RNA is performed by RT-PCR in serum samples obtained post treatment. Genotyping of HCV is performed by reverse hybridization assay. Emotional and psychological states are measured using suitable health-related quality of life scales.

Following baseline measurements, patients begin receiving treatment. They are randomized and treated with either D2E7 or placebo in a blinded fashion. Patients are administered 40 mg of D2E7 in a biweekly dosing regiment, although this dose and the frequency of the dose can be adjusted by an ordinarily skilled artisan with knowledge of HCV treatments. Patients are monitored at least every 4 weeks, with repeated assays like those which were performed prior to the initiation of the D2E7 treatment A decrease in HCV levels relative to those who received only placebo is evidenced by a weaker RT-PCR signal.

**Example 26: TNFα Inhibitor in Mouse Model for Psoriasis**

*Study of TNF antibody in SCID mouse model of psoriasis*

Severe Combined Immunodeficient (SCID) mice that have undergone transplantation of human psoriasis plaques are selected as an animal model to study psoriasis because these mice retain the typical clinical and histological features of psoriasis for a prolonged period (Nickoloff et al. (1995) Am J Pathol 146:580-8).

2-3 month old female out bred C.B17 SCID mice are obtained from a pathogen-free animal breeding facility. Human skin specimens are taken from white male patients with chronic plaque psoriasis. The spindle-shaped skin specimens 1 x 3 cm inches in size comprising clinically involved skin are obtained under local anesthesia and are prepared for transplantation by removing subcutaneous fat, held in cooled phosphate-buffered saline (PBS). Skin specimens are grafted within 1-2 hours.

The full-thickness skin specimens are dissected into pieces 8-10 mm in diameter and are then transplanted on to the back of the mice, each mouse carrying one transplant. For the surgical procedure, mice are anesthetized by intraperitoneal injection (i.p.) of a 1:1 mixture of midazolam and fentanyl dihydrogen citrate. A spindle-shaped piece of full thickness skin is grafted onto a corresponding excisional full thickness defect of the shaved central dorsum and is fixed by 6-0 atraumatic monofilament sutures. After a sterile Vaseline impregnated gauze is applied, the grant is protected from injury by suturing a skin pouch over the transplanted area using the adjacent lateral skin. The sutures and over-tied pouch are left in place until they resolve spontaneously after 2-3 weeks.

The SCID-human skin chimeras exhibit symptoms similar to human psoriasis. A transplanted plaque on the SCID mouse shows clinical features typical of psoriasis including scales, erythema, and thickening. This model also exhibits histological features typical of psoriasis including parakeratosis, acanthosis, elongated rete ridges, supra-papillary thinning, and lymphomononuclear infiltrates in the papillary dermis.

Transplanted SCID mice are are injected subcutaneously at the site of the lesion with either a placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). The experimental groups receive daily subcutaneous injections per week of TNF antibody or a placebo. Improvement in the TNF antibody treated SCID mice is evidenced by a reduction in the symptoms associated with the psoriasis plaques.

**Examples 27: TNFα Inhibitor in Clinical Studies for Psoriasis**

*D2E7 in human subjects with psoriasis*

Patients with moderate to severe chronic plaque psoriasis are selected for the study. None of the patients will have received any psoriasis treatments for at least 4 weeks or any topical treatments for at least 2 weeks before study entry. Doses of D2E7 begin at 40 mg weekly or 40 mg every other week administered by subcutaneous injection.

Patients are examined clinically every 2-4 weeks. Clinical activity of psoriatic skin lesions is evaluated by means of the Psoriasis Area and Severity Index (PASI) (Fredriksson and Pettersson (1978) Dermatologica 157:238-44) and the Physician's Global Assessment by the same investigator to ensure consistent evaluations. At week 12, the primary end point of proportion of patients achieving at least 75% reduction in PASI score compared to baseline is determined. Pruritus is assessed by using a validated scale. Quality of life assessments are measured using validated instruments, including, but not limited to the DLQI, SF-36, and EQ-5D. Full-body photographs excluding the face are taken at scheduled visits throughout the study.

Skin biopsy specimens are obtained at scheduled intervals during the tudy to correlate histology and biomarkers in the skin with treatment. A biopsy of normal skin is obtained at baseline for comparison with psoriatic skin.

**Example 28. TNFα Inhibitor In Animal Model for Behcet's Disease**

*Study of TNF antibody in Behcet's syndrome mouse model*

The following study is perfumed using the mouse HSV model of Behcet's disease (Hirata, Y., et al. (1993) Acta. Otolaryngol. Suppl. 503:79). Earlobes of mice which express human TNFα (see EMBO J (1991) 10:4025-4031 for further description) are scratched with a needle, then inoculated with 1.0 x 1.0[6] plaque-forming units (pfu)/mL of Herpes Simplex Virus type 1 (HSV1) (KOS strain) solution, which causes inflammatory cells to accumulate in and around the blood vessels. As a result, intestinal, oral, ear lobe, and genital epithelial lesions occur. A mouse with Behcet's disease-like syndrome is defined as a mouse with two or more symptoms, which are similar to the typical morphological changes seen in human Behcet's disease.

A monoclonal anti-TNFα antibody which is known to bind and neutralize mouse TNFα, *e.g.*, antibody TN3 (TN3-19.12) (see Marzi et al. (1995) Shock 3:27; Williams et al. (1992) Proc Natl Acad Sci U S A. 89:9784; BD Biosciences Pharmingen) is administered to the HSV-induced Behcet's syndrome mice in a range of doses both before and after inoculation, or from the day of lesion occurrence. Appropriate placebo controls are also administered. Hair loss, ulceration of the mouth and genital skin, and eye involvement is monitored, and tissue samples are collected from lesions. Tissue samples are formalin-fixed and paraffin-embedded for sectional analysis. Lesion sections are stained with hematoxylin and eosin and examined for the appearance of inflammatory cell. As a control, 30 mice are inoculated at the same site with a culture medium. Four weeks later, a second inoculation is performed using the same method, followed by 16 weeks of observation.

Mice are examined for hair regrowth and a decrease in ulcerations in the treated mice as compared to placebo treated mice. Improvements in lesions in treated mice, as determined through visual inspection and histological analysis, noting a decrease in inflammation at the site of the ulceration and a decrease in the number of inflammatory cells, *e.g.,* T cells, at the site of the ulceration also are further indications of improvements.

**Example 29. TNFα Inhibitor In Treating Kawasaki's Disease**

*Effect of TNF antibody in Kawasaki's Disease using* L. casei *mouse model*

Using the mouse *L. casei* model of Kawasaki's disease (Lehman, T.J., et al. (1985) Arthritis Rheum 28:652; Duong, T.T. (2002) Int Immunol 15:79; Brahn, E., et al. (1999) Clin Immunol 90:147), the following study is performed. Coronary arteritis is induced in mice expressing human TNFα (see above) with a single intraperitoneal (ip) injection of *Lactobacillus casei* cell fragments. It has been shown that histologic sections of the hearts of mice treated with *L. casei,* resemble the vasculitis and aneurysms observed in the medium-sized coronary arteries of children with Kawasaki disease (Lehman et al. (1985) Arthritis Rheum 28:652; Duong (2002) Int Immunol 15:79; Brahn et al. (1999) Clin Immunol 90:147).

*L. casei* injected mice are administered a either control placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen) intraperitoneally through standard protocols. Hearts from injected mice are harvested on day 14 (early disease) or at the end of the study (established disease). Histologic sections are scored blindly for vasculitis.

A decrease in coronary arteritis is assessed by determining a reduction in inflammatory lesions of the coronary vessel wall of TNF antibody treated mice as compared to placebo treated animals. A decrease in coronary arteritis is assessed as a reduction in inflammatory mononuclear cell infiltrate of the coronary vessel wall accompanied by a reduction in intimal proliferation and less narrowing of the vessel lumen as compared to placebo treated animals.

**Examples 30. TNFα Inhibitor In Animal Model for Kawasaki's Disease**

*TNF antibody in Kawasaki's Disease using ANCA Mouse Model*

The following study is performed using the mouse anti-endothelial cell antibodies (ANCA) model of Kawasaki's Disease (Grunebaum et al. (2002) Clin. Exp. Immunol. 130:233; Blank et al. (1995) Clin. Exp. Immunol. 102:120; Tomer et al. (1995) Arthritis Rheum. 38:1375; Damianovich et al. (1996) J. Immunol. 156:4946). Animals are immunized with anti-endothelial cell antibodies (ANCA) containing proteinase 3-specific antibodies derived from a Wegenez's granulomatosis patient's plasma Mice are immunized with purified ANCA and control mice are injected with normal IgG. Mice are administered weekly doses of either a placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen) for upto four months. Three months after the immunization with the human ANCA, mice develop endogenous antibodies to ANCA. Mice are euthanized ,and lungs, kidneys, and heart are examined histologically for lymphoid cell infiltration surrounding arterioles and venules, as well as deposition of Igs at the outer part of blood vessel walls like that observed in patients with Kawasaki's Disease (Grunebaum et al. (2002) Clin. Exp. Immunol. 130:233; Blank et al. (1995) Clin. Exp. Immunol. 102:120; Tomer et al. (i995) Arthritis Rheum. 38:1375; Damianovich et al. (1996) J. Immunol. 156:4946). A decrease in lymphoid cell infiltration and IgG deposition in vessel walls and a decrease in antibody titre of ANCA is indicative of an improvement in Kawasaki's disease.

**Examples 31. TNFα Inhibitor in Treatment of Kawasaki's Disease**

*Clinical study of D2E7 in human subjects with Kawasaki's disease*

Patients suffering from Kawasaki's Disease (KD) are enrolled into the study; all patients have fever and at least 4 of the 5 clinical criteria published for KD (Barron, K.S., et al. (1999) J. Rheumatol 26:170). Case-controls are also identified. The diameter of the coronary arteries is measured by echocardiography and corrected for body surface area. Electrocardiograms are screened for typical changes that may be present in KD including prolonged PR or QT interval, abnormal Q waves, ST- and T-wave changes, low voltages or arrhythmias. KD patients are administered either D2E7 in biweekly and weekly doses of 40 mg or a placebo. Dosages may be adjusted by an ordinarily skilled artisan knowledgeable in KD. Patients are monitored for fever reduction. Adjuvant therapy, e.g, corticosteroids, are administered as needed. Patients are monitored and follow-up echocardiography is used to determine if coronary artery damage has occurred or whether an improvement in coronary artery lesions, demonstrated through improved echocardiogram results has occurred.

**Example 32. TNFα Inhibitor in Treatment of Behcet's Disease**

*Clinical Study of D2E7 in Human Subjects With Behcet's Disease*

Patients for the study are selected because they fulfill International Study Group criteria, which requires the presence of oral ulceration plus any two of genital ulceration, typical defined eye lesions, typical defined skin lesions, or a positive pathergy test (Lancet. (1990) 335:1078; Kaklamani, V.G. et al. (2001) Semin. Arthritis Rheum. 30:299) for a mean of 6 years. Behcet's patients are administered either D2B7 in biweekly and weekly doses of 40 mg or a placebo. Dosages may be adjusted by an ordinarily skilled artisan knowledgeable in Behcet's disease. Treated and placebo patients are given a systemic examination and detailed ophthalmological assessment, including visual acuity, measurement of intraocular pressure, slit-lamp biomicroscopy, and indirect ophthalmoscopy of the posterior segment followed by fundus photography, both before and following the treatment regime. Patients are examined for an improvement in the documented symptoms associated with Behcet's disease, e.g., reduction in eye inflammation and reduction in number or severity of mouth ulcers.

**Example 33: TNFα Inhibitor in Animal Model for Lupus**

*Study of TNF antibody in mouse lupus model*

The MRL/lpr mouse model is chosen to study lupus (Reilly and Gilkeson (2002) Immunologic Research. 25(2):143-153; Mishra et al. (2003) J Clin Invest. 111(4):539-552). MRL/lpr mice exhibit the onset of an accelerated autoimmune syndrome with polyclonal B cell activation and hypergammaglobulinemia beginning at about 8 weeks of age. In MRL/lpr mice, there is serologic evidence of an array of autoantibodies, including anti-double- stranded DNA (anti-dsDNA) autoantibodies and hypocomplementemia by 12-16 weeks of age. MRL/lpr mice exhibit clinical signs of arthritis, massive lymphadenopathy, splenomegaly, vasculitis, and glomerulonephritis (GN) by the age of 16-24 weeks. Approximately 50% of MRL/lpr mice die by 24 weeks of age, primarily from renal failure.

Eight week old female MRL/lpr mice are used in this study. At fourteen weeks, MRL/lpr mice are injected intraperitoneally (i.p.) with either varying concentrations of a placebo or Rats are allowed to recover and are administered doses of either a placebo or a monoclonal anti-TNFα antibody which is known to bind and neutralize mouse TNFα, *e.g.,* antibody TN3 (TN3-19.12) (see Marzi et al. (1995) Shock 3:27; Williams et al. (1992) Proc Natl Acad Sci U S A. 89:9784; BD Biosciences Pharmingen). The experimental groups receive daily subcutaneous injections per week of TNF antibody or a placebo.

Some patients with lupus develop lupus nephritis which is defined by persistent inflammation (irritation and swelling) in the kidney. These patients may eventually develop renal failure and require dialysis or kidney transplantation. To examine the progression of renal disease, MRL/lpr mice are placed in metabolic cages for 24-hour urine collections after injection with D2E7. Urinary albumin excretion is determined pre and post treatment with D2E7 by ELISA using a standard curve of known concentrations of mouse albumin (Cappel Research products, Durham, North Carolina, USA, as described in Weinberg et al. (1994) J Exp Med. 179:651). Improvements in early disease manifestations and progression of proteinuria are evidenced by a decrease in mean albumin excretion after treatment.

Mice are sacrificed at week 19 by cervical dislocation after isoflurane anesthesia and the kidneys are removed. One kidney is fixed with buffered formalin, embedded in paraffin, sectioned and is stained with H&E. Renal pathology is examined and graded by standard methods for glomerular inflammation, proliferation, crescent formation, and necrosis. Interstitial changes and vasculitis are also noted. Scores from 0 to 3 are assigned or each of the features, and then added together to yield a final renal score, as described by Watson et al. (1992) J Exp Med. 176:1645-1656. Scores for necrosis and crescent formation are doubled prior to adding. For example, glomerular inflammation is graded as follows: 0, normal; 1, few inflammatory cells; 2, moderate inflammation; and 3, severe inflammation. Improvements are evidenced by minimal signs of inflammation or cellular proliferation (a lower renal pathology index) in the kidney section from the D2E7 treated mouse when compared to the placebo treated mouse.

Spleen weight is also measured to determine the delay or prevention of the progression of lupus activity in the mice. Spleen size is an indicator of lupus activity that reflects the underlying immunopathology of the disease. MRL/lpr mice develop massive splenomegaly and lymphadenopathy with disease progression. To determine spleen size, at age 19 weeks, mice animals from each group (treatment and placebo) are sacrificed and the mean spleen weights determined. A lower mean spleen weight indicates an improvement in lupus.

## Example 34: TNFα Inhibitor Treatment for Lupus

*Study examining D2E7 in human subjects with lupus*

Patients with diagnosed lupus are selected for the study based. Patients are selected based on their presentation of symptoms commonly associated with lupus including fever, fatigue, general discomfort, uneasiness or ill feeling (malaise), weight loss, skin rash, "butterfly" rash, sunlight aggravates skin rash, sensitivity to sunlight, joint pain and swelling, arthritis, swollen glands, muscle aches, nausea and vomiting, pleuritic chest pain, seizures, and psychosis. Additional symptoms include blood in the urine, coughing up blood, nosebleed, swallowing difficulty, skin color is patchy, red spots on skin, fingers that change color upon pressure or in the cold (Raynaud's phenomenon), numbness and tingling, mouth sores, hair loss, abdominal pain and visual disturbance. Patients are given a physical examination to determine whether or not they exhibit any of the characteristic symptoms indicative of lupus. The diagnosis of lupus is based upon the presence of at least four out of eleven typical characteristics of the disease.

Tests to determine the presence of these disease manifestations may vary but will include some of the following: antinuclear antibody (ANA) panel including anti-DNA and anti-Smith antibodies, with the latter two tests generally positive in lupus alone; characteristic skin rash or lesions; chest X-ray showing pleuritis or pericarditis; listening to the chest with a stethoscope to reveal heart friction rub or pleural friction rub; urinalysis to show blood, casts, or protein in the urine; a complete blood cell count showing a decrease in some cell types; kidney biopsy; and neurological examination. This disease may also alter the results of the following tests: WBC count; serum globulin electrophoresis; rheumatoid factor; protein, urine; protein electrophoresis - serum; mononucleosis spot test; erythrocyte sedimentation rate (ESR); cryoglobulins; direct Coombs' test; complement component 3 (C3); complement; antithyroid microsomal antibody; antithyroglobulin antibody; antimitochondrial antibody; and anti-smooth muscle antibody.

Patients are randomly divided into experimental and placebo groups, and are administered either D2B7 or the placebo. Dosage ranges are used in the study to determine what dose is most effective for treating lupus. Dosages should begin at 40 mg, which is the D2E7 dose which has been found to be most effective at treating rheumatoid arthritis in patients. Patients are given 4 to 7 infusions of either D2E7 or placebo. Patients are re-examined every other week to determine if lupus symptoms are reduced or treated, determined by a reduction in the ESR and C-reactive protein (CRP) levels.

## Examples 35: TNFα Inhibitor on Sjögren's Syndrome

*Study examining D2E7 in human subjects with Sjögren's syndrome.*

Patients who meet the European and the American College of Rheumatology classification for primary Sjogren's disease are selected for the study (see Vitali et a. (1993) Arthritis Rheum 36:340-7; Fox et al. (1986) Arthritis Rheum. 29:577-85). Patients are at least 18 years old. At the time of enrollment all patients have active primary Sjogren's disease which is defined as the presence at screening of at least an elevated erythrocyte sedimentation rate (ESR; >25/mm/hr) or hypergammaglobulinemia (>1.4 gm/liter). Disease-modifying antirheumatic drugs (DMARDs) and corticosteroids are not allowed during the study and are discontinued at least 4 weeks before baseline. Exclusion criteria include serious infection in the previous 3 months, latent tuberculosis, documented human immunodeficiency virus or hepatitis C virus infection, life threatening vasculitis, known malignancy, concomitant severe or uncontrolled disease, and the presence of any other connective tissue disease.

The study includes administering 3 infusions of D2B7 (at a dosage of about 40 mg) at weeks 0,2, and 6 and 2 follow-up visits at weeks 10 and 14. Patients are allowed to continue artificial tears, provided that the dosage and schedule are stable throughout the study.

Clinical, ophthalmologic, and biologic evaluations are performed at baseline and at weeks 2, 4, 5, 10, and 14. Clinical assessments are performed by the same physician. These include a general physical examination, a dry mouth evaluation (using a scale of 0-2 where 0= no dryness, 1=mild-to moderate dryness, and 2=severe dryness), and a speech test (number of times the word "puttica" can be repeated during a 2-minute period, a technique presented by P.J. Shirlaw at the conference on New Advances in Basic Science, Diagnosis and Treatment of Sjögren's Syndrome, London, January 1997). In addition, unstimulated whole saliva is collected for 5 minutes using the spitting technique according to established methods, and samples are weighed on an analytical balance to determine the volume of saliva obtained (1 gm=1ml) (Navazesh (1993) Ann NY Acad Sci 694:72-7). A dry eye evaluation is also performed (scored on a scale of 0-2, where 0=no symptoms, 1=mild-to-moderate symptoms relieved by artificial tears (ATs), and 2= severe symptoms unrelieved by ATs), and the frequency of use of ATs is determined.

Patients are also given a fatigue evaluation (0-100 mm visual analog scale (VAS)) and answer a fatigue questionnaire (0=no fatigue, 1 = mild fatigue not interfering with daily activities, 2= moderate fatigue that interferes with daily activities, and 3 =fatigue with severely reduced activities). The clinical assessment may also include a tender joint count (maximum 64), tender point count (maximum 18), and patient's assessment of pain (0-100-mm VAS). Patient's and physician's global assessments were made using a 0-100 mm VAS.

All ophthalmologic assessments are performed by the same physician and include a fluorescein tear film breakup time (TBUT) test, the Schirmer I test, and a corneal evaluation performed by lissamine green staining (van Bijsterveld score of 0-9). Biologic parameters are measured through out the study and include the ESR, C-reactive protein level (CRP), complete blood cell count, renal and liver function tests, creating phosphokinase levels, serum levels of IgA, IgM, IgG, antinuclear antibodies (ANA), and rheumatoid factor(RF, and lymphocyte typing (numbers of CD4= and CD8= cells). Diminishment in the symptoms associated with Sjögren's syndrome symptoms include reduction in the tender points and pain in the peripheral joints.

## Example 36: TNFα Inhibitor on Juvenile Rheumatoid Arthritis

*Study examining D2E7 in children with juvenile rheumatoid arthritis*

Patients with diagnosed juvenile rheumatoid arthritis (JRA) are selected for the study. Patients receive D2E7 for 16 weeks and are then randomly divided into experimental and placebo groups. Patients are then administered either D2E7 or the placebo. Patients are administered a dosage range of between about 20 mg/m$^2$ /BSA (Body surface area) to a maximum of 40 mg every other week. Patients are given subcutaneous injections of either D2E7 or placebo on every other week for the duration of the treatment. Patients are re-examined every other week to determine if the symptoms of JRA are reduced or treated. Improvements in JRA are determined by a decrease in the clinical symptoms of the disease. Improvement in JRA is determing using criteria defined by Giannini (Giannini et al. (1997) Arthritis & Rheumatism 40:1202). Using this criteria, the definition of improvement is at least a 30% improvement from baseline in 3 of any 6 variables in the core set, with no more than 1 of the remaining variables worsening by >30%. The variables in the core set consist of physician global assessment of disease activity, parent/patient assessment of overall well-being (each scored on a 10-cm Visual Analog Scale), functional ability, number of joints with active arthritis, number of joints with limited range of motion, and erythrocyte sedimentation rate.

## Example 37: Crystallization of D2E7 F(ab)'$_2$ fragment

*Generation and purification of the D2E7 F(ab)'$_2$ Fragment*

A D2E7 F(ab)'$_2$ fragment was generated and purified according to the following procedure. Two ml of D2E7 IgG (approximately 63 mg/ml) was dialyzed against 1 liter of Buffer A (20 mM NaOAc, pH 4) overnight. After dialysis, the protein was diluted to a concentration of 20 mg/ml. Immobilized pepsin (Pierce; 6.7 ml of slurry) was mixed with 27 ml of Buffer A, mixed, and centrifuged (Beckman floor centrifuge, 5000 rpm, 10 min). The supernatant was removed, and this washing procedure was repeated twice more. The washed immobilized pepsin was re-suspended in 13.3 ml of Buffer A. D2E7 (7.275 ml, 20 mg/ml, 145.5 mg) was mixed with 7.725 ml of Buffer A Bnd 7.5 ml of the washed immobilized pepsin slurry. The D2E7/pepsin mixture was incubated at 37 °C for 4.5 hr with shaking (300 rpm). The immobilized pepsin was then separated by centrifugation. Analysis of the supernatant by SDS-PAGE indicated that the digestion of D2E7 was essentially complete (~115 kDa band unreduced, ~30 and ~32 kDa bands reduced).

The D2E7 F(ab)'$_2$ fragment was separated from intact D2E7 and Fc fragments using Protein A chromatography. One-half of the above reaction supernatant (10 ml) was diluted with 10 ml of Buffer B (20 mM Na phosphate, pH 7), filtered through a 0.45 μm Acrodisk filter, and loaded onto a 5 ml Protein A Sepharose column (Pharmacia.Hi-Trap; previously washed with 50 ml of Buffer B). Fractions were collected. After the protein mixture was loaded, the column was washed with Buffer B until the absorbance at 280 nm re-established a baseline. Bound proteins were eluted with 5 ml of Buffer C (100 mM citric acid, pH 3); these fractions were neutralized by adding 0.2 ml of 2 M Tris•HCl, pH 8.9. Fractions were analyzed by SDS-PAGE; those that contained the D2E7 F(ab)'$_2$ fragment were pooled (~42 ml). Protein concentrations were determined by absorbance at 280 nm in 6 M guanidine•HCl, pH 7 (calculated extinction coefficients: D2E7, 1.39 (AU-ml)/mg; F(ab)'$_2$, 1.36 (AU-ml)/mg). The flow-though pool contained ~38.2 mg protein (concentration, 0.91 mg/ml), which represents a 79% yield of F(ab)'$_2$ (theoretical yield is ~2/3 of starting material, divided by two [only half purified], i.e. ~48.5 mg).

The D2E7 F(ab)'$_2$ fragment was further purified by size-exclusion chromatography. The pooled Protein A flow-through was concentrated from ~42 to ~20 ml, and a portion (5 ml, ~7.5 mg) was then chromatographed on a Superdex 200 column (26/60, Pharmacia) previously equilibrated (and eluted) with Buffer D (20 mM HEPES, pH 7, 150 mM NaCl, 0.1 mM EDTA). Two peaks were noted by absorbance at 280 nm: Peak 1, eluting at 172-200 ml, consisted of F(ab)'$_2$ (analysis by SDS-PAGE; ~115 kDa band unreduced, ~30 and -32 kDa bands reduced); Peak 2, eluting at 236-248 ml, consisted of low molecular weight fragment(s) (~15 kDa, reduced or unreduced). Peak 1 was concentrated to 5.3 mg/ml for crystallization trials.

*Crystallization of the D2E7 F(ab)'$_2$ Fragment*

The D2E7 F(ab)'$_2$ fragment (5.3 mg/ml in 20 mM HEPES, pH 7,150 mM NaCl, 0.1 mM EDTA) was crystallized using the sitting drop vapor diffusion method by mixing equal volumes of F(ab)'$_2$ and crystallization buffer (approx. 1 μl of each) and allowing the mixture to equilibrate against the crystallization Buffer Bt 4 or 18 °C. The crystallization buffers used consisted of the Hampton Research Crystal Screens I (solutions 1-48) and II (solutions 1-48), Emerald Biostructures Wizard Screens I and II (each solutions 1-48), and the Jena Biosciences screens 1-10 (each solutions 1-24). Crystals were obtained under many different conditions, as summarized in Table 1.

Table 1. Summary of crystallization conditions for the D2E7 F(ab)'$_2$ fragment.

| Screen | Solution | Temp °C | Condition | Result |
|---|---|---|---|---|
| Hampton 1 | 32 | 4 | 2.0 M $(NH_4)_2SO_4$ | tiny needle clusters |
| Hampton 1 | 46 | 4 | 0.2 M $Ca(Oac)_2$, 0.1 M Na cacodylate pH 6.5, 18% PBG8K | medium sized needle clusters |
| Hampton 1 | 48 | 4 | 0.1 M Tris HCl pH 8.5, 2.0 M $NH_4H_2PO_4$ | micro needle clusters |
| Hampton 2 | 2 | 4 | 0.01 M hexadecyltrimethylammonium bromide, 0.5 M NaCl, 0.01 M $MgCl_2$ | small shard crystals |
| Hampton 2 | 13 | 4 | 0.2 M $(NH_4)_2SO_4$, 0.1 M NaOAc pH 4.6, 30% PEG MME 2000 | small needle clusters |
| Hampton 2 | 15 | 4 | 0.5 M $(NH_4)_2SO_4$, 0.1MNaOAc pH 5.6, 1.0M $Li_2SO_4$ | large needle clusters |
| Hampton 2 | 16 | 4 | 0.5M NaCl, 0.1M NaOAc pH 5.6, 4% Ethylene Imine polymer | large irregular crystal |
| Hampton 1 | 34 | 18 | 0.1 NaOAc pH 4.6, 2.0 M Na Formate | needle clusters |
| Hampton 1 | 35 | 18 | 0.1M Hepes pH 7.5, 0.8M mono-sodium dihydrogen phosphate, 0.8M mono-potasium dihydrogen phosphate | needle clusters |
| Hampton 2 | 9 | 18 | 0.1M NaOAc pH 4.6, 2.0M NaCl | dense needle clusters |
| Hampton 2 | 12 | 18 | 0.1M $CdCl_2$, 0.1M NaOAc pH 4.6, 30% PEG 400 | needles & amorphous crystal |
| Hampton 2 | 15 | 18 | 0.5M $(NH_4)_2SO_4$, 0.1M NaOAc pH 5.6, 1.0M $Li_2SO_4$ | tiny needle clusters |
| Wizard I | 27 | 4 | 1.2M NaH2PO4, 0.8M K2HPO4, 0.1M CAPS pH 10.5, 0.2 M $Li_2SO_4$ | Medium large needle clusters |

(continued)

| Screen | Solution | Temp °C | Condition | Result |
|--------|----------|---------|-----------|--------|
| Wizard I | 30 | 4 | 1.26M $(NH_4)_2SO_4$, 0.1 M NaOAc pH 4.5,0.2M NaCl | small needle clusters |
| Wizard II | 8 | 4 | 10% PEG 8K, 0.1M Na/K phosphate pH 6.2, 0.2M NaCl | Large plate crystals grown in clusters |
| Wizard H | 43 | 4 | 10% PEK 8K, 0.1M Tris pH 7.0, 0.2 M MgCl2 | micro needle clusters |
| Wizard I | 4 | 18 | 35% MPD, 0.1M Imidazole pH 8.0, 0.2M MgCl2 | rod shaped crystal |
| Wizard I | 27 | 18 | 1.2M NaH2PO4, 0.8M K2HPO4, 0.1M CAPS pH 10.5, 0.2 M $Li_2SO_4$ | Needle clusters |
| Wizard II | 7 | 18 | 30% PEG 3K, 0.1M Tris pH 8.5, 0.2M NaCl | tiny needle clusters |
| Wizard II | 11 | 18 | 10% 2-propanol, 0.1M cacodylate pH 6.5, 0.2M Zn(Oac)2 | tiny hexagonal or rhombohedral crystal |
| Wizard II | 46 | 18 | 1.0M AP, 0.1M Imidazole pH 8.0, 0.2M NaCl | 1 irregular crystal |
| JB 1 | D6 | 4 | 30% PEG 3K, 0.1M Tris HCl pH 8.5, 0.2M $Li_2SO_4$ | tiny needles in precipitate |
| JB 2 | B6 | 4 | 20% PEG 4K, 0.1M Tris HCl pH 8.5, 0.2M Na Cacodylate | tiny needle cluster balls |
| JB 3 | A1 | 4 | 8% PEG 4K, 0.8M LiCl, 0.1M Tris HCl pH 8.5 | Large frost-like crystals |
| JB 3 | B1 | 4 | 15% PEG 4K, 0.2M $(NH_4)_2SO_4$ | tiny needle clusters |
| JB 3 | D5 | 4 | 30% PEG 4K, 0.1M Na Citrate pH 5.6,0.2M $NH_4OAc$ | tiny needles in precipitate. |
| JB 4 | B1 | 4 | 15% PEG 6K, 0.05M KCl, 0.01M $MgCl_2$ | needle cluster balls |
| JB 3 | A6 | 18 | 12% PEG 4K, 0.1M NaOAc pH 4.6, 0.2M $NH_4OAc$ | needle clusters |
| JB 3 | B1 | 18 | 15% PEG 4K, 0.2M $(NH_4)_2SO_4$ | needle clusters in precipitate |
| JB 3 | C6 | 18 | 25% PEG 4K, 0.1M Na Citrate pH 5.6, 0.2M $NH_4OAc$ | long, thin needles |
| JB 4 | C5 | 18 | 8% PEG 8K, 0.2 M LiCl, 0.05M MgSO4 | frost-like crystals |
| JB 5 | A3 | 4 | 15% PEG 8K, 0.2M $(NH_4)_2SO_4$ | long single needles in phase separation |
| JB 5 | A4 | 4 | 15% PEG 8K, 0.5M $Li_2SO_4$ | tiny needle clusters |
| JB 5 | A5 | 4 | 15% PEG 8K, 0.1M Na MES pH 6.5, 0.2M $Ca(OAc)_2$ | needle cluster balls |
| JB 6 | B2 | 4 | 1.6M $(NH_4)_2SO_4$, 0.5 LiCl | tiny needle cluster balls |
| JB 6 | C2 | 4 | 2.0 M$(NH_4)_2SO_4$, 0.1M NaOAc pH 4.6 | micro needle clusters |
| JB 10 | D3 | 18 | 2.0M Na Formate, 0.1M NaOAc pH 4.6 | needle clusters |

The following conditions (as described in Table 1) produced crystals which can be used for diffraction quality crystals: Wizard II, 11, 8, 10% 2-propanol, 0.1M cacodylate pH 6.5, 0.2M Zn(Oac)2, tiny hexagonal or rhom. Xtals; Wizard II, 10% PEG 8K, 0.1M Na/K phosphate pH 6.2, 0.2M NaCl, large plate xtals grown in clusters; JB 3, C6, 18, 25% PEG 4K, 0.1M Na Citrate pH 5.6, 0.2M Ammonium Acetate, long, thin needles; Hampton 2, 15, 18, 0.5M AS, 0.1M Na Acetate trihydrate pH 5.6, 1.0M Li Sulfate monohydrate, tiny needle clusters.

**Example 38: Crystallization of D2E7 Fab fragment**

*Generation and purification of the D2E7 Fab Fragment*

A D2E7 Fab fragment was generated and purified according to the following procedure. Four ml of D2E7 IgG (diluted to about 20 mg/ml) was diluted with 4 ml of Buffer E (20 mM Na phosphate, 5 mM cysteine•HCl, 10 mM EDTA, pH7) and mixed with 6.5 ml of a slurry of immobilized papain (Pierce, 1%; previously washed twice with 26 ml of Buffer E). The D2E7/papain mixture was incubated at 37 °C overnight with shaking (300 rpm). The immobilized papain and precipitated protein were separated by centrifugation; analysis of the supernatant by SDS-PAGE indicated that the digestion of D2E7 was partially complete (~55, 50, 34, and 30 kDa bands unreduced, with some intact and partially digested D2E7 at ~115 and ~150 kDa; ~30 and ~32 kDa bands reduced, as well as a ~50 kDa band). Nonetheless, the digestion was halted and subjected to purification.

The D2E7 Fab fragment was purified by Protein A chromatography and Superdex 200 size-exclusion chromatography essentially as described above for the F(ab)'$_2$ fragment. The Protein A column flow-through pool (21 ml) contained ~9.2 mg (0.44 mg/ml), whereas the Protein A eluate (4 ml) contained ~19.5 mg (4.9 mg/ml). Analysis by SDS-PAGE indicated that the flow-through was essentially pure Fab fragment (~48 and ~30 kDa unreduced, broad band at ~30 kDa reduced), whereas the eluate was intact and partially-digested D2E7. The Fab fragment was further purified on a Superdex 200 column, eluting at 216-232 ml, i.e., as expected, after the F(ab)'$_2$ fragment but before the small Fc fragments. The D2E7 Fab fragment concentrated to 12.7 mg/ml for crystallization trials, as described below.

*Crystallization of the D2E7 Fab Fragment*

The D2E7 Fab fragment (12.7 mg/ml in 20 mM HEPES, pH 7, 150 mM NaCl, 0.1 mM EDTA) was crystallized using the sitting drop vapor diffusion method essentially as described above for the F(ab)'$_2$ fragment. Crystals were obtained under many different conditions, as summarized in Table 2.

Table 2. Summary of crystallization conditions for the D2E7 Fab fragment

| Screen | Solution | Temp °C | Condition | Result |
|---|---|---|---|---|
| Hampton 1 | 4 | 4 | 0.1M Tris pH 8.5, 2M $(NH_4)_2SO_4$ | wispy needles |
| Hampton 1 | 10 | 4 | 0.2M $NH_4OAc$, 0.1M NaOAc pH 4.6, 30% PEG 4K | wispy needle clusters |
| Hampton 1 | 18 | 4 | 0.2M $Mg(OAc)_2$, 0.1M Na Cacodylate pH 6.5, 20% PEG 8K | needle clusters |
| Hampton 1 | 20 | 4 | 0.2M $(NH_4)_2SO_4$, 0.1M NaOAc pH 4.6, 25% PEG 4K | tiny needle clusters |
| Hampton 1 | 32 | 4 | 2M $(NH_4)_2SO_4$ | long, wispy needles |
| Hampton 1 | 33 | 4 | 4M Na Formate | tiny needle clusters |
| Hampton 1 | 38 | 4 | 0.1M Hepes pH 7.5 | tiny needle clusters |
| Hampton 1 | 43 | 4 | 30% PEG 1500 | tiny needle clusters |
| Hampton 1 | 46 | 4 | 0.2M $Ca(OAc)_2$, 0.1M Na Cacodylate pH 6.5, 18% PEG 8K | large plate clusters |
| Hampton 1 | 47 | 4 | 0.1M NaOAc pH 4.6, 2M $(NH_4)_2SO_4$ | long, wispy needles |
| Hampton 2 | 1 | 4 | 2M NaCl, 10% PEG 6K | small plate clusters |

(continued)

| Screen | Solution | Temp °C | Condition | Result |
|--------|----------|---------|-----------|--------|
| Hampton 2 | 2 | 4 | 0.01M Hexadecyltrimethylammonium bromide, 0.5M NaCl, 0.01 MgCl$_2$ | round & irregular plates |
| Hampton 2 | 5 | 4 | 2M (NH$_4$)$_2$SO$_4$, 5% isopropanol | long fiber ropes |
| Hampton 2 | 13 | 4 | 0.2M (NH$_4$)$_2$SO$_4$, 0.1M NaOAc pH 4.6, 25% PEG MME 2K | tiny, wispy needle clusters |
| Hampton 2 | 14 | 4 | 0.2M K/Na Tatrate, 0.1M Na Citrate pH 5.6, 2M (NH$_4$)$_2$SO$_4$ | tiny needle clusters |
| Hampton 2 | 27 | 4 | 0.01M ZuSO$_4$, 0.1 MES pH 6.5, 25% PEG MME 550 | tiny needle clusters |
| Hampton 2 | 28 | 4 | 30% MPD | tiny needle clusters |
| Hampton 1 | 4 | 18 | 0.1M Tris pH 8.5, 2M (NH$_4$)$_2$SO$_4$ | needle clusters |
| Hampton 1 | 9 | 18 | 0.2M NH$_4$OAc, 0.1M Na Citrate pH 5.6, 30% PEG 4K | needle clusters |
| Hampton 1 | 17 | 18 | 0.2M Li$_2$SO$_4$, 0.1M Tris pH 8.5, 30% PEG 4K | long, wispy needles |
| Hampton 1 | 32 | 18 | 2M (NH$_4$)$_2$SO$_4$ | needle clusters |
| Hampton 1 | 33 | 18 | 4M Na Formate | tiny needle clusters |
| Hampton 1 | 38 | 18 | 0.1M Hepes pH 7.5 | fiber bundles |
| Hampton 1 | 43 | 18 | 30% PEG 1500 | tiny needle clusters |
| Hampton 1 | 47 | 18 | 0.1M NaOAc pH 4.6, 2M (NH$_4$)$_2$SO$_4$ | tiny needle clusters |
| Hampton 2 | 1 | 18 | 2M NaCl, 10% PEG 6K | long, wispy needle clusters |
| Hampton 2 | 5 | 18 | 2M (NH$_4$)$_2$SO$_4$, 5% 2-propanol | tiny needle clusters |
| Hampton 2 | 9 | 18 | 0.1M NaOAc pH 4.6, 2M NaCl | long, wispy needles |
| Hampton 2 | 13 | 18 | 0.2M (NH$_4$)$_2$SO$_4$, 0.1M NaOAc pH 4.6, 25% PEG MME 2K | tiny needle clusters |
| Hampton 2 | 14 | 18 | 0.2M K/Na Tartrate, 0.1M Na Citrate pH 5.6, 2M (NH$_4$)$_2$SO$_4$ | long wispy needles |
| Hampton 2 | 27 | 18 | 0.01M ZnSO$_4$, 0.1 MES pH 6.5, 25% PEG MME 550 | tiny needle clusters |
| Wizard I | 20 | 4 | 0.4M NaH$_2$PO$_4$/1.6M K$_2$HPO$_4$, 0.1M Imidazole pH 8, 0.2M NaCl | tiny needle clusters |
| Wizard I | 28 | 4 | 20% PEG 3K, 0.1M Hepes pH 7.5, 0.2M NaCl | large orthorhombic plate clusters |

(continued)

| Screen | Solution | Temp °C | Condition | Result |
|---|---|---|---|---|
| Wizard I | 31 | 4 | 20% PEG 8K, 0.1M phosphate citrate pH 4.2, 0.2M NaCl | wispy needle clusters |
| Wizard I | 39 | 4 | 20% PEG 1K, 0.1M phosphate citrate pH 4.2, 0.2M Li$_2$SO$_4$ | needle clusters |
| Wizard II | 3 | 4 | 20% PEG 8K, 0.1M Tris pH 8.5, 0.2M MgCl$_2$ | large hexagonal or orthorhombic plate cluster in phase sep |
| Wizard II | 4 | 4 | 2M (NH$_4$)$_2$SO$_4$, 0.1M Cacodylate pH 6.5, 0.2 NaCl | tiny needle clusters |
| Wizard II | 9 | 4 | 2M (NH$_4$)$_2$SO$_4$, 0.1M phosphate citrate pH 4.2 | tiny, wispy needle clusters |
| Wizard II | 28 | 4 | 20% PEG 8K, 0.1M MES pH 6, 0.2M Ca(OAc)$_2$ | tiny needle clusters; large wispy needle clusters |
| Wizard II | 35 | 4 | 0.8M NaH$_2$PO$_4$/1.2M K$_2$HPO$_4$, 0.1M NaOAc pH 4.5 | tiny fiber bundles |
| Wizard II | 38 | 4 | 2.5M NaCl, 0.1M NaOAc pH 4.5, 0.2M Li$_2$SO$_4$ | long wispy needles |
| Wizard II | 47 | 4 | 2.5M NaCl, 0.1M Imidazole pH 8, 0.2M Zu(OAc)$_2$ | tiny needle clusters |
| Wizard I | 6 | 18 | 20% PEG 3K, 0.1M Citrate pH 5.5 | needle clusters |
| Wizard I | 20 | 18 | 0.4M NaH$_2$PO$_4$/1.6M K$_2$HPO$_4$, 0.1M Imidazole pH 8, 0.2M NaCl | tiny needle clusters |
| Wizard I | 27 | 18 | 1.2M NaH$_2$PO$_4$/0.8M K$_2$HPO$_4$, 0.1M CAPS pH 10, 0.2M Li$_2$SO$_4$ | wispy needle clusters |
| Wizard I | 30 | 18 | 1.26M (NH$_4$)$_2$SO$_4$, 0.1M NaOAc pH 4.5, 0.2M NaCl | wispy needles |
| Wizard I | 31 | 18 | 20% PEG 8K, 0.1M phosphate citrate pH 4.2, 0.2M NaCl | tiny needle clusters |
| Wizard I | 33 | 18 | 2M (NH$_4$)$_2$SO$_4$, 0.1M CAPS pH 10.5, 0.2M Li$_2$SO$_4$ | fiber bundles |
| Wizard I | 39 | 18 | 20% PEG 1K, 0.1M phosphate citrate pH 4.2, 0.2M Li$_2$SO$_4$ | needle clusters |
| Wizard II | 4 | 18 | 2M (NH$_4$)$_2$SO$_4$, 0.1M Cocodylate pH 6.5, 0.2 NaCl | needle clusters |
| Wizard II | 9 | 18 | 2M (NH$_4$)$_2$SO$_4$, 0.1M phosphate citrate pH 4.2 | wispy needles |
| Wizard II | 35 | 18 | 0.8M NaH$_2$PO$_4$/1.2M K$_2$HPO$_4$, 0.1M NaOAc pH 4.5 | tiny needle clusters |
| Wizard II | 38 | 18 | 2.5M NaCl, 0.1M NaOAc pH 4.5, 0.2M Li$_2$SO$_4$ | tiny needle clusters |

The following conditions (as described in Table 2) produced crystals which can be used for diffraction quality crystals: Hampton 2,1,4C, 2M NaCl, 10% PEG 6K, small plate clusters; Hampton 1 46, 4C, 0.2M Ca Acetate, 0.1M Na Cacodylate, pH 6.5, 18% PEG 8K, large plate clusters; Wizard I, 28, 4C, 20% PEG 3K, 0.1M Hepes pH 7.5, 0.2M NaCl, large orthorhombic plate clusters; Wizard II 3, 4C, 20% PEG 8K, 0.1M Tris pH 8.5, 0.2M MgCl$_2$, lrg hex or orth plate cluster in phase sep.

## EQUIVALENTS

Those skilled in the art will recognize, or be able to ascertain using no more than routine experimentation, many equivalents to the specific embodiments of the invention described herein. Such equivalents are intended to be encompassed by the following embodiments.

Embodiment A

The present invention provides a method of treating a TNFα-related disorder in a subject, wherein the TNFα-related disorder is selected from the group consisting of a spondyloarthropathy, a pulmonary disorder, a coronary disorder, a metabolic disorder, anemia, pain, a hepatic disorder, a skin disorder, a nail disorder, or vasculitis, comprising administering to the subject a therapeutically effective amount of a neutralizing, high affinity TNFα antibody, such that said TNFα-related disorder is treated.

Embodiment B

The present invention also provides a method of treating a TNFα-related disorder in a subject, wherein the TNFα-related disorder is selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis, such that said TNFα-related disorder is treated.

Embodiment C

The present invention further provides a method of treating a TNFα-related disorder in a subject, wherein the TNFα-related disorder is selected from the group consisting of age-related cachexia, Alzheimer's disease, brain edema, inflammatory brain injury, chronic fatigue syndrome, dermatomyositis, drug reactions, edema in and/or around the spinal cord, familial periodic fevers, Felty's syndrome, fibrosis, glomerulonephritides (e.g. post-streptococcal glomerulonephritis or IgA nephropathy), loosening of prostheses, microscopic polyangiitis, mixed connective tissue disorder, multiple myeloma, cancer and cachexia, multiple organ disorder, myelo dysplastic syndrome, orchitism osteolysis, pancreatitis, including acute, chronic, and pancreatic abscess, periodontal disease polymyositis, progressive renal failure, pseudogout, pyoderma gangrenosum, relapsing polychondritis, rheumatic heart disease, sarcoidosis, sclerosing cholangitis, stroke, thoracoabdominal aortic aneurysm repair (TAAA), TNF receptor associated periodic syndrome (TRAPS), symptoms related to Yellow Fever vaccination, inflammatory diseases associated with the ear, chronic ear inflammation, or pediatric ear inflammation. In still another embodiment of the invention, the TNFα-related disorder is a Crohn's disease-related disorder, juvenile arthritis/Still's disease (JRA), uveitis, sciatica, prostatitis, endometriosis, choroidal neovascularization, lupus, Sjogren's syndrome, and wet macular degeneration, such that said TNFα-related disorder is treated.

Preferred embodiments of Embodiment A, B or C

In a preferred method of Embodiment A, B or C, the antibody is an isolated human antibody, or an antigen-binding portion thereof, that dissociates from human TNFα with a $K_d$ of 1 x $10^{-8}$ M or less and a $K_{off}$ rate constant of 1 x $10^{-3}$ s$^{-1}$ or less, both determined by surface plasmon resonance, and neutralizes human TNFα cytotoxicity in a standard *in vitro* L929 assay with an $IC_{50}$ of 1 x $10^{-7}$ M or less.

In a preferred method of Embodiment A, B or C, the antibody is an isolated human antibody, or an antigen-binding portion thereof with the following characteristics:

a) dissociates from human TNFα with a $K_{off}$ rate constant of 1 x $10^{-3}$ s$^{-1}$ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7,8 and/or 9;
c) has a heavy chain CDR3 domain comprising the amino acid sequence of SEQ ID NO: 4, or modified from SEQ ID NO: 4 by a single alanine substitution at position 2, 3, 4, 5, 6, 8, 9, 10 or 11, or by one to five conservative amino acid substitutions at positions 2,3,4,5,6, 8, 9, 10, 11 and/or 12.

In a preferred method of Embodiment A, B or C, the antibody is an isolated human antibody, or an antigen-binding portion thereof, with a light chain variable region (LCVR) comprising the amino acid sequence of SEQ ID NO:1 and a heavy chain variable region (HCVR) comprising the amino acid sequence of SEQ ID NO: 2.

In a preferred method of Embodiment A, B or C, the antibody is D2E7.

Embodiment D

The present invention also provides a method of treating a subject suffering from a TNFα-related disorder, wherein the TNFα-related disorder is selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis, comprising administering a therapeutically effective amount of a TNFα antibody, or an antigen-binding fragment thereof, to the subject, wherein the antibody dissociates from human TNFα with a $K_d$ of 1 x $10^{-8}$ M or less and a $K_{off}$ rate constant of 1 x $10^{-3}$ $s^{-1}$ or less, both determined by surface plasmon resonance, and neutralizes human TNFα cytotoxicity in a standard *in vitro* L929 assay with an $IC_{50}$ of 1 x $10^{-7}$ M or less, such that said TNFα-related disorder is treated.

## Embodiment E

In addition, the present invention provides a method of treating a subject suffering from a TNFα-related disorder, wherein the TNFα-related disorder is selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis, comprising administering a therapeutically effective amount a TNFα antibody, or an antigen-binding fragment thereof, with the following characteristics:

a) dissociates from human TNFα with a $K_{off}$ rate constant of 1 x $10^{-3}$ $s^{-1}$ or less, as determined by surface plasmon resonance;
b) has a light chain CDR3 domain comprising the amino acid sequence of SEQ ID NO: 3, or modified from SEQ ID NO: 3 by a single alanine substitution at position 1, 4, 5, 7 or 8 or by one to five conservative amino acid substitutions at positions 1, 3, 4, 6, 7, 8 and/or 9;
c) has a heavy chain CDR3 domain comprising the amino acid sequence of SEQ ID NO: 4, or modified from SEQ ID NO: 4 by a single alanine substitution at position 2, 3, 4, 5, 5, 8, 9, 10 or 11 or by one to five conservative amino acid substitutions at positions 2, 3, 4, 5, 6, S, 9, 10, 11 and/or 12, such that said TNFα-related disorder is treated.

## Embodiment F

The present invention further provides a method of treating a subject suffering from a TNFα-related disorder selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis, comprising administering a therapeutically effective amount a TNFα antibody, or an antigen-binding fragment thereof, with a light chain variable region (LCVR) comprising the amino acid sequence of SEQ ID NO: 1 and a heavy chain variable region (HCVR) comprising the amino acid sequence of SEQ ID NO: 2, such that said TNFα-related disorder is treated.

## Preferred embodiments of Embodiment D, E or F

In a preferred method of Embodiment D, E or F, the TNFα antibody, or antigen binding fragment thereof, is D2E7.
In a preferred method of Embodiment D, E or F, the TNFα antibody is administered with at least one additional therapeutic agent.

## Embodiment G

The present invention additionally provides a method of treating a subject suffering from a TNFα-related disorder selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis,such that said TNFα-related disorder is treated.
In the method of Embodiment G, preferably D2E7 is administered with at least one additional therapeutic agent.

## Embodiment H - Kits

The present invention also provides a kit comprising:

a) a pharmaceutical composition comprising a TNFα antibody, or an antigen binding portion thereof, and a pharmaceutically acceptable carrier; and

b) instructions for administering to a subject the TNFα antibody pharmaceutical composition for treating a subject who is suffering from a TNFα-related disorder.

In reference to such kit, preferably the TNFα-related disorder selected from the group consisting of Behcet's disease, ankylosing spondylitis, asthma, chronic obstructive pulmonary disease (COPD), idiopathic pulmonary fibrosis (IPF), restenosis, diabetes, anemia, pain, a Crohn's disease-related disorder, juvenile rheumatoid arthritis (JRA), a hepatitis C virus infection, psoriasis, psoriatic arthritis, and chronic plaque psoriasis.

In reference to such preferred kits the TNFα antibody, or an antigen binding portion thereof, is D2E7.

## Claims

1.  Use of adalimumab for the manufacture of a medicament for the treatment of chronic plaque psoriasis, wherein adalimumab is administered in an amount of 40 mg.

2.  The use of claim 1, wherein adalimumab is administered parenterally.

3.  The use of claim 2, wherein adalimumab is administered by intramuscular or subcutaneous injection.

4.  The use of any one of claims 1 to 3, wherein adalimumab is administered with at least one additional therapeutic agent.

5.  The use of claim 4, wherein the at least one additional therapeutic agent is a topical corticosteroid, a vitamin D analog, a topical or oral retinoid, or a combination thereof.

6.  The use of any one of claims 1 to 3, wherein adalimumab is administered to a patient with moderate to severe chronic plaque psoriasis.

7.  Use of adalimumab for the manufacture of a medicament for the treatment of moderate to severe chronic plaque psoriasis in a patient, wherein a 40 mg dose of adalimumab is administered every other week to the patient by subcutaneous injection.

8.  The use of any one of claims 1 to 7, wherein 12 weeks of said treatment achieves at least a 75% reduction in Psoriasis Area and Severity Index (PASI) score.

**Amended claims in accordance with Rule 137(2) EPC.**

1. Use of adalimumab for the manufacture of a medicament for the treatment of moderate to severe chronic plaque psoriasis in a patient, wherein a 40 mg dose of adalimumab is administered every other week to the patient by subcutaneous injection.

2. The use of claim 1, wherein 12 weeks of said treatment achieves at least a 75% reduction in Psoriasis Area and Severity Index (PASI) score.

Europäisches
Patentamt

European
Patent Office

Office européen
des brevets

# EUROPEAN SEARCH REPORT

Application Number

EP 15 15 7149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 97/29131 A (BASF AG [DE]; SALFELD JOCHEN G [US]; ALLEN DEBORAH J [GB]; KAYMAKCALAN) 14 August 1997 (1997-08-14) * page 3, line 19; claims 1,9,15,45,46,61,62,64-73; examples 1-4 * | 1-8 | INV. C07K16/24 A61K39/395 |
| Y,D | TAKEMATSU H ET AL: "Absence of tumor necrosis factor-alpha in suction blister fluids and stratum corneum from patients with psoriasis.", ARCHIVES OF DERMATOLOGICAL RESEARCH 1989, vol. 281, no. 6, 1989, pages 398-400, XP002500126, ISSN: 0340-3696 * the whole document * | 1-8 | |
| Y,D | GROM A A ET AL: "Patterns of expression of tumor necrosis factor alpha, tumor necrosis factor beta, and their receptors in synovia of patients with juvenile rheumatoid arthritis and juvenile spondylarthropathy.", ARTHRITIS AND RHEUMATISM OCT 1996, vol. 39, no. 10, October 1996 (1996-10), pages 1703-1710, XP009107328, ISSN: 0004-3591 * the whole document * | 1-8 | |

TECHNICAL FIELDS
SEARCHED      (IPC)

C07K

-/--

The present search report has been drawn up for all claims

| Place of search | Date of completion of the search | Examiner |
|---|---|---|
| Munich | 23 July 2015 | Offermann, Stefanie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

Application Number

EP 15 15 7149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | RUDOLF E SCHOPF ET AL: "Treatment of psoriasis with the chimeric monoclonal antibody against tumor necrosis factor alpha, infliximab", JOURNAL OF THE AMERICAN ACADEMY OF DERMATOLOGY, MOSBY, INC, US, vol. 46, no. 6, 1 June 2002 (2002-06-01), pages 886-891, XP002643246, ISSN: 0190-9622, DOI: 10.1067/MJD.2002.120472 [retrieved on 2002-08-22] * the whole document * | 1-8 | |
| Y,P | EDMUND CAUZA ET AL: "Treatment of psoriatic arthritis and psoriasis vulgaris with the tumor necrosis factor inhibitor infliximab", RHEUMATOLOGY INTERNATIONAL, vol. 22, no. 6, 4 September 2002 (2002-09-04), pages 227-232, XP055078282, ISSN: 0172-8172, DOI: 10.1007/s00296-002-0246-3 * the whole document * | 1-6 | |
| Y,P | ANONYMOUS: "Humira (adalimumab Rx Only tear at Perforation to Dispense Patient Information", CLINICAL PHARMACOLOGY, DEKKER, NEW YORK, NY, US, 20 December 2002 (2002-12-20), pages 1-16, XP003031409, ISSN: 0892-001X * the whole document * | 1-6 | |

TECHNICAL FIELDS SEARCHED (IPC)

-/--

The present search report has been drawn up for all claims

| Place of search | Date of completion of the search | Examiner |
|---|---|---|
| Munich | 23 July 2015 | Offermann, Stefanie |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
   after the filing date
D : document cited in the application
L : document cited for other reasons
    .................................................................................
& : member of the same patent family, corresponding
   document

EPO FORM 1503 03.82 (P04C01)

# EUROPEAN SEARCH REPORT

Application Number

EP 15 15 7149

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 92/16553 A (UNIV NEW YORK [US]; CENTOCOR INC [US]) 1 October 1992 (1992-10-01) * the whole document * | 1-8 | |
| A | EP 0 614 984 A (MILES INC [US] BAYER AG [US]) 14 September 1994 (1994-09-14) * the whole document * | 1-8 | |
| A | BODMER M ET AL: "Preclinial review of anti-tumor necrosis factor monoclonal antibodies", CRITICAL CARE MEDICINE, WILLIAMS AND WILKINGS COMPANY, BALTIMORE, MA, US, vol. 21, no. 10, 1 October 1993 (1993-10-01), pages S441-S446, XP009056980, ISSN: 0090-3493 * the whole document * | 1-8 | |
| A | BAUGH J A ET AL: "MECHANISMS FOR MODULATING TNFALPHA IN IMMUNE AND INFLAMMATORY DISEASE", CURRENT OPINION IN DRUG DISCOVERY AND DEVELOPMENT, CURRENT DRUGS, LONDON, GB, vol. 4, no. 5, 1 January 2001 (2001-01-01), pages 635-650, XP001145475, ISSN: 1367-6733 * the whole document * | 1-8 | **TECHNICAL FIELDS SEARCHED (IPC)** |

The present search report has been drawn up for all claims

| Place of search | Date of completion of the search | Examiner |
|---|---|---|
| Munich | 23 July 2015 | Offermann, Stefanie |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

&  : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 15 15 7149

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2015

| Patent document cited in search report | | Publication date | Patent family member(s) | | Publication date |
|---|---|---|---|---|---|
| WO 9729131 | A | 14-08-1997 | AT | 239041 T | 15-05-2003 |
| | | | AU | 722077 B2 | 20-07-2000 |
| | | | BG | 64564 B1 | 29-07-2005 |
| | | | BG | 64776 B1 | 31-03-2006 |
| | | | BG | 66195 B1 | 31-01-2012 |
| | | | BR | 9707379 A | 13-04-1999 |
| | | | CA | 2243459 A1 | 14-08-1997 |
| | | | CN | 1215407 A | 28-04-1999 |
| | | | CN | 1504752 A | 16-06-2004 |
| | | | CN | 101712720 A | 26-05-2010 |
| | | | CN | 102070715 A | 25-05-2011 |
| | | | CN | 103275221 A | 04-09-2013 |
| | | | CY | 2463 B1 | 03-06-2005 |
| | | | CZ | 9802476 A3 | 11-11-1998 |
| | | | DE | 69721548 T2 | 01-04-2004 |
| | | | DE | 122004000003 I1 | 23-09-2004 |
| | | | DE | 122004000003 I2 | 29-03-2007 |
| | | | DE | 122004000004 I1 | 12-08-2004 |
| | | | DK | 0929578 T3 | 25-08-2003 |
| | | | EP | 0929578 A1 | 21-07-1999 |
| | | | ES | 2198552 T3 | 01-02-2004 |
| | | | HK | 1019452 A1 | 17-08-2007 |
| | | | HK | 1066860 A1 | 27-03-2009 |
| | | | HK | 1125972 A1 | 17-04-2014 |
| | | | HU | 228630 B1 | 29-04-2013 |
| | | | HU | 230048 B1 | 29-06-2015 |
| | | | HU | 9901874 A2 | 28-09-1999 |
| | | | IL | 125697 A | 20-03-2005 |
| | | | JP | 3861118 B2 | 20-12-2006 |
| | | | JP | 4404181 B2 | 27-01-2010 |
| | | | JP | 4890997 B2 | 07-03-2012 |
| | | | JP | 5422501 B2 | 19-02-2014 |
| | | | JP | 5689902 B2 | 25-03-2015 |
| | | | JP | 2000507810 A | 27-06-2000 |
| | | | JP | 2003177130 A | 27-06-2003 |
| | | | JP | 2007045828 A | 22-02-2007 |
| | | | JP | 2010209119 A | 24-09-2010 |
| | | | JP | 2013091666 A | 16-05-2013 |
| | | | JP | 2014055150 A | 27-03-2014 |
| | | | JP | 2015108011 A | 11-06-2015 |
| | | | LU | 91062 I2 | 26-04-2004 |
| | | | NL | 300143 I1 | 03-05-2004 |
| | | | NO | 320657 B1 | 09-01-2006 |
| | | | NO | 322755 B1 | 04-12-2006 |
| | | | NO | 983627 A | 07-10-1998 |
| | | | NO | 20026202 A | 07-10-1998 |

## ANNEX TO THE EUROPEAN SEARCH REPORT
## ON EUROPEAN PATENT APPLICATION NO.

EP 15 15 7149

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2015

| Patent document cited in search report | | Publication date | Patent family member(s) | | Publication date |
|---|---|---|---|---|---|
| | | | NZ | 331579 A | 29-06-2001 |
| | | | NZ | 512006 A | 27-05-2005 |
| | | | NZ | 536216 A | 31-08-2006 |
| | | | NZ | 562935 A | 26-06-2009 |
| | | | NZ | 576716 A | 25-02-2011 |
| | | | PL | 193499 B1 | 28-02-2007 |
| | | | PL | 328411 A1 | 18-01-1999 |
| | | | PT | 929578 E | 30-09-2003 |
| | | | RO | 119831 B1 | 29-04-2005 |
| | | | RO | 123028 B1 | 30-07-2010 |
| | | | RU | 2003120859 A | 10-01-2005 |
| | | | RU | 2012102323 A | 27-07-2013 |
| | | | SI | 9720020 A | 31-10-1999 |
| | | | SK | 106298 A3 | 10-03-1999 |
| | | | TR | 9801532 T2 | 23-11-1998 |
| | | | UA | 82823 C2 | 26-05-2008 |
| | | | US | 6258562 B1 | 10-07-2001 |
| | | | US | 2003092059 A1 | 15-05-2003 |
| | | | US | 2007249813 A1 | 25-10-2007 |
| | | | US | 2009155205 A1 | 18-06-2009 |
| | | | US | 2010040604 A1 | 18-02-2010 |
| | | | US | 2012258114 A1 | 11-10-2012 |
| | | | US | 2013115224 A1 | 09-05-2013 |
| | | | US | 2013330356 A1 | 12-12-2013 |
| | | | US | 2013330357 A1 | 12-12-2013 |
| | | | WO | 9729131 A1 | 14-08-1997 |
| WO 9216553 A | | 01-10-1992 | AT | 201234 T | 15-06-2001 |
| | | | AT | 366316 T | 15-07-2007 |
| | | | AU | 668864 B2 | 23-05-1996 |
| | | | AU | 1764992 A | 21-10-1992 |
| | | | CA | 2106299 A1 | 19-09-1992 |
| | | | CA | 2736076 A1 | 19-09-1992 |
| | | | DE | 06001779 T1 | 14-01-2010 |
| | | | DE | 07006112 T1 | 21-01-2010 |
| | | | DE | 07012625 T1 | 21-01-2010 |
| | | | DE | 07012626 T1 | 21-01-2010 |
| | | | DE | 10199067 I1 | 21-02-2002 |
| | | | DE | 69231828 D1 | 21-06-2001 |
| | | | DE | 69231828 T2 | 05-10-2006 |
| | | | DE | 69233701 T2 | 10-04-2008 |
| | | | DK | 0610201 T3 | 18-06-2001 |
| | | | DK | 1097945 T3 | 05-11-2007 |
| | | | EP | 0610201 A1 | 17-08-1994 |
| | | | EP | 1097945 A2 | 09-05-2001 |
| | | | EP | 1681305 A2 | 19-07-2006 |

For more details about this annex : see Official Journal of the European Patent Office, No. 12/82

**EP 2 942 359 A1**

ANNEX TO THE EUROPEAN SEARCH REPORT
ON EUROPEAN PATENT APPLICATION NO.

EP 15 15 7149

This annex lists the patent family members relating to the patent documents cited in the above-mentioned European search report.
The members are as contained in the European Patent Office EDP file on
The European Patent Office is in no way liable for these particulars which are merely given for the purpose of information.

23-07-2015

| Patent document cited in search report | Publication date | Patent family member(s) | Publication date |
|---|---|---|---|
| | | EP 1857553 A1 | 21-11-2007 |
| | | EP 1905782 A1 | 02-04-2008 |
| | | ES 2156859 T3 | 01-08-2001 |
| | | ES 2289997 T3 | 16-02-2008 |
| | | GR 3036375 T3 | 30-11-2001 |
| | | HK 1037923 A1 | 21-09-2007 |
| | | JP H06506120 A | 14-07-1994 |
| | | JP 2004180686 A | 02-07-2004 |
| | | JP 2007197457 A | 09-08-2007 |
| | | JP 2007254477 A | 04-10-2007 |
| | | JP 2008156371 A | 10-07-2008 |
| | | JP 2008195724 A | 28-08-2008 |
| | | JP 2011155992 A | 18-08-2011 |
| | | JP 2012087144 A | 10-05-2012 |
| | | LU 90854 I2 | 04-02-2002 |
| | | NL 300069 I1 | 01-02-2002 |
| | | WO 9216553 A1 | 01-10-1992 |
| EP 0614984 A | 14-09-1994 | AT 204299 T | 15-09-2001 |
| | | CA 2116888 A1 | 06-09-1994 |
| | | DE 69427928 D1 | 20-09-2001 |
| | | DE 69427928 T2 | 29-11-2001 |
| | | DE 122009000074 I1 | 01-12-2011 |
| | | DK 0614984 T3 | 05-11-2001 |
| | | EP 0614984 A2 | 14-09-1994 |
| | | ES 2159529 T3 | 16-10-2001 |
| | | JP 4225519 B2 | 18-02-2009 |
| | | JP H06319587 A | 22-11-1994 |
| | | JP 2009046487 A | 05-03-2009 |
| | | LU 91661 I2 | 10-05-2010 |
| | | NL 300421 I1 | 04-01-2010 |
| | | PT 614984 E | 28-12-2001 |
| | | US 5654407 A | 05-08-1997 |

For more details about this annex : see Official Journal of the European Patent Office, No. 12/82

85

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 39727502 P
- US 41108102 P
- US 41749002 P
- US 45577703 P
- US 6090382 A
- US 6258562 B
- US 6509015 B
- US 80118501 A
- US 30235602 A
- US 16365702 A
- US 13371502 A
- US 5231024 A, Moeller
- EP 260610 B1, Moeller, A.
- US 6258562 A
- US 6509015 A
- US 801185 A
- US 10302356 A
- WO 9103553 A
- WO 09406476 A
- US 5656272 A
- EP 0154316 A
- EP 0401384 A
- US 4816397 A, Boss
- US 5168062 A, Stinski
- US 4510245 A, Bell
- US 4968615 A, Schaffner
- US 4399216 A
- US 4634665 A
- US 5179017 A, Axel
- US 5223409 A, Ladner
- WO 9218619 A, Kang
- WO 9117271 A, Dower
- WO 9220791 A, Winter
- WO 9215679 A, Markland
- WO 9301288 A, Breitling
- WO 9201047 A, McCafferty
- WO 9209690 A, Garrard
- WO 9406476 A
- WO 9319751 A
- WO 02072636 A

### Non-patent literature cited in the description

- **MOELLER et al.** *Cytokine,* 1990, vol. 2, 162
- **VASILLI.** *Annu. Rev. Immunol.,* 1992, vol. 10, 411
- **TRACEY ; CERAMI.** *Annu. Rev. Med.,* 1994, vol. 45, 491
- **PENNICA, D. et al.** *Nature,* 1984, vol. 312, 724-729
- **DAVIS, J.M. et al.** *Biochemistry,* 1987, vol. 26, 1322-1326
- **JONES, E.Y. et al.** *Nature,* 1989, vol. 338, 225-228
- **WARD et al.** *Nature,* 1989, vol. 341, 544-546
- **BIRD et al.** *Science,* 1988, vol. 242, 423-426
- **HUSTON et al.** *Proc. Natl. Acad. Sci. USA,* 1988, vol. 85, 5879-5883
- **HOLLIGER, P. et al.** *Proc. Natl. Acad. Sci. USA,* 1993, vol. 90, 6444-6448
- **POLJAK, R.J. et al.** *Structure,* 1994, vol. 2, 1121-1123
- **SONGSIVILAI ; LACHMANN.** *Clin. Exp. Immunol.,* 1990, vol. 79, 315-321
- **KOSTELNY et al.** *J. Immunol.,* 1992, vol. 148, 1547-1553
- **TAYLOR, L.D. et al.** *Nucl. Acids Res.,* 1992, vol. 20, 6287
- **JÖNSSON et al.** *Ann. Biol Clin.,* 1993, vol. 51, 19
- **JÖNSSON et al.** *Biotechniques,* 1991, vol. 11, 620-627
- **JOHNSSON et al.** *J. Mol. Recognit.,* 1995, vol. 8, 125
- **JOHNNSON et al.** *Anal. Biochem.,* 1991, vol. 198, 268
- *Focus on Growth Factors,* 1992, vol. 3, 4-10
- **CANFIELD, S.M. ; S.L. MORRISON.** *J. Exp. Med.,* 1991, vol. 173, 1483-1491
- **LUND, J. et al.** *J. of Immunol.,* 1991, vol. 147, 2657-2662
- Molecular Cloning; A Laboratory Manual. Cold Spring Harbor, 1989
- Current Protocols in Molecular Biology. Greene Publishing Associates, 1989
- **KABAT, E.A. et al.** Sequences of Proteins of Immunological Interest. NIH Publication No. 91-3242, 1991

- **TOMLINSON, I.M. et al.** The Repertoire of Human Germline VH Sequences Reveals about Fifty Groups of YH Segments with Different Hypervariable Loops. *J. Mol. Biol.,* 1992, vol. 227, 776-798
- **COX, J.P.L. et al.** A Directory of Human Germ-line V78 Segments Reveals a Strong Bias in their Usage. *Eur. J. Immunol.,* 1994, vol. 24, 827-836
- **MCCAFFERTY et al.** *Nature,* 1990, vol. 348, 552-554
- **GOEDDEL.** Gene Expression Technology: Methods in Enzymology. Academic Press, 1990, vol. 185
- **BOSS, M.A. ; WOOD, C. R.** *Immunology Today,* 1985, vol. 6, 12-13
- **URLAUB ; CHASIN.** *Proc. Natl. Acad. Sci. USA,* 1980, vol. 77, 4216-4220
- **R.J. KAUFMAN ; P.A. SHARP.** *Mol. Biol,* 1982, vol. 159, 601-621
- **FUCHS et al.** *Bio/Technology,* 1991, vol. 9, 1370-1.372
- **HAY et al.** *Hum Antibod Hybridomas,* 1992, vol. 3, 81-85
- **HUSE et al.** *Science,* 1989, vol. 246, 1275-1281
- **GRIFFITHS et al.** *EMBO J,* 1993, vol. 12, 725-734
- **HAWKINS et al.** *J Mol Biol,* 1992, vol. 226, 889-896
- **CLACKSON et al.** *Nature,* 1991, vol. 352, 624-628
- **GRAM et al.** *PNAS,* 1992, vol. 89, 3576-3580
- **GARRARD et al.** *Bio/Technology,* 1991, vol. 9, 1373-1377
- **HOOGENBOOM et al.** *Nuc Acid Res,* 1991, vol. 19, 4133-4137
- **BARBAS et al.** *PNAS,* 1991, vol. 88, 7978-7982
- **MOELLER, A. et al.** *Cytokine,* 1990, vol. 2, 162-169
- **VASILLI, P.** *Annu. Rev Immunol.,* 1992, vol. 10, 411-452
- **CERAMI, A.** *Annu. Rev. Med.,* 1994, vol. 45, 491-503
- **TAUROG et al.** The Spondylarthritides. Oxford:Oxford University Press, 1998
- **SHIMIZU et al.** *Proc Natl Acad Sci. USA,* 1990, vol. 87, 6441
- **NICKOLOFF.** *Investig Dermatol Symp Proc.,* 2000, vol. 5, 67
- **AUSTIN et al.** *Am J Pathol.,* 1995, vol. 146, 1529
- **LERNER et al.** *J Invest Dermatol.,* 1986, vol. 87, 299
- **BAEDER et al.** *Clin Exp Immunol.,* 1992, vol. 89, 174
- **HASEYAMA et al.** *Tohoku J Exp Med.,* 2002, vol. 198, 233
- **MAKINO et al.** *Exp.Anim.,* 1980, vol. 29, 1
- **KOLB.** *Diabetes/Metabolism Reviews,* 1987, vol. 3, 751
- **HAMADA et al.** *Metabolism,* 2001, vol. 50, 1282
- **COLEMAN.** *Diabetologia,* 1978, vol. 14, 141
- **BAILEY et al.** *Int.J.Obesity,* 1982, vol. 6, 11
- **NOSE, M. et al.** *Am. J. Path. 1,* 1996, vol. 49, 1763
- **KINJOH et al.** *Proc. Natl. Acad. Sci., USA,* 1993, vol. 90, 3413
- **BROUWER, E. et al.** *J. Exp. Med.,* 1993, vol. 177, 905
- **HESSEL, EM. et al.** *Eur J Pharmacol.,* 1995, vol. 293, 401
- **KEAST D et al.** *J. Pathol.,* 1981, vol. 135, 249
- **FERNS et al.** *Science,* 1991, vol. 253, 1129
- **CLOWES et al.** *Lab. Invest.,* 1983, vol. 49, 208
- **LINDNER et al.** *Circ Res.,* 1993, vol. 73, 792
- **COCCIA et al.** *Exp Hematology,* 2001, vol. 29, 1201-1209
- **BENNETT ; ZIE.** *Pain,* 1988, vol. 33, 87-107
- **KIM ; CHUNG.** *Pain,* 1992, vol. 50, 355-363
- **VERJANS et al.** *Arthritis Rheum.,* 1991, vol. 34 (4), 486
- **VERJANS et al.** *Clin. Exp Immunol.,* 1994, vol. 97 (1), 45
- **KAIJTZEL et al.** *Hum Immunol,* 1999, vol. 60 (2), 140
- **DUFFY et al.** *ACR 66th Annual Scientific Meeting Abstract,* 2002
- **PARTSCH et al.** *Ann Rheum Dis.,* 1998, vol. 57, 691
- **RITCHLIN et al.** *J Rheumatol,* 1998, vol. 25, 1544
- **BRAUN et al.** *Arthritics Rheum.,* 1999, vol. 42 (10), 2039
- **ZEIDLER et al.** *Rheum Dis Clin North Am.,* 1992, vol. 18, 187
- **PIQUET PF et al.** *J Exp Med.,* 1989, vol. 170, 655-63
- **WHYTE M et al.** *Am J Respir Crit Care Med,* 2000, vol. 162, 755-8
- **ANTICEVICH SZ et al.** *Eur J Pharmacol.,* 1995, vol. 284, 221-5
- **CORBETT EL et al.** *Am J Respir Crit Care Med,* 2002, vol. . 165, 690-3
- **PIQUET et al.** *Am J Pathol,* 1993, vol. 143, 651
- **NASH et al.** *Histopathology,* 1993, vol. 22, 343
- **ZHANG et al.** *J Immunol,* 1993, vol. 150, 4188
- American Thoracic Society. *Am. J. Respir. Crit. Care Med.,* 2000, vol. 161, 646
- **THOMAS PS et al.** *Am J Respir Crit Care Med,* 1995, vol. 152, 76-80

- **THOMAS PS ; HEYWOOD G.** *Thorax,* 2002, vol. 57, 774-8
- **ORDONEZ CL. et al.** *Am J Respir Crit Care Med,* 2000, vol. 161, 1185
- **KIPS et al.** *Am Rev Respir Dis,* 1992, vol. 145, 332
- NAEPP Expert Panel Report Guidelines for the Diagnosis and Management of Asthma-Update on Selected Topics 2002. *JACI,* 2002, vol. 110, S141-S209
- Guidelines for the Diagnosis and Management of Asthma. NIH Publication 97-4051, July 1997
- **KEATINGS VM.** *Chest,* 2000, vol. 118, 971-5
- **SAKAO S et al.** *Am J Respir Crit Car e Med,* 2001, vol. 163, 420-22
- **SAKAO S et al.** *Chest,* 2002, vol. 122, 416-20
- **MEDALL et al.** *Heart,* 1997, vol. 78 (3), 273
- **ZHOU et al.** *Atherosclerosis,* 2002, vol. 161, 153
- **JAVED et al.** *Exp and Mol Pathol,* 2002, vol. 73, 104
- **ZIMMERMAN et al.** *Am J Phsiol Regul Integr Comp Physiol,* 2002, vol. 283, R505
- Myointimal Hyperplasia. **COLBURN ; MOORE.** Vascular Surgery: A Comprehensive Review. Saunders, 1998, 690-709
- **BERK ; HARRIS.** *Adv. Intern. Med.,* 1995, vol. 40, 455-501
- **CLAGETT et al.** *J. Vase. Surg,* 1986, vol. 3, 10-23
- **LEVINE et al.** *N Engl JMed,* 1990, vol. 323, 236
- **TORRE-AMIONE et al.** *J Am Coll Cardiol,* 1996, vol. 27, 1201
- **CHUNG et al.** *Circulation,* 2003, vol. 107, 3133
- **LIBBY.** *Circulation,* 1995, vol. 91, 2844
- **NAKAMURA et al.** *J. Cardiol.,* 2003, vol. 41, 41
- **SUGANO et al.** *FASEB J,* 2002, vol. 16, 1421
- **BALBAY et al.** *Angiology,* 2001, vol. 52, 109
- **SPIEGELMAN ; HOTAMISLIGIL.** *Cell,* 1993, vol. 73, 625
- **CHU et al.** *Int J Obes Relat Metab Disord.,* 2000, vol. 24, 1085
- **ISHII et al.** *Metabolism,* 2000, vol. 49, 1616
- **NAVARRO J.F. ; MORA C.** *Maca, Am J Kidney Dis.,* July 2003, vol. 42 (1), 53-61
- **DAIMON M et al.** *Diabetes Care.,* July 2003, vol. 26 (7), 2015-20
- **ZHANG M et al.** *J Tongji Med Univ.,* 1999, vol. 19 (3), 203-5
- **BARBIERI M et al.** *Am J Hypertens,* July 2003, vol. 16 (7), 537-43
- **MCCALL, J. et al.** *Br. J. Surg.,* 1992, vol. 79, 361-3
- **BENJAFIELD et al.** *Diabetes Care.,* 2001, vol. 24, 753
- **QIANG, X. et al.** *Diabetologia,* 1998, vol. 41, 1321-6
- **PFEIFFER et al.** *Horm Metab Res.,* 1997, vol. 29, 111
- **SCHOLZ et al.** *Trends Microbiol.,* 2003, vol. 11, 171
- **LEE et al.** *Hum Immunol.,* 2003, vol. 64, 614
- **NAVARRO et al.** *Am J Kidney Dis.,* 2003, vol. 42, 53
- **DAIMON et al.** *Diabetes Care.,* 2003, vol. 26, 2015
- **ZHANG et al.** *J Tongji Med Univ.,* 1999, vol. 19, 203
- **BARBIERI et al.** *Am J Hypertens,* 2003, vol. 16, 537
- **VENN et al.** *Arthritis Rheum.,* 1993, vol. 36, 819
- **WESTACOTT et al.** *J Rheumatol.,* 1994, vol. 21, 1710
- **DEVARAJ et al.** *Circulation,* 2000, vol. 102, 191
- **HATTORI Y et al.** *Cardiovasc Res.,* 2000, vol. 46, 188
- **CLAUSELL N et al.** *Cardiovasc Pathol.,* 1999, vol. 8, 145
- **PIHLAJAMAKI J et al.** *Obes Res.,* 2003, vol. 11, 912
- **TSUDA et al.** *J Nutr.,* 2003, vol. 133, 2125
- **JONGEN-LAVRENCIC M. et al.** *J. Rheumatol.,* 1997, vol. 24 (8), 1504-9
- **DEMETER J. et al.** *Ann Hematol.,* 2002, vol. 81 (10), 566-9
- **DICATO M.** *The Oncologist,* 2003, vol. 8 (1), 19-21
- **SORKIN, LS. et al.** *Neuroscience,* 1997, vol. 81 (1), 255-62
- **HUYGEN FJ. et al.** *Mediators Inflamm,* 2002, vol. 11 (1), 47-51
- **PARADA CA et al.** *Eur J Neurosci.,* 2003, vol. 17 (9), 1847-52
- **SOMMER C.** *Schmerz,* 1999, vol. 13 (5), 315-23
- **EMPL M et al.** *Neurology,* 2001, vol. 56 (10), 1371-7
- **SCHAFERS M et al.** *J Neurosci,* 2003, vol. 23 (7), 3028-38
- **MYERS.** *Regional Anesthesia,* 1995, vol. 20, 173-184
- **COELHO A. et al.** *Am J Physiol Gastrointest Liver Physiol.,* 2000, vol. 279, G781-G790
- **COELHO A et al.** *Brain Res Bull.,* 2000, vol. 52 (3), 223-8
- **COLLETTI LM. et al.** *J Clin Invest,* 1990, vol. 85 (6), 1936-43
- **TIEGS G.** *Acta Gastroenterol Belg,* 1997, vol. 60 (2), 176-9
- **FERNANDEZ ED. et al.** *J Endotoxin Res.,* 2000, vol. 6 (4), 321-8

- Current Indications, Contra Indications and Timing for Liver Transplantation. **WIESNER, R. H.** Transplantation of the Liver. Saunders (publ.), 1996
- Partial Hypertension: The Role of Liver Transplantation. **KLEIN, A. W.** Current Surgical Therapy. Musby (publ.), 1998
- **GONZALEZ-AMARO.** *J Exp Med.,* 1994, vol. 179, 841-8
- **NELSON DR et al.** *Dig Dis Sci,* 1997, vol. 42, 2487-94
- **KALLINOWSKI B et al.** *Clin Exp Imnunol.,* 1998, vol. 111, 269-77
- **COOKSON S. et al.** *Hepatology,* 1999, vol. 30 (4), 851-6
- **JAZRAWI S. et al.** *Liver Transpl,* 2003, vol. 9 (4), 377-82
- **JOHNSON P. J. et al.** *Hepatology,* 1993, vol. 18, 998-1005
- **HOMBERG J. C. et al.** *Hepatology,* 1987, vol. 7, 1333-1339
- **MAGGIORE G. et al.** *J. Pediatr. Gastroenterol Nutr,* 1993, vol. 17, 376-381
- **MARTINI E. et al.** *Hepatology,* 1998, vol. 8, 1662-1666
- **VALENTI L. et al.** *Gastrocnerology,* 2002, vol. 122 (2), 274-80
- **LI Z. et al.** *Hepatology,* 2003, vol. 37 (2), 343-50
- **KASAHARA S. et al.** *J Virol,* 2003, vol. 77 (4), 2469-76
- **WANG F.S.** *World J Gastroenterol,* 2003, vol. 9 (4), 641-4
- **BIERMER M. et al.** *J Virol,* 2003, vol. 77 (7), 4033-42
- **BRUCCOLERI A. et al.** *Hepatology,* 1997, vol. 25 (1), 133-41
- **LUSTER M.I. et al.** *Ann NY Acad Sci.,* 2000, vol. 919, 214-20
- **SIMEONOVA P. et al.** *Toxicol Appl Pharmacol.,* 2001, vol. 177 (2), 112-20
- **TAKENAKA K. et al.** *Dig Dis Sci.,* 1998, vol. 43 (4), 887-92
- **NAGAKI M. et al.** *J Hepatol.,* 1999, vol. 31 (6), 997-1005
- **STREETZ K. et al.** *Gastroenterology,* 2000, vol. 119 (2), 446-60
- **CRESPO J. et al.** *Hepatology,* 2001, vol. 34 (6), 1158-63
- **LUDWIG, J. R. et al.** *Mayo Clin. Proc.,* 1980, vol. 55, 434
- **POWELL E. et al.** *Hepatol.,* 1990, vol. 11, 74
- **TAKEMATSU et al.** *Arch Dermatol Res.,* 1989, vol. 281, 398
- **VICTOR ; GOTTLIEB.** *J Drugs Dermatol,* 2002, vol. 1 (3), 264
- **ASADULLAH et al.** *Br J Dermatol.,* 1999, vol. 141, 94
- **SKLAVOUNOU et al.** *J Oral Pathol Med.,* 2000, vol. 29, 370
- **REUSS-BORST et al.** *Br J Haematol.,* 1993, vol. 84, 356
- **TUTUNCU Z et al.** *Clin Exp Rheumatol.,* 2002, vol. 20 (6), S146-51
- **MACKIEWICZ Z et al.** *Clin Exp Rheumatol.,* 2003, vol. 21 (1), 41-8
- **MUROTA H et al.** *Arthritis Rheum.,* 2003, vol. 48 (4), 1117-25
- **DEGUCHI et al.** *Lancet,* 1989, vol. 2, 745
- **SNELLER, M.C.** *Cleve. Clin. J. Med.,* 2002, vol. 69, SII40-3
- **SCHETT, G. et al.** *Ann. Rheum. Dis.,* 2002, vol. 61, 463
- **STRAUB, R.H. et al.** *Rheumatology (Oxford),* 2002, vol. 41, 423
- **UDDHAMMAR, A. et al.** *Br. J. Rheumatol.,* 1998, vol. 37, 766
- **KOBAYASHI, Y. ; NUMANO, F.** *Intern. Med,* 2002, vol. 41, 44
- **FRAGA, A. ; MEDINA F.** *Curr. Rheumatol. Rep.,* 2002, vol. 4, 30
- **DIGIROLAMO, N. et al.** *J. Leukoc. Biol.,* 1997, vol. 61, 667
- **SUNDEL, R.P.** *Curr. Rheumatol. Rep.,* 2002, vol. 4, 474
- **GEDALIA, A.** *Curr. Rheumatol. Rep.,* 2002, vol. 4, 25
- **SFIKAKIS, P.P.** *Ann. Rheum. Dis.,* 2002, vol. 61, ii51-3
- **DOGAN, D. ; FARAH, C.** *Oftalmologia,* 2002, vol. 52, 23
- Inflammatory Disease of Blood Vessels. Marcel Dekker, Inc, 2001, 473
- **MARQUEZ, J. et al.** *Curr. Rheumatol. Rep.,* 2003, vol. 5, 128
- **HARMAN, L.E. ; MARGO, C.E.** *Surv. Ophthalmol.,* 1998, vol. 42, 458
- **GROSS, W.L.** *Curr. Opin. Rheumatol.,* 2002, vol. 14, 11
- **CHURG, W.A.** *Mod. Pathol.,* 2001, vol. 14, 1284
- **TRACY, K.J. et al.** *Science,* 1986, vol. 234, 470-474
- **SUN, X-M. et al.** *J. Clin. Invest.,* 1988, vol. 81, 1328-1331
- **MACDONALD, T.T. et al.** *Clin. Exp. Immunol,* 1990, vol. 81, 301-305
- **GROM et al.** *Arthritis Rheum.,* 1996, vol. 39, 1703
- **MANGGE et al.** *Arthritis Rheum.,* 1995, vol. 8, 211
- **EISERMANN J et al.** *Fertil Steril,* 1988, vol. 50, 573
- **HALME J.** *Am J Obstet Gynecol,* 1989, vol. 161, 1718

- **MORI H et al.** *Am J Reprod Immunol,* 1991, vol. 26, 62
- **TAKETANI Y et al.** *Am J Obstet Gynecol,* 1992, vol. 167, 265
- **OVERTON C.** *Hum Reprod,* 1996, vol. 11, 380
- **ALEXANDER RB et al.** *Urology,* 1998, vol. 52, 744
- **NADLER RB et al.** *J Urol,* 2000, vol. 164, 214
- **ORHAN et al.** *Int J Urol,* 2001, vol. 8, 495
- **ASAKAWA K et al.** *Hinyokika Kiyo,* 2001, vol. 47, 459
- **HARRIS et al.** *Prostate,* 2000, vol. 44, 25
- **SHVIDEL et al.** *Hematol J.,* 2002, vol. 3, 32
- **STUDNICKA-BENKE et al.** *Br J Rheumatol.,* 1996, vol. 35, 1067
- **OH H et al.** *Invest Ophthalmol Vis Sci,* 1999, vol. 40, 1891
- **OZAKTAY et al.** *Eur Spine J,* 2002, vol. 11, 467
- **BRISBY et al.** *Lur Spine J,* 2002, vol. . 11, 62
- **KOSKI et al.** *Clin Exp Rheumatol.,* 2001, vol. 19, 131
- **WAKEFIELD ; LLOYD.** *Cytokine,* 1992, vol. 4, 1
- **WOON et al.** *Curr Eye Res,* 1998, vol. . 17, 955
- **TSUTSUMIMATO et al.** *JBone Miner Res,* 1999, vol. 14, 1751
- Sustained and Controlled Release Drug Delivery Systems. Marcel Dekker, Inc, 1978
- *Arthritis & Rheumatism,* 1994, vol. 37, S295
- *J. Invest. Med.,* 1996, vol. 44, 235A
- *Arthritis & Rheumatism,* 1995, vol. 38, S185
- *Arthritis & Rheumatism,* 1993, vol. 36, 1223
- *Anthritis & Rheumatism,* 1996, vol. 39 (9), S284
- *Amer. J. Physiol. - Heart and Circulatory Physiology,* 1995, vol. 268, 37-42
- *Arthritis & Rheumatism,* 1996, vol. 39 (9), S282
- *Arthritis & Rheumatism,* 1996, vol. 39 (9), S81
- *Arthritis & Rheumatism,* 1996, vol. 39 (9), S82
- *Arthritis & Rheumatism,* 1996, vol. 39 (9), S131
- *Inflammation Research,* 1996, vol. 45, 103-107
- *Arthritis & Rheumatism,* 1996, vol. 39 (9), S284
- *Arthritis & Rheumatism,* 1996, vol. 39 (9), S280
- *Neuro Report,* 1996, vol. 7, 1209-1213
- *Arthritis & Rheumatism,* 1996, vol. 39 (9), S281
- *Arthritis & Rheumatism,* 1996, vol. 39 (9), S296
- *Arthritis & Rheumatism,* 1996, vol. 39 (9), S308
- *Arthritis & Rheumatism,* 1996, vol. 39 (9), S120
- **DELUCA et al.** *Rheum. Dis. Clin. North Am.,* 1995, vol. 21, 759-777
- **ZHANG et al.** *Curr Rheumatol Rep.,* 2002, vol. 4, 507
- **MARZI et al.** *Shock,* 1995, vol. 3, 27
- **WILLIAMS et al.** *Proc Natl Acad Sci USA.,* 1992, vol. 89, 9784
- **MOLL et al.** *Ann Rheum Dis,* 1973, vol. 32, 354
- **VAN DER LINDEN et al.** *Arthritis Rheum,* 1984, vol. 27, 361
- **GARRETT et al.** *J Rheumatol,* 1994, vol. 21, 2286
- **JENKINSON et al.** *J Rheumatol,* 1994, vol. 21, 1694
- **CALIN et al.** *J Rheumatol,* 1994, vol. 21, 2281
- **HESSEL, E.M. et al.** *Eur. J. Pharmacol.,* 1995, vol. 293, 401
- **DAPHNE, T. et al.** *Am. J. Respir. Cell Mol. Biol.,* 2001, vol. 25, 751
- **WILLIAMS et al.** *Proc Natl Acad Sci U S A.,* 1992, vol. 89, 9784
- **HOFSTRA, C.L. et al.** *Inflamm. Res.,* 1999, vol. 48, 602
- **KEAST, D. et al.** *J. Pathol.,* 1981, vol. 135, 249
- **HAUTMAKI, R.D. et al.** *Science,* 1997, vol. 277, 2002
- **HAUTMAKI et al.** *Science,* 1997, vol. 277, 2002
- **COHN, L. et al.** *J. Exp. Med.,* 1997, vol. 186, 1737
- **RAY, P. et al.** *J. Clin. Invest.,* 1997, vol. 100, 2501
- **BOWDEN, D.H.** *Lab. Invest.,* 1984, vol. 50, 487
- **TOKUDA, A. et al.** *J. Immunol.,* 2000, vol. 164, 2745
- **SMITH et al.** *J. Immunol.,* 1994, vol. 153, 4704
- **YONEYAMA et al.** *J. Clin. Invest.,* 1998, vol. 102, 1933
- **MURAI et al.** *J. Clin. Invest.,* 1999, vol. 104, 49
- **JONES, P.W. et al.** *Resp. Med.,* 1991, vol. 85, 25
- **STULBARG, M. ; ADAMS, L. DYSPNEA.** Textbook of Respiratory Medicine. WB Saunders, 2000, 541-552
- **KUMAR ; LINDNER.** *Arterioscler. Thromb. Vasc. Biol.,* 1997, vol. 17, 2238
- **DE WAARD et al.** *Arterioscler. Thromb. Vasc. Biol.,* 2002, vol. 22, 1978
- **KUMAR et al.** *Circulation,* 1997, vol. 96, 4333

- **LENTZ SR et al.** *Circulation,* 2002, vol. 106 (7), 842-6
- **SUNDELL CL et al.** *CIRCULATION,* 2003, vol. 305 (3), 1116-23
- **JACKSON et al.** *Am Heart J,* 2003, vol. 145, 875
- **BURNS et al.** *J Am Coll Cardiol.,* 2002, vol. 39, 30
- **UEDA et al.** *Diabetes,* May 1999, vol. 48, 1168-1174
- **WESTMAN.** *Diabetologia,* 1968, vol. 4, 141
- **BRAY ; YORK.** *Physiological reviews,* 1971, vol. 51, 598
- **CATANI et al.** *Braz. J. Med. Biol. Res.,* 2003, vol. 36, 693
- **BENNETT ; ZIE.** *Pain,* 1988, vol. 33, 87
- **SHIMIZU et al.** *Proc. Natl. Acad Sci. USA,* 1990, vol. 87, 6441
- **WEINER et al.** *Lancet,* 1990, vol. 335, 1
- **SHIMIZU et al.** *Proc. Natl. Acad. Sci. USA,* 1985, vol. 82, 2138
- **NICKOLOFF et al.** *Am J Pathol,* 1995, vol. 146, 580-8
- **FREDRIKSSON ; PETTERSSON.** *Dermatologica,* 1978, vol. 157, 238-44
- **HIRATA, Y. et al.** *Acta. Otolaryngol.,* 1993, vol. 503, 79
- *EMBO J,* 1991, vol. 10, 4025-4031
- **LEHMAN, T.J. et al.** *Arthritis Rheum,* 1985, vol. 28, 652
- **DUONG, T.T.** *Int Immunol,* 1985, vol. 15, 79
- **BRAHN, E. et al.** *Clin Immunol,* 1999, vol. 90, 147
- **LEHMAN et al.** *Arthritis Rheum,* 1985, vol. 28, 652

- **DUONG.** *Int Immunol,* 2002, vol. 15, 79
- **BRAHN et al.** *Clin Immunol,* 1999, vol. 90, 147
- **GRUNEBAUM et al.** *Clin. Exp. Immunol.,* 2002, vol. 130, 233
- **BLANK et al.** *Clin. Exp. Immunol.,* 1995, vol. 102, 120
- **TOMER et al.** *Arthritis Rheum.,* 1995, vol. 38, 1375
- **DAMIANOVICH et al.** *J. Immunol.,* 1996, vol. 156, 4946
- **DAMIANOVICH et al.** *J. Immunol,* 1996, vol. 156, 4946
- **BARRON, K.S. et al.** *J. Rheumatol,* 1999, vol. 26, 170
- *Lancet.,* 1990, vol. 335, 1078
- **KAKLAMANI, V.G. et al.** *Semin. Arthritis Rheum,* 2001, vol. 30, 299
- **REILLY ; GILKESON.** *Immunologic Research,* 2002, vol. 25 (2), 143-153
- **MISHRA et al.** *J Clin Invest,* 2003, vol. 111 (4), 539-552
- **WEINBERG et al.** *J Exp Med.,* 1994, vol. 179, 651
- **WATSON et al.** *J Exp Med.,* 1992, vol. 176, 1645-1656
- **VITALI.** *Arthritis Rheum,* 1993, vol. 36, 340-7
- **FOX et al.** *Arthritis Rheum,* 1986, vol. 29, 577-85
- **P.J. SHIRLAW.** *the conference on New Advances in Basic Science, Diagnosis and Treatment of Sjögren's Syndrome,* January 1997
- **NAVAZESH.** *Ann NY Acad Sci,* 1993, vol. 694, 72-7
- **GIANNINI et al.** *Arthritis & Rheumatism,* 1997, vol. 40, 1202